(19) Europäisches Patentamt
European Patent Office
Office européen des brevets

(11) Veröffentlichungsnummer: **0 591 808 A1**

(12) # EUROPÄISCHE PATENTANMELDUNG

(21) Anmeldenummer: **93115565.9**

(22) Anmeldetag: **27.09.93**

(51) Int. Cl.$^5$: **C07D 519/00**, A61K 31/47, //(C07D519/00,501:00,471:00), (C07D519/00,501:00,498:00)

(30) Priorität: **09.10.92 DE 4234078**

(43) Veröffentlichungstag der Anmeldung:
**13.04.94 Patentblatt 94/15**

(84) Benannte Vertragsstaaten:
**AT BE CH DE DK ES FR GB GR IE IT LI LU MC NL PT SE**

(71) Anmelder: **BAYER AG**

**D-51368 Leverkusen(DE)**

(72) Erfinder: **Petersen, Uwe, Dr.**
**Auf dem Forst 4**
**D-51375 Leverkusen(DE)**
Erfinder: **Schröck, Wilfried, Dr.**
**Heinrich-Heine-Strasse 45**
**D-42327 Wuppertal(DE)**
Erfinder: **Häbich, Dieter, Dr.**
**Krummacher Strasse 82**
**D-42115 Wuppertal(DE)**
Erfinder: **Krebs, Andreas, Dr.**
**Am Gartenfeld 70**
**D-51519 Odenthal(DE)**
Erfinder: **Schenke, Thomas, Dr.**
**Mühlenstrasse 113**
**D-51469 Bergisch Gladbach(DE)**
Erfinder: **Philipps, Thomas, Dr.**
**Silesiusstrasse 74**
**D-51065 Köln(DE)**
Erfinder: **Grohe, Klaus, Dr.**
**Am Wasserturm 10**
**D-51519 Odenthal(DE)**
Erfinder: **Endermann, Rainer, Dr.**
**In den Birken 152a**
**D-42113 Wuppertal(DE)**
Erfinder: **Bremm, Klaus-Dieter, Dr.**
**Girardetstrasse 120**
**D-42109 Wuppertal(DE)**
Erfinder: **Metzger, Karl-Georg, Dr.**
**Pahlkestrasse 75**
**D-42115 Wuppertal(DE)**

(54) **Chinoloncarbonsäuren.**

(57) Die Erfindung betrifft neue Chinolon- und Naphthyridoncarbonsäure-Derivate, die mit einem $\beta$-Lactamantibiotikum verknüpft sind, ihre Salze, Verfahren zu ihrer Herstellung sowie diese enthaltende antibakterielle Mittel.

EP 0 591 808 A1

Die Erfindung betrifft neue Chinolon- und Naphthyridoncarbonsäure-Derivate, die mit einem β-Lactamantibiotikum verknüpft sind, ihre Salze, Verfahrenzu ihrer Herstellung sowie diese enthaltende antibakterielle Mittel.

Aus den europäischen Patentanmeldungen 0 335 297 und 0 492 277 sind bereits Verbindungen bekanntgeworden, in welchen ein Chinolon mit einem Cephalosporin verknüpft ist. Die Verbindungen zeigen jedoch gegenüber grampositiven Bakterien eine zu geringe Wirksamkeit.

Es wurden Verbindungen der Formel (I) gefunden

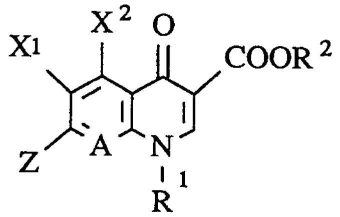

(I),

in welcher

$X^1$ für Halogen,

$X^2$ für Wasserstoff, Amino, Alkylamino mit 1 bis 4 Kohlenstoffatomen, Dialkylamino mit 1 bis 3 Kohlenstoffatomen je Alkylgruppe, Hydroxy, Alkoxy mit 1 bis 4 Kohlenstoffatomen, Mercapto, Alkylthio mit 1 bis 4 Kohlenstoffatomen, Arylthio, Halogen oder Methyl,

$R^1$ für Alkyl mit 1 bis 4 Kohlenstoffatomen, Alkenyl mit 2 bis 4 Kohlenstoffatomen, Cycloalkyl mit 3 bis 6 Kohlenstoffatomen, Bicyclo[1.1.1]pent-1-yl, 1,1-Dimethylpropargyl, 3-Oxetanyl, 2-Hydroxyethyl, 2-Fluorethyl, Methoxy, Amino, Methylamino, Ethylamino, Dimethylamino, gegebenenfalls durch 1 oder 2 Fluoratome substituiertes Phenyl,

$R^2$ für Wasserstoff, gegebenenfalls durch Hydroxy, Methoxy, Amino, Methylamino oder Dimethylamino substituiertes Alkyl mit 1 bis 5 Kohlenstoffatomen oder (5-Methyl-2-oxo-1,3-dioxol-4-yl)-methyl,

A für N oder C-$R^5$ steht, worin

$R^5$ für Wasserstoff, Halogen, Methyl, Alkenyl mit 2 bis 3 Kohlenstoffatomen, Alkinyl mit 2 bis 3 Kohlenstoffatomen, Hydroxy oder Methoxy steht oder auch gemeinsam mit $R^1$ eine Brücke der Struktur

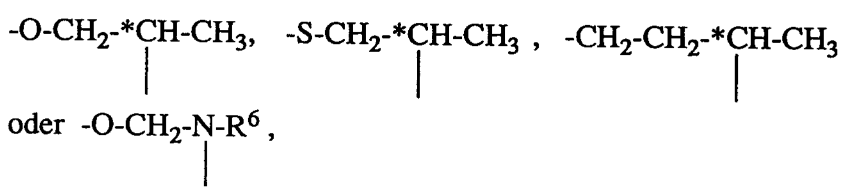

worin $R^6$ Wasserstoff, Methyl oder Formyl bedeutet,
bilden kann, und

Z für einen Rest der Struktur

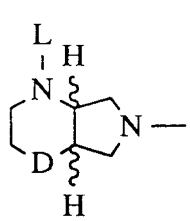

oder

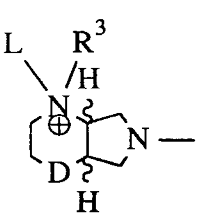

steht, worin

D für $CH_2$ oder O,

$R^3$ für Methyl oder Ethyl und

L für einen Rest der Struktur

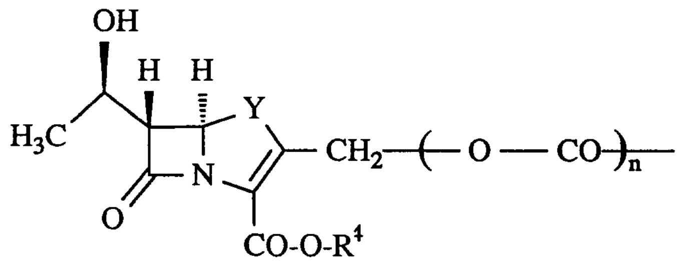

oder

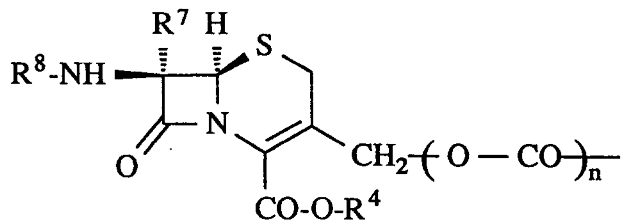

steht, worin

- n für 0 oder 1,
- Y für $CH_2$, $CH\text{-}CH_3$ oder S,
- $R^4$ für H, Benzyl, 4-Methoxybenzyl, Benzhydryl, Allyl, (5-Methyl-2-oxo-1,3-dioxol-4-yl)-methyl oder einen Rest

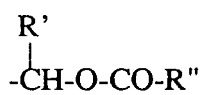

steht,
in welchem

- R' H oder Methyl und
- R" Ethoxy oder tert.-Butyl bedeutet,
- $R^7$ für H oder Methoxy und
- $R^8$ für H, Tri-($C_1$-$C_4$-alkyl)-silyl, Acyl, $C_1$-$C_4$-Alkoxycarbonyl, Benzyloxycarbonyl, Allyloxycarbonyl, Isopropenyloxycarbonyl,

steht,
und deren pharmazeutisch verwendbaren Hydrate und Säureadditionssalze sowie die Alkali-, Erdalkali-, Silber- und Guanidiniumsalze der zugrundeliegenden Carbonsäuren. Sie weisen eine hohe antibakterielle Wirkung insbesondere gegenüber grampositiven Bakterien auf.

Bevorzugt sind die Verbindungen der Formel (I),
in welcher

- $X^1$ für Fluor,
- $X^2$ für Wasserstoff, Amino, Methylamino, Hydroxy, Methoxy, Fluor, Chlor, Brom oder Methyl,
- $R^1$ für Alkyl mit 1 bis 3 Kohlenstoffatomen, Vinyl, Cycloalkyl mit 3 bis 4 Kohlenstoffatomen, gegebenenfalls durch 1 oder 2 Fluoratome substituiertes Phenyl,
- $R^2$ für Wasserstoff, gegebenenfalls durch Amino, Methylamino oder Dimethylamino substituiertes Alkyl mit 1 bis 2 Kohlenstoffatomen oder (5-Methyl-2-oxo-1,3-dioxol-4-yl)-methyl,
- A für N oder $C\text{-}R^5$ steht, worin
- $R^5$ für Wasserstoff, Fluor, Chlor, Methyl, Vinyl, Ethinyl oder Methoxy steht oder auch gemeinsam mit $R^1$ eine Brücke der Struktur

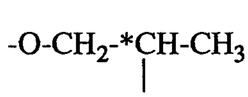 oder 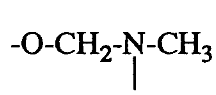

bilden kann und

Z für einen Rest der Struktur

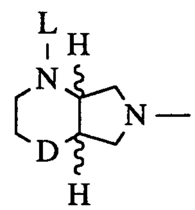

oder

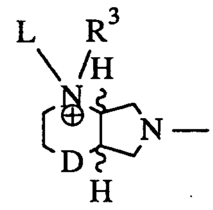

steht, worin

D für $CH_2$ oder O,

$R^3$ für Methyl und

L für einen Rest der Struktur

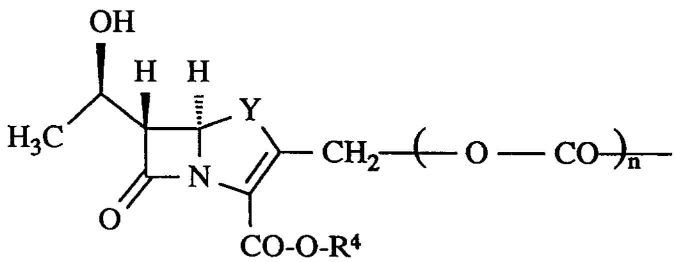

oder

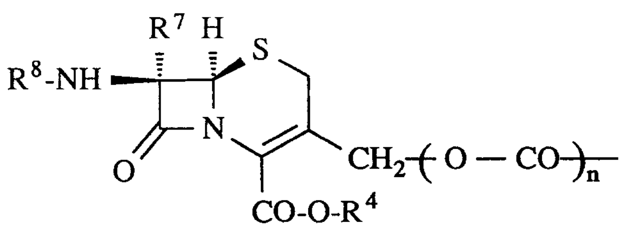

steht, worin

n für 0 oder 1,

Y für $CH_2$ oder S,

$R^4$ für H, Benzhydryl, Allyl, oder einen Rest

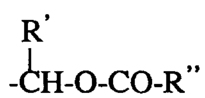

steht, in welchem

R' H oder Methyl und

R" tert.-Butyl bedeutet,

$R^7$ für H und

$R^8$ für H, $(CH_3)_3Si$-,

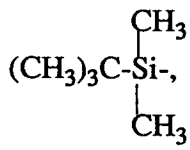

tert.-Butoxycarbonyl, Benzyloxycarbonyl, Allyloxycarbonyl, Isopropenyloxycarbonyl,

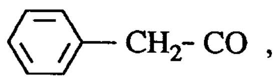

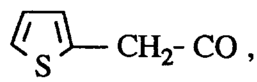

$NC\text{-}CH_2CO\text{-}$ ,

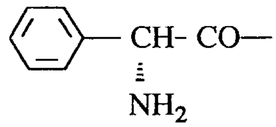 ,

$NC\text{-}CH_2S\text{-}CH_2CO\text{-}$,

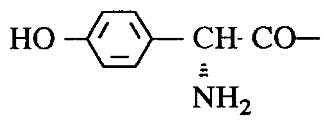 ,

$F_2CH\text{-}S\text{-}CH_2CO\text{-}$,

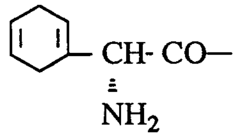 ,

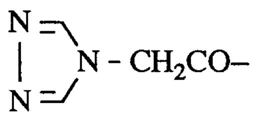 ,

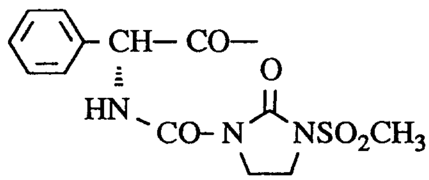 ,

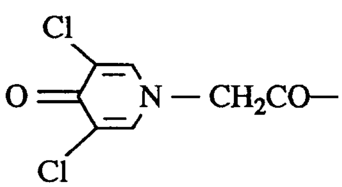 ,

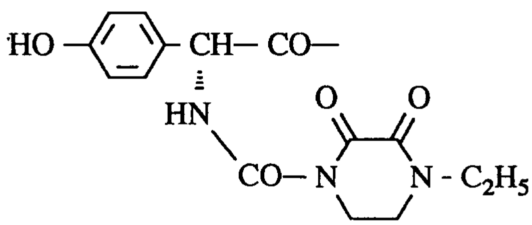 ,

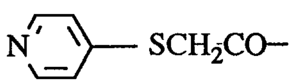 ,

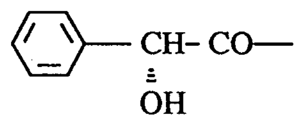 ,

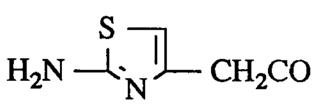 ,

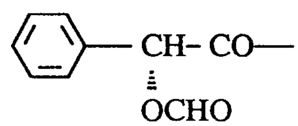 ,

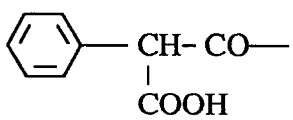 ,

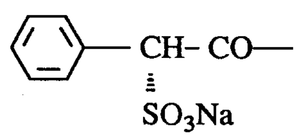 ,

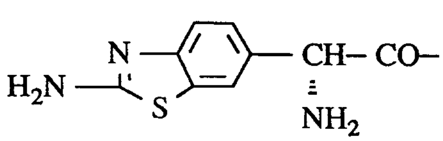 ,

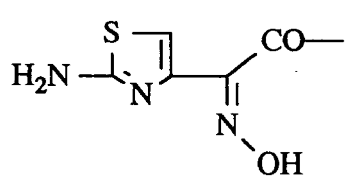 ,

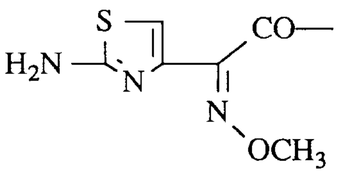 , 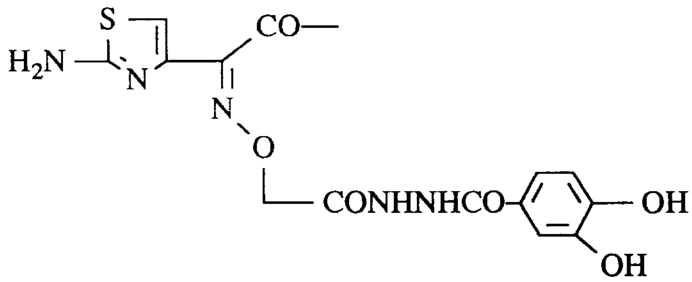 ,

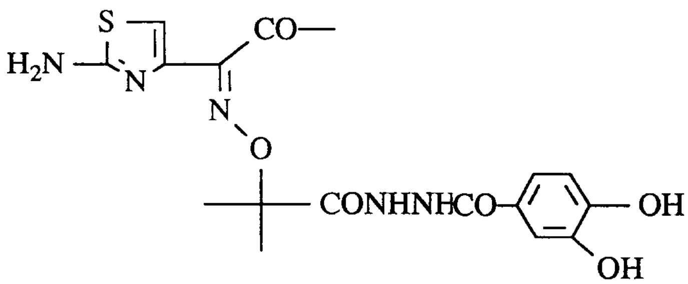 ,

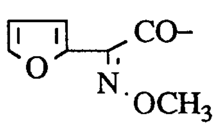 , 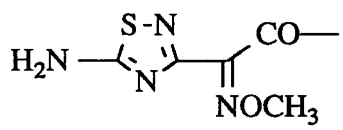 ,

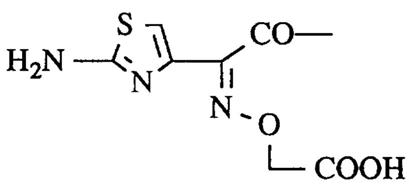 , 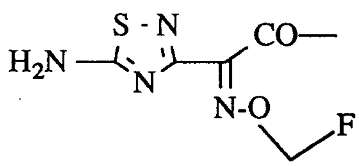 ,

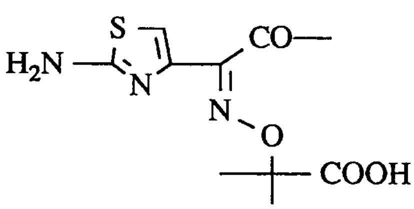 , 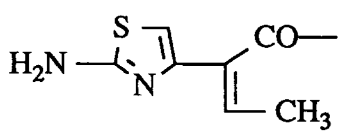 ,

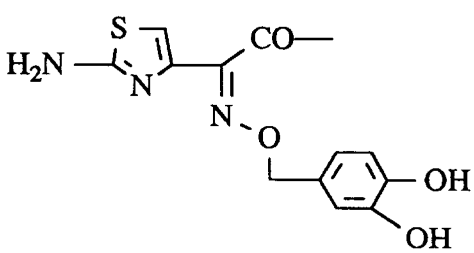 , 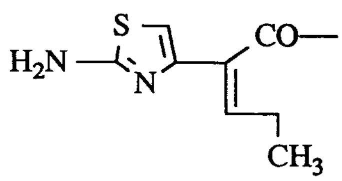 ,

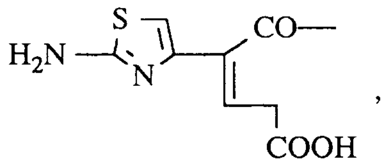

steht,

und deren pharmazeutisch verwendbaren Hydrate und Säureadditionssalze sowie die Alkali-, Erdalkali-, Silber- und Guanidiniumsalze der zugrundeliegenden Carbonsäuren.

Besonders bevorzugt sind die Verbindungen der Formel (I),

in welcher

- $X^1$ für Fluor,
- $X^2$ für Wasserstoff, Amino oder Fluor,
- $R^1$ für Alkyl mit 1 bis 2 Kohlenstoffatomen, Cyclopropyl, gegebenenfalls durch 1 oder 2 Fluoratome substituiertes Phenyl,
- $R^2$ für Wasserstoff oder Alkyl mit 1 bis 2 Kohlenstoffatomen,
- A für N oder C-$R^5$ steht,

  worin
- $R^5$ für Wasserstoff, Fluor, Chlor oder Methoxy steht oder auch gemeinsam mit $R^1$ eine Brücke der Struktur

$$-O-CH_2-{}^*CH-CH_3$$

  bilden kann,
- Z für einen Rest der Struktur

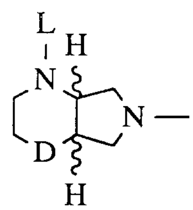

  steht, worin
- D für $CH_2$ oder O,
- L für einen Rest der Struktur

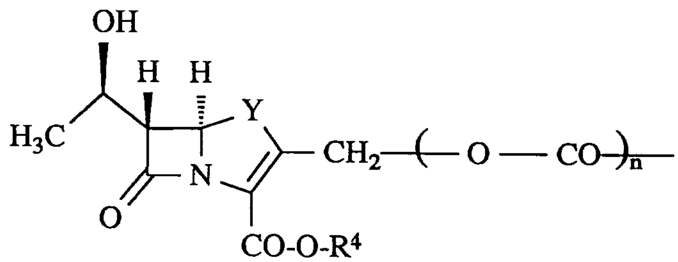

oder

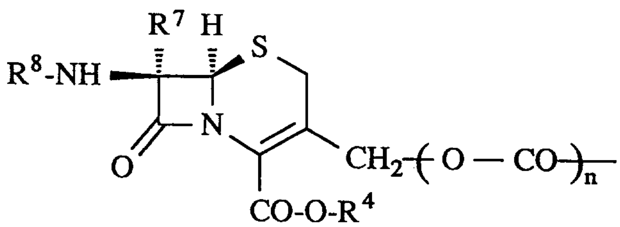

steht, worin

n für 0 oder 1,

Y für S,

$R^4$ für H, Benzhydryl, Allyl,

$R^7$ für H und

$R^8$ für H, $(CH_3)_3Si$-,

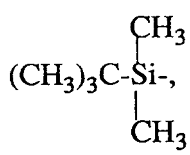

tert.-Butoxycarbonyl, Allyloxycarbonyl,

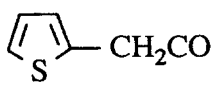 , 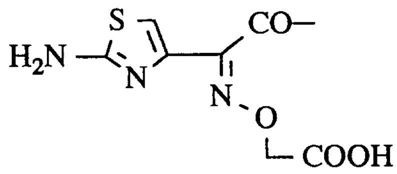 ,

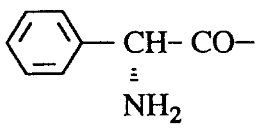 , 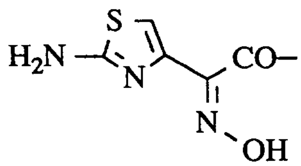 ,

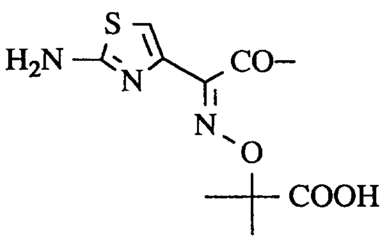 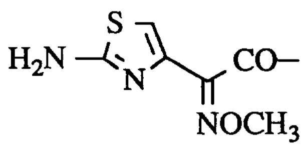

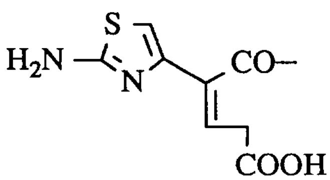

steht,

und deren pharmazeutisch verwendbaren Hydrate und Säureadditionssalze sowie die Alkali-, Erdalkali-, Silber- und Guanidiniumsalze der zugrundeliegenden Carbonsäuren.

Weiterhin wurde gefunden, daß man die Verbindungen der Formel (I) erhält, wenn man eine Verbindung der Formel (II)

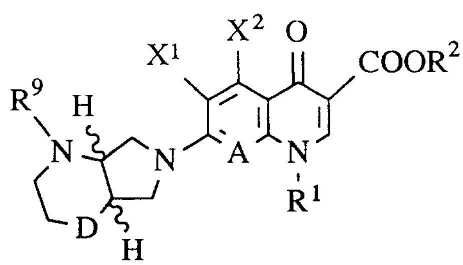 (II)

in welcher

| | |
|---|---|
| A, $R^1$, $R^2$, $X^1$, $X^2$ und D | die oben angegebene Bedeutung haben, und |
| $R^9$ | für H, $CH_3$ oder $C_2H_5$ steht, |

mit Verbindungen der Formel (III)

$L-X^3$ (III),

in welcher

| | |
|---|---|
| L | die oben angegebene Bedeutung hat und |
| $X^3$ | für Halogen, insbesondere Chlor, Brom oder Jod, oder Acetoxy steht, |

gegebenenfalls in Gegenwart von Säurefängern umsetzt und gegebenenfalls vorhandene Schutzgruppen abspaltet.

Verwendet man beispielsweise 8-Chlor-1-cyclopropyl-7-([S,S]-2,8-diazabicyclo[4.3.0]non-8-yl)-6-fluor-1,4-dihydro-4-oxo-3-chinolincarbonsäure (2) und (6R, 7R) 3-Chlormethyl-8-oxo-7-phenylacetylamino-5-thia-1-aza-bicyclo[4.2.0]oct-2-en-2-carbonsäurebenzhydrylester (1) als Ausgangsstoffe, so kann der Reaktionsverlauf durch folgendes Formelschema wiedergegeben werden:

Schema 1:

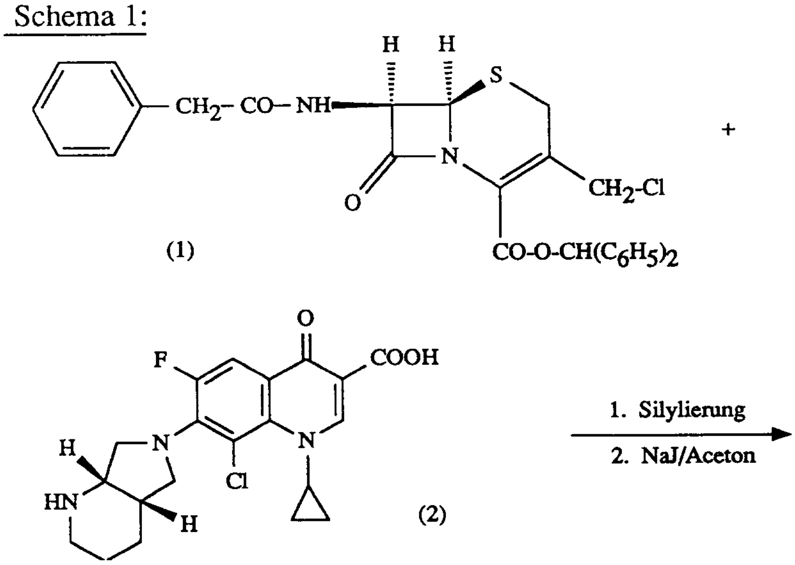


(1) + (2)

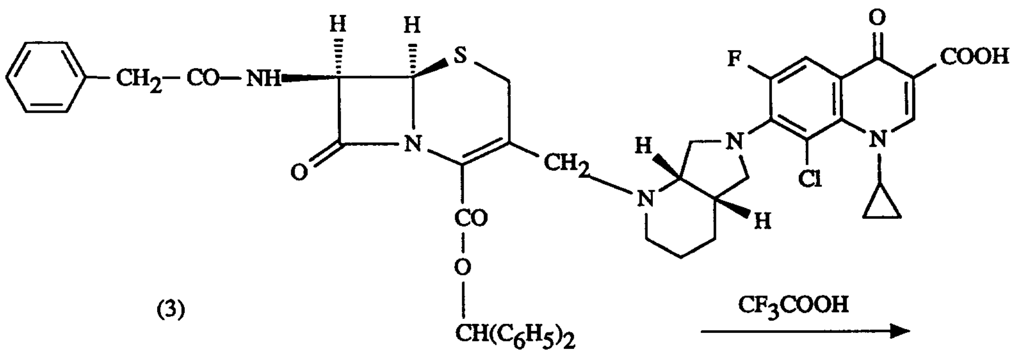


(3)

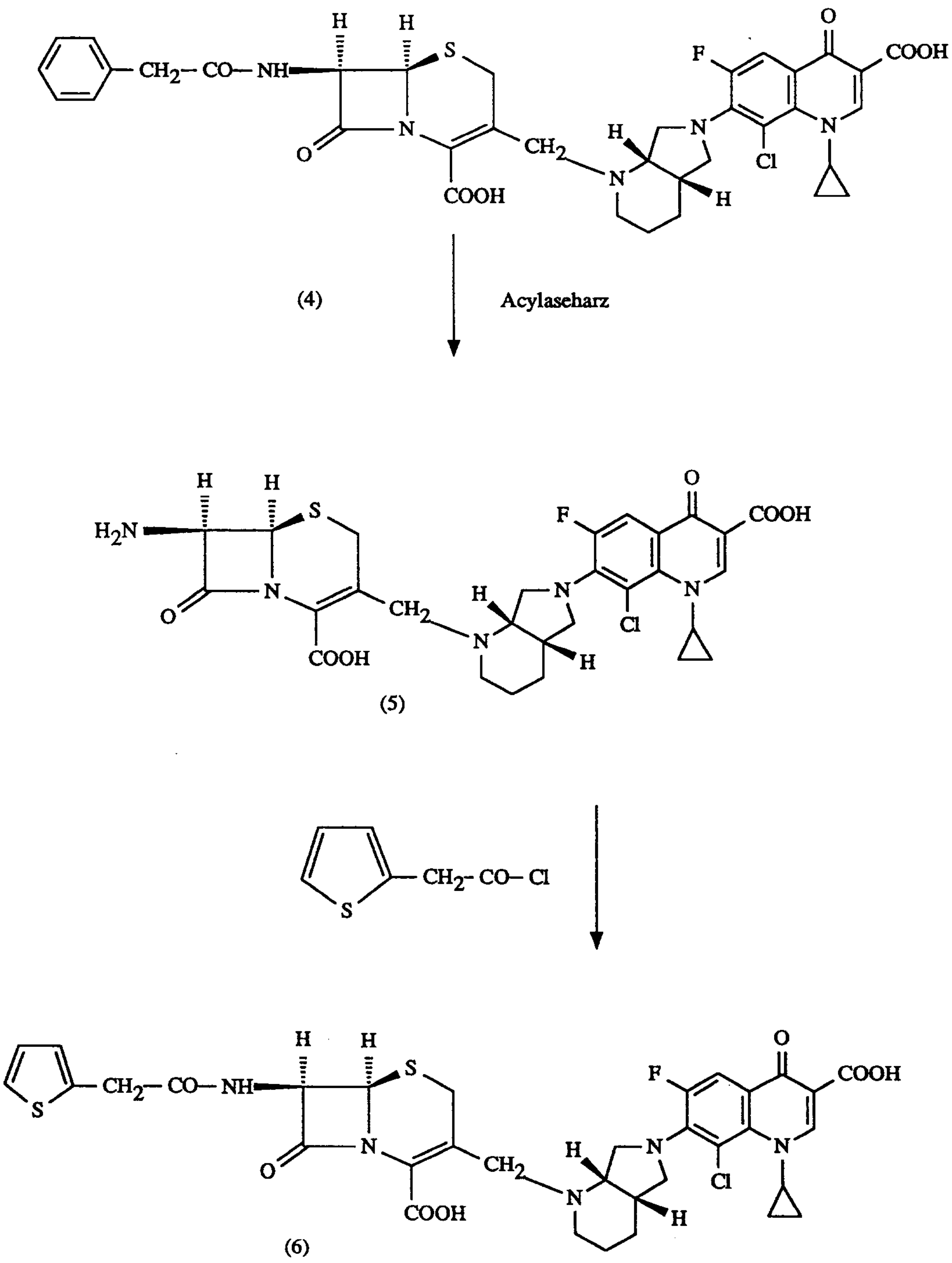


(4)

(5)

(6)

Der Wirkstoff vom Typ der Formel (4) kann beispielsweise mit einer Acylase zur Verbindung (5) deacyliert werden. Diese 7-Aminocephalosporansäure (5) ist ein geeignetes Ausgangsprodukt für die Verknüpfung mit verschiedenen Acylresten zu neuen Wirkstoffen vom Typ (6), in denen der Chinolonanteil auch durch andere Chinolonreste der vorliegenden Erfindung ersetzt sein kann.

Verwendet mann beispielsweise 6-(1-tert-Butyl-dimethyl-silyloxy-ethyl)-3-hydroxymethyl-7-oxo-4-thia-1-aza-bicyclo[3.2.0]hept-2-en-2-carbonsäure-allylester (7) und die Chinonloncarbonsäure (2) als Ausgangsverbindungen, so kann der Reaktionsverlauf durch folgendes Formelschema wiedergegeben werden:

Schema 2 :

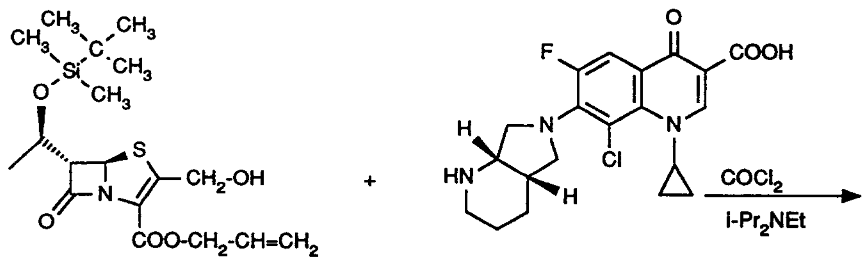


(7) (2)

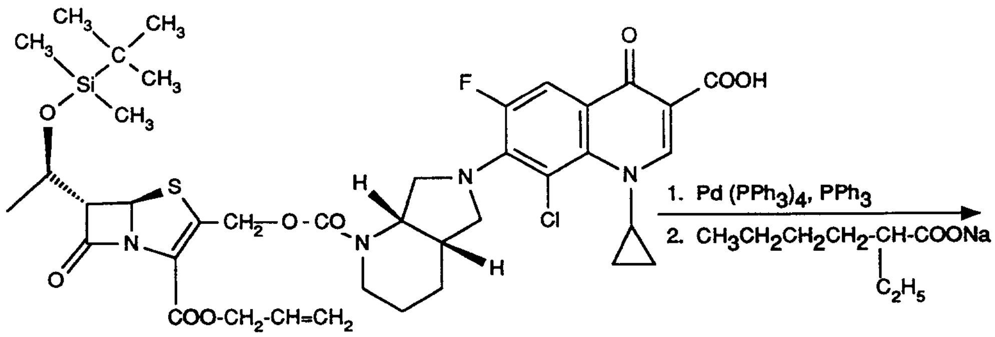


(8)

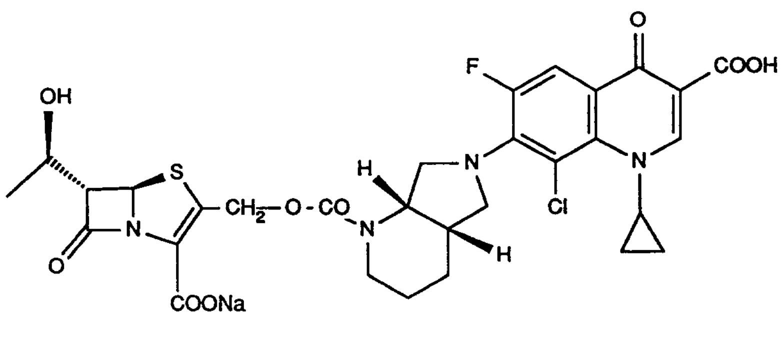

(9)

Verwendet man beispielsweise 3-Acetoxymethyl-7-[2-(2-tritylamino-thiazol-4-yl)-2-methoxyimino-acetylamino]-8-oxo-5-thia-1-aza-bicylo[4.2.0}oct-2-en-2-carbonsäure (10) und die Chinoloncarbonsäure (2) als Ausgangsverbindungen, so kann der Reaktionsverlauf durch folgendes Formelschema wiedergegeben werden:

Schema 3:

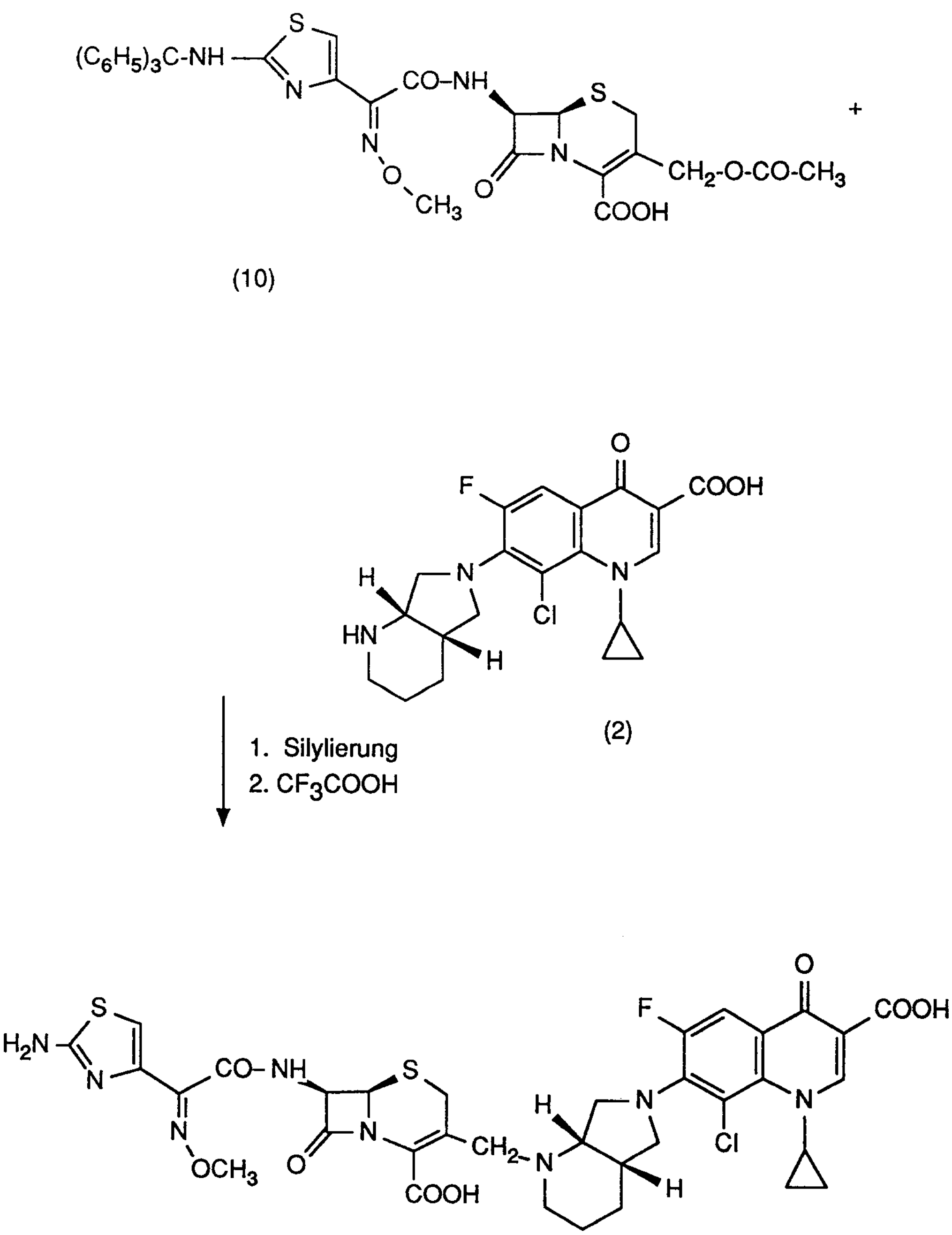


(10)

(2)

(11)

Man kann beispielsweise auch von 7-Amino-3-chlormethyl-8-oxo-5-thia-1-aza-bicyclo[4.2.0]oct-2-en-2-carbonsaurebenzhydrylester (12) ausgehen, der nach Acylierung mit einem aktivierten Carbonsäurederivat zu (13) beispielsweise mit der Chinolincarbonsäure (2) zu (14) umgesetzt werden kann. Nach Abspaltung der Esterschutzgruppen wird (15) erhalten:

Schema 4:

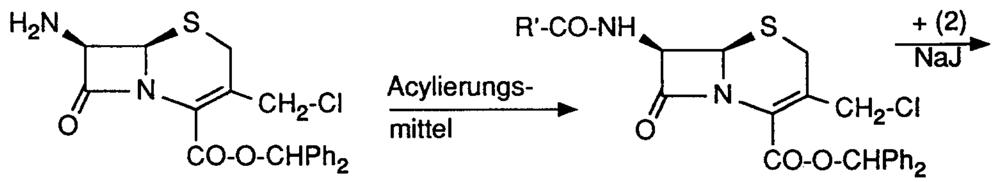


(12) (13)

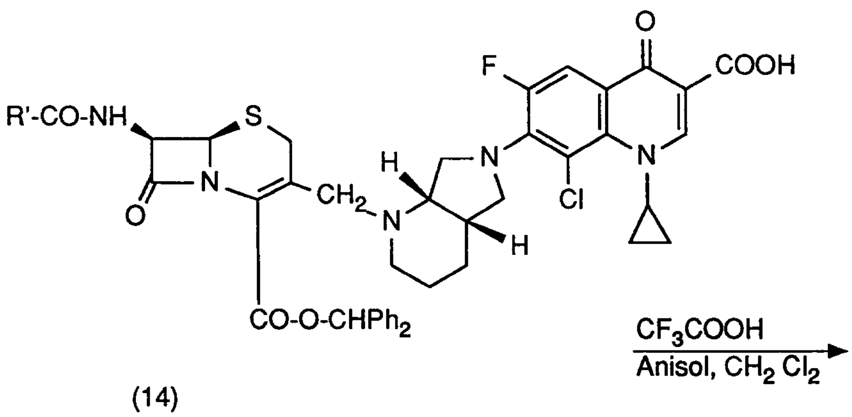


(14)

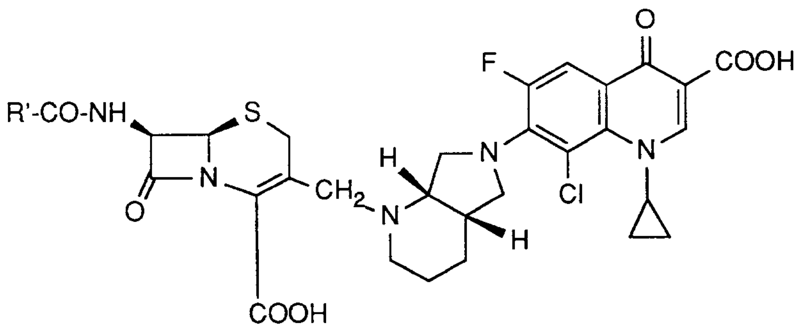

(15)

R' = 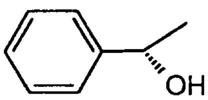 , 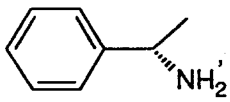 , 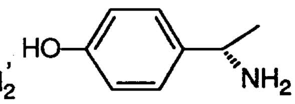 ,

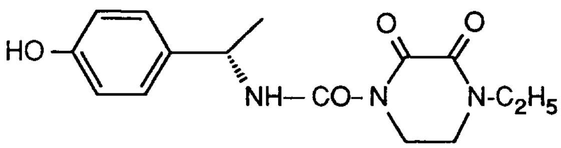 , 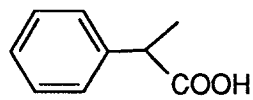

Setzt man nach der in Schema 4 angegebenen Reaktionsfolge die Verbindung (12) mit 2-(2-Amino-thiazol-4-yl)-2-oxo-thioessigsäure-benzothiazol-2-yl-S-ester (16), so erhält man ein Reaktionsprodukt (17), das mit Hydroxylaminderivaten (18) zu den Wirkstoffen (19) kondensiert werden kann:

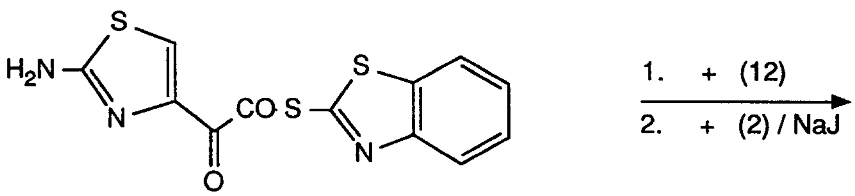


(16)

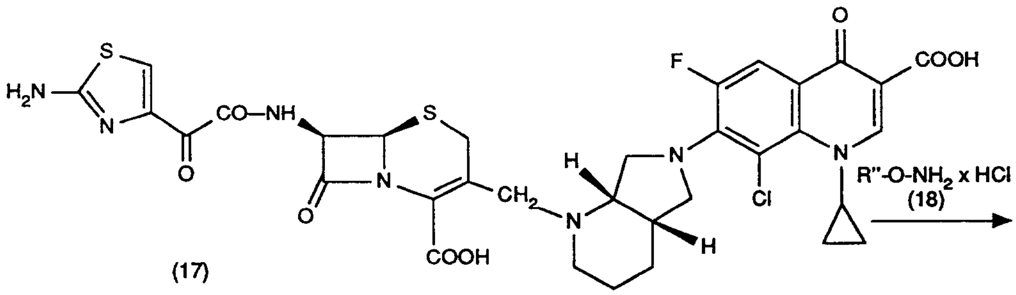


(17)

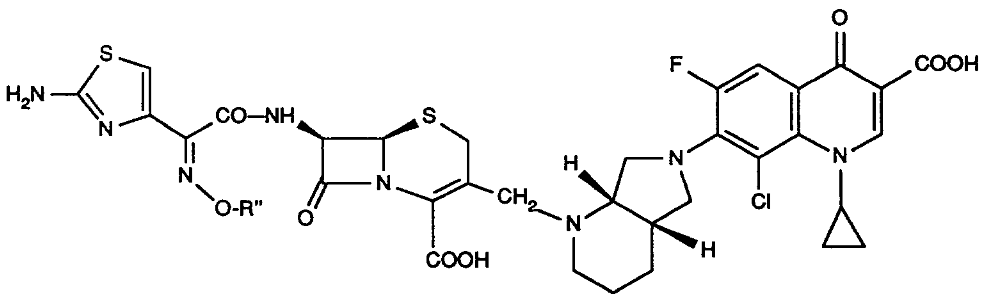

(19)

$$R'' = H,\ CH_3,\ CH_2\text{-}COOH,\quad -\underset{CH_3}{\overset{CH_3}{\underset{|}{\overset{|}{C}}}}-COOH$$

Die Verknüpfung eines Carbapenems mit dem Chinolon kann beispielsweise auch gemäß dem folgenden Formelschema erfolgen:

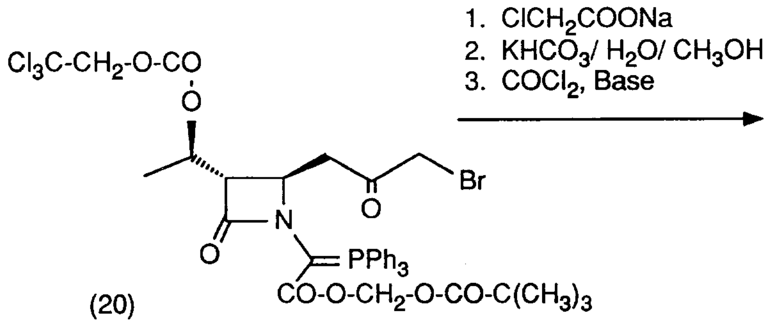
1. $ClCH_2COONa$
2. $KHCO_3$/ $H_2O$/ $CH_3OH$
3. $COCl_2$, Base

(20)

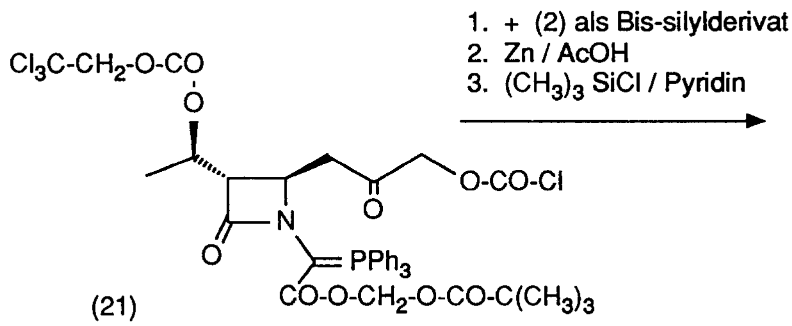
1. + (2) als Bis-silylderivat
2. Zn / AcOH
3. $(CH_3)_3$ SiCl / Pyridin

(21)

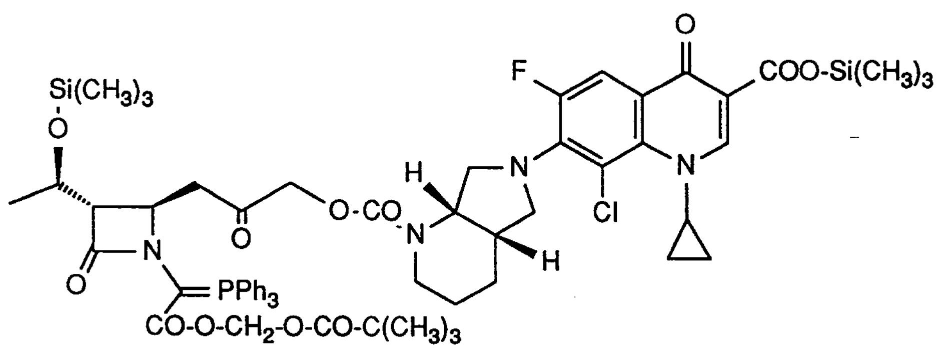

(22)

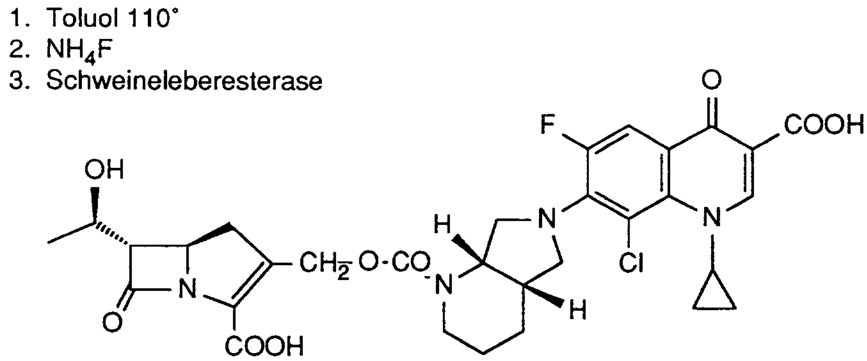


(23)

Die Ausgangsverbindungen der Formel (I) sind als racemische Verbindungen teilweise aus der EP-A-0 350 733 bekannt, Enantiomerenreine Ausgangsverbindungen der Formel (II) sind neu.

Zur Herstellung von enantiomerenreinen Zwischenverbindungen der Formel (II) setzt man eine Verbindung der Formel (IV)

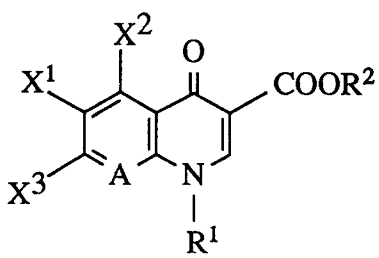

(IV),

in welcher

A, $R^1$, $R^2$, $X^1$ und $X^2$ die oben angegebene Bedeutung haben und

$X^3$ für Halogen, insbesondere Fluor oder Chlor steht,

mit enantiomerenreinen Verbindungen der Formel (V)

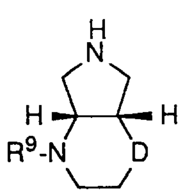 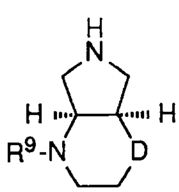 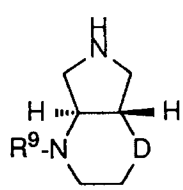 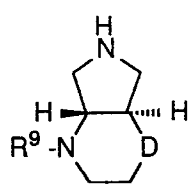 (V)

in welcher

D für O oder $CH_2$ und

$R^9$ für H, $CH_3$ oder $C_2H_5$ steht,

gegebenenfalls in Gegenwart von Säurefängern um.

Verwendet man beispielsweise 8-Chlor-1-cyclopropyl-6,7-difluor-1,4-dihydro-4-oxo-3-chinolincarbonsäure und [S,S]-2,8-Diazabicyclo[4.3.0]nonan als Ausgangsverbindungen, so kann der Reaktionsverlauf durch folgendes Formelschema wiedergegeben werden:

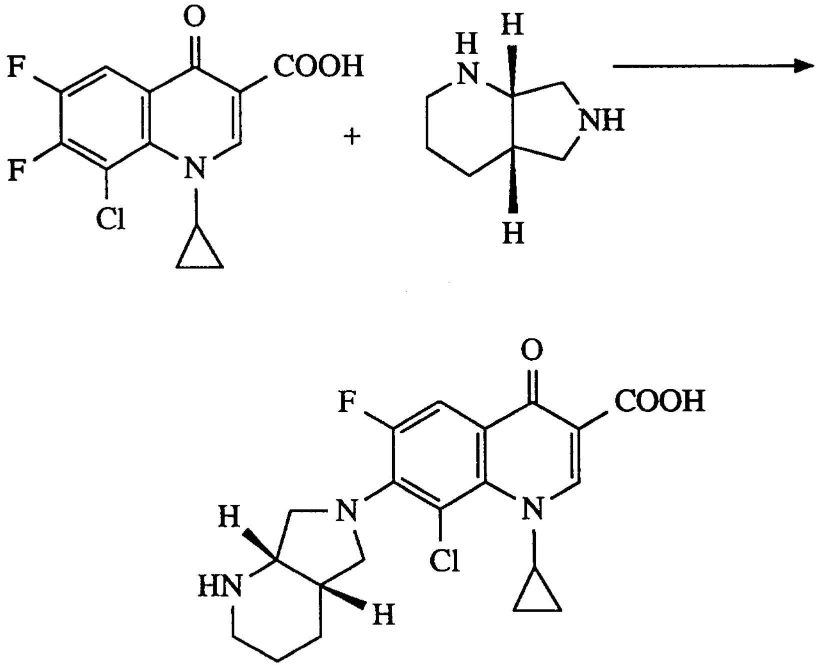

Die bicyclischen Amine (V) sind als enantiomerenreine Verbindungen neu. Sie können nach folgenden Verfahren hergestellt werden:

1. Die racemischen bicyclischen Amine (a)

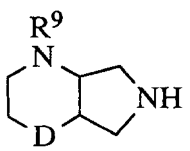

(a)

in welcher

D für O oder $CH_2$ und

$R^9$ für H, $CH_3$ oder $C_2H_5$ steht,

können mit enantiomerenreinen Säuren, z.B. Carbonsäuren oder Sulfonsäuren wie N-Acetyl-L-glutaminsäure, N-Benzoyl-L-alanin, 3-Bromcampher-9-sulfonsäure, Campher-3-carbonsäure, cis-Camphersäure, Campher-10-sulfonsäure, O,O'-Dibenzoyl-weinsäure, D- oder L-Weinsäure, Mandelsäure, α-Methoxyphenylessigsäure, 1-Phenyl-ethansulfonsäure, α-Phenyl-bernsteinsäure zu einem Gemisch der diastereomeren Salze umgesetzt werden, die sich durch fraktionierte Kristallisation zu den diastereomerenreinen Salzen trennen lassen (siehe P.Newman, Optical Resolution Procedures for Chemical Compounds, Volume 1). Das molare Verhältnis zwischen Amin und enantiomerenreiner Säure kann in einem größeren Bereich variiert werden. Durch Behandlung dieser Salze mit Alkali- oder Erdalkalihydroxiden lassen sich die enantiomerenreinen Amine freisetzen.

2. In ähnlicher Weise, wie unter 1. beschrieben, läßt sich eine Racematspaltung der basischen Zwischenstufen, die bei der Herstellung der racemischen bicyclischen Amine auftreten, mit den oben aufgeführten enantiomerenreinen Säuren durchführen. Beispiele für derartige basische Zwischenstufen sind:

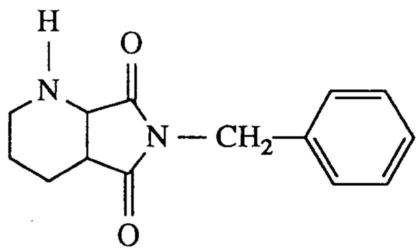 (b)

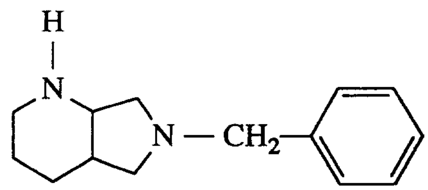 (c)

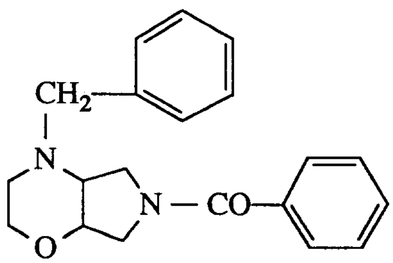 (d)

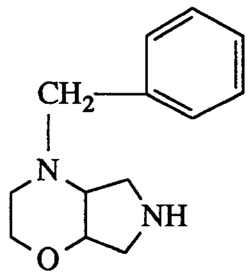 (e)

Im folgenden Formelschema sei als Beispiel für eine Racematspaltung die Auftrennung von 8-Benzyl-cis-2,8-diazabicyclo[4.3.0]nonan über die Tartrate in die Enantiomeren und deren Überführung in die enantiomerenreinen cis-2,8-Diazabicyclo[4.3.0]nonane dargestellt:

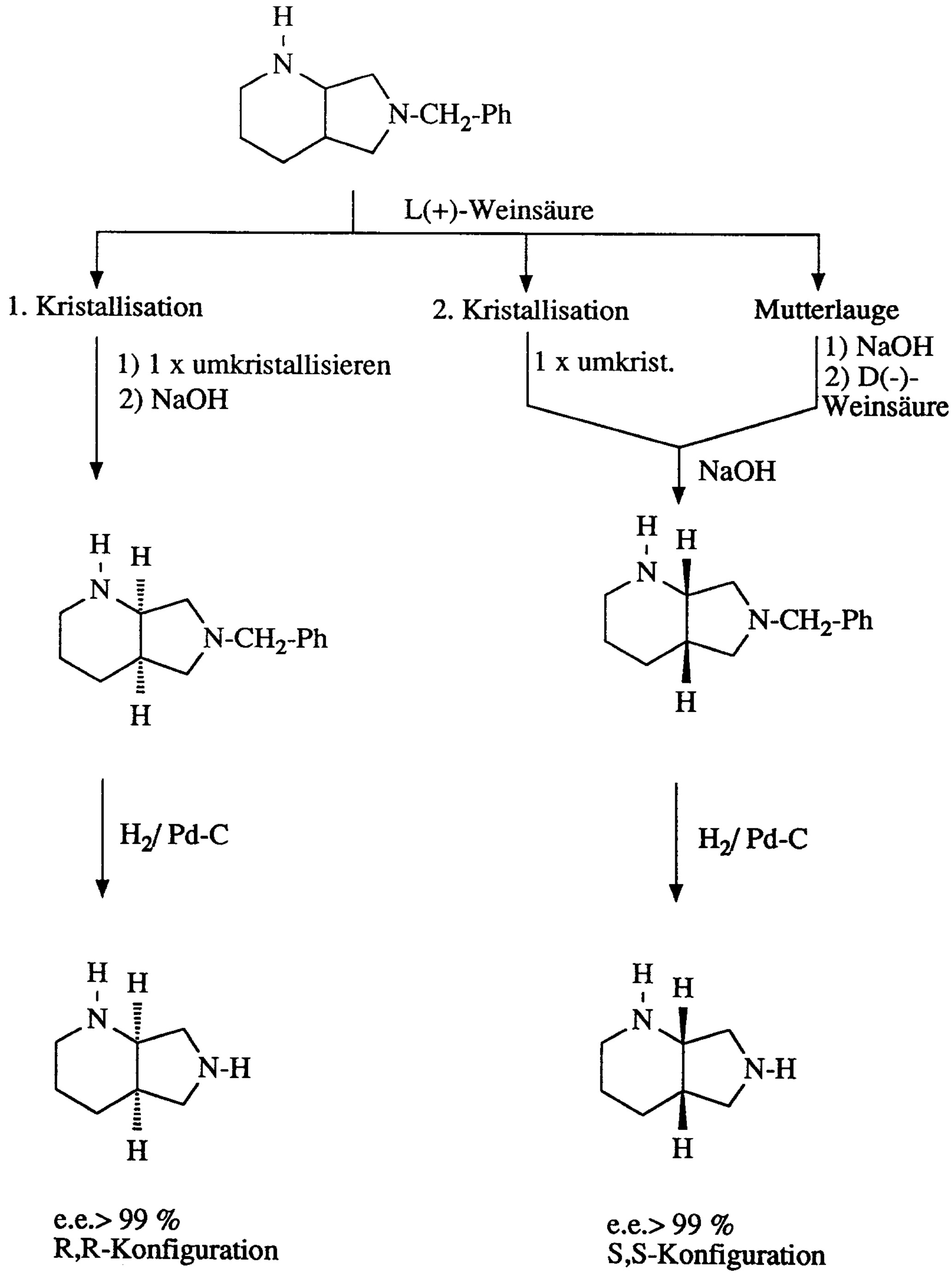


3. Sowohl die racemischen Amine (a) als auch die basischen Zwischenstufen (b) - (e) können, gegebenenfalls nach Acylierung, über chirale Trägermaterialien chromatographisch getrennt werden (siehe z.B. G. Blaschke, Angew. Chem. 92, 14 [1980]).

4. Sowohl die racemischen Amine (a) als auch die basischen Zwischenstufen (b), (c), (e) können durch chemische Verknüpfung mit chiralen Acylresten in Diastereomerengemische überführt werden, die sich durch Destillation, Kristallisation oder Chromatographie in die diastereomerenreinen Acylderivate trennen lassen, aus denen sich durch Verseifung die enantiomerenreinen Amine isolieren lassen. Beispiele für Reagentien zur Verknüpfung mit chiralen Acylresten sind: α-Methoxy-α-trifluormethyl-phenylacetylchlorid, Menthylisocyanat, D- oder L-α-Phenyl-ethyl-isocyanat, Chlorameisensäurementhylester, Campher-10-sulfonsäurechlorid.

5. Im Verlauf der Synthese der bicyclischen Amine (a) können statt achiraler auch chirale Schutzgruppen eingeführt werden. Man gelangt auf diese Weise zu Diastereomeren, die sich trennen lassen. Zum Beispiel kann bei der Synthese von cis-2,8-Diazabicyclo[4.3.0]nonan der Benzylrest durch den R- oder S-konfigurierten α-Phenylethylrest ersetzt werden:

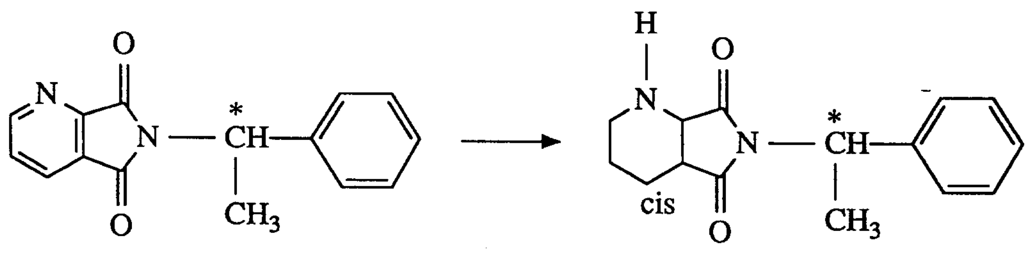

6. Die enantiomerenreinen Amine (VI) können auch aus enantiomerenreinen Vorstufen, wie z.B. [R,R]- oder [S,S]-3,4-Dihydroxypyrrolidin, das am Stickstoff durch eine Schutzgruppe geschützt sein sollte, aufgebaut werden.

Als Beispiel für die Synthese eines enantiomerenreinen Amins, ausgehend von enantiomerenreinem 1-Benzyl-3,4-dihydroxy-pyrrolidin, sei folgendes Formelschema angegeben:

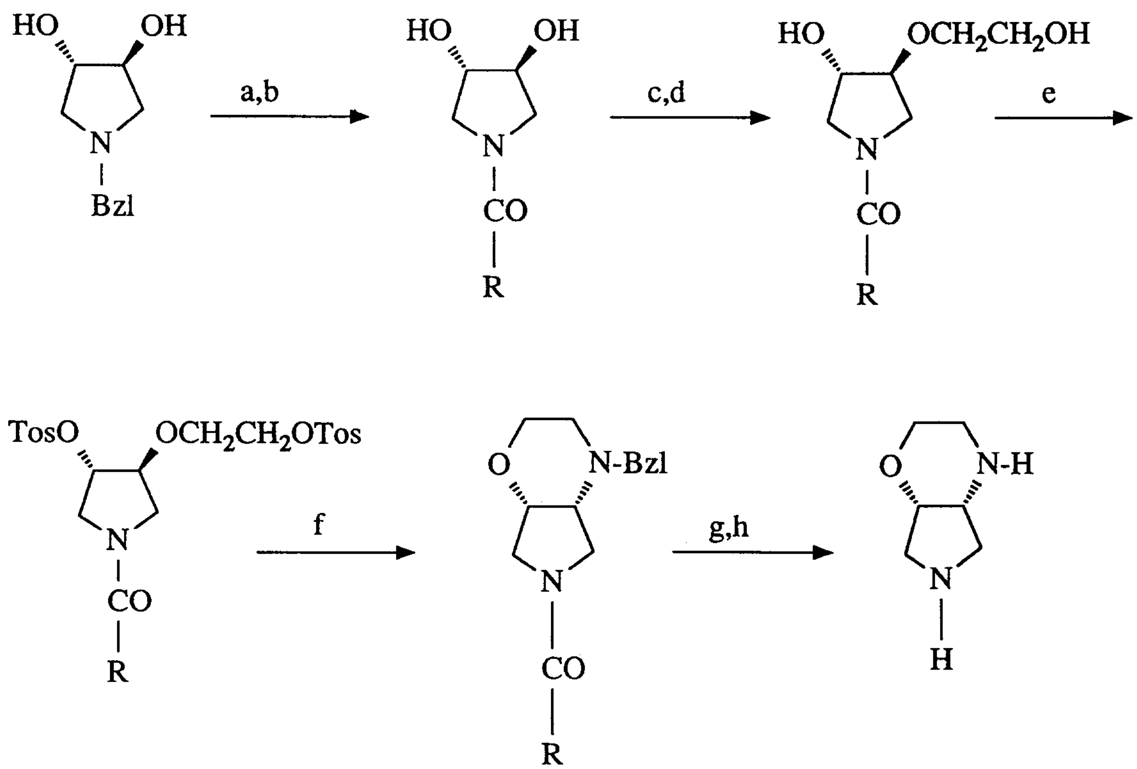


| | | | | |
|---|---|---|---|---|
| R = | zum Beispiel $(CH_3)_3C$-O, | | | |
| a: | $H_2$, Pd/A-Kohle | | | |
| b: | Acylierung | | | |
| c: | NaH, $BrCH_2COOC_2H_5$ | oder | c: | $CH_2$=CH-$CH_2$Br, NaH, |
| d: | $LiBH_4$ | | d: | $O_3$, $NaBH_4$, |
| e: | Tosylchlorid, $NEt_3$, | | | |
| f: | Benzylamin, Xylol, Rückfluß | | | |
| g: | Hydrolyse | | | |
| h: | $H_2$, Pd/A-Kohle | | | |

Als Beispiele für Verbindungen der Formel (V) seien genannt: cis-2,8-Diazabicyclo[4.3.0]nonan,
cis-2-Oxa-5,8-diazabicyclo[4.3.0]nonan,
trans-2-Oxa-5,8-diazabicyclo[4.3.0]nonan,
S,S-2,8-Diazabicyclo[4.3.0]nonan,
1R,6S-2-Oxa-5,8-diazabicyclo[4.3.0]nonan,
1S,6R-2-Oxa-5,8-diazabicyclo[4.3.0]nonan,
1R,6R-2-Oxa-5,8-diazabicyclo[4.3.0]nonan,
1S,6S-2-Oxa-5,8-diazabicyclo[4.3.0]nonan.

Die Umsetzung von (IV) mit (V), bei der die Verbindungen (V) auch in Form ihrer Salze, wie z.B. der Hydrochloride eingesetzt werden können, wird vorzugsweise in einem Verdünnungsmittel wie Dimethylsulfoxid, N,N-Dimethylformamid, N-Methylpyrrolidon, Hexamethyl-phosphorsäuretrisamid, Sulfolan, Acetonitril, Wasser, einem Alkohol wie Methanol, Ethanol, n-Propanol, Isopropanol, Glykolmonomethylether oder Pyridin vorgenommen. Ebenso können Gemische dieser Verdünnungsmittel verwendet werden.

Als Säurebinder können alle üblichen anorganischen und organischen Säurebindungsmittel verwendet werden. Hierzu gehören vorzugsweise die Alkalihydroxide, Alkalicarbonate, organische Amine und Amidine. Als besonders geeignet seien im einzelnen genannt: Triethylamin, 1,4-Diazabicyclo[2.2.2]octan (DABCO),

1,8-Diazabicyclo[5.4.0]undec-7-en (DBU) oder überschüssiges Amin (V).

Die Reaktionstemperaturen können in einem größeren Bereich variiert werden. Im allgemeinen arbeitet man zwischen etwa 20 und 200 °C, vorzugsweise zwischen 80 und 180 °C.

Die Umsetzung kann bei Normaldruck, aber auch bei erhöhtem Druck durchgeführt werden. Im allgemeinen arbeitet man bei Drucken zwischen etwa 1 und 100 bar, vorzugsweise zwischen 1 und 10 bar.

Bei der Durchführung dieses Verfahrens setzt man auf 1 Mol der Verbindung (IV) 1 bis 15 Mol, vorzugsweise 1 bis 6 Mol der Verbindung (V) ein.

Als Beispiele für Zwischenverbindungen der Formel (II), die sowohl als Racemate als auch als enantiomerenreine oder diasteromerenreine Verbindungen verwendet werden können, seien genannt:

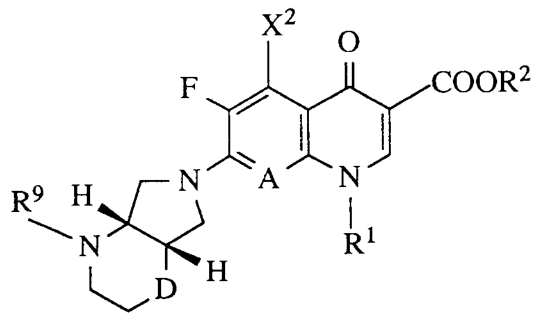

(II)

| $R^1$ | $R^2$ | $R^9$ | $X^2$ | D | A |
|---|---|---|---|---|---|
| Cyclopropyl | $C_2H_5$ | H | H | $CH_2$ | C-H |
| $F-CH_2CH_2$ | H | H | H | $CH_2$ | C-F |
| Cyclopropyl | $C_2H_5$ | H | H | $CH_2$ | C-Cl |
| Cyclopropyl | H | H | H | $CH_2$ | $C-OCH_3$ |
| Cyclopropyl | H | H | H | $CH_2$ | $C-CH_3$ |
| Cyclopropyl | $C_2H_5$ | H | H | $CH_2$ | N |
| Cyclopropyl | H | H | Br | $CH_2$ | C-F |
| Cyclopropyl | H | H | Cl | $CH_2$ | C-F |
| Cyclopropyl | H | H | $CH_3$ | $CH_2$ | C-F |
| Cyclopropyl | $C_2H_5$ | H | $NH_2$ | $CH_2$ | C-F |
| Cyclopropyl | H | $CH_3$ | H | $CH_2$ | C-H |
| Cyclopropyl | $C_2H_5$ | $CH_3$ | H | $CH_2$ | C-F |
| Cyclopropyl | H | $CH_3$ | H | $CH_2$ | C-Cl |
| $C_2H_5$ | H | $C_2H_5$ | H | $CH_2$ | C-F |
| Cyclopropyl | H | H | H | O | $C-CH_3$ |
| Cyclopropyl | H | H | H | O | N |
| Cyclopropyl | H | H | Br | O | C-F |
| Cyclopropyl | H | H | Cl | O | C-F |
| Cyclopropyl | H | H | $CH_3$ | O | C-F |
| Cyclopropyl | H | H | $NH_2$ | O | C-F |

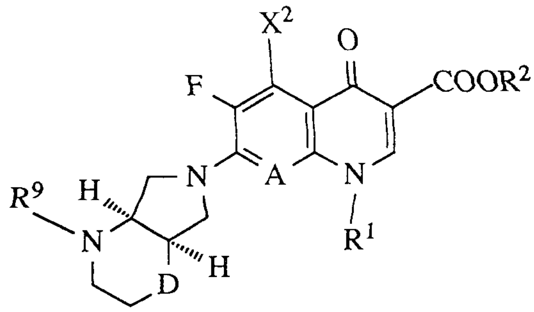

(II)

| $R^1$ | $R^2$ | $R^9$ | $X^2$ | D | A |
|---|---|---|---|---|---|
| Cyclopropyl | $CH_3$ | H | H | $CH_2$ | C-H |
| Cyclopropyl | $CH_2CH_2F$ | H | H | $CH_2$ | C-F |
| Cyclopropyl | $CH_2CH_2OH$ | H | H | $CH_2$ | C-Cl |
| Cyclopropyl | H | H | H | $CH_2$ | C-$OCH_3$ |
| Cyclopropyl | H | H | H | $CH_2$ | C-$CH_3$ |
| Cyclopropyl | H | H | H | $CH_2$ | N |
| Cyclopropyl | H | $CH_3$ | H | $CH_2$ | C-F |
| Cyclopropyl | H | H | F | $CH_2$ | C-F |
| Cyclopropyl | H | $C_2H_5$ | H | $CH_2$ | C-F |
| Cyclopropyl | H | H | $NH_2$ | $CH_2$ | C-F |
| Cyclopropyl | H | H | H | O | C-H |
| Cyclopropyl | $CH_3$ | H | H | O | C-F |
| $C_2H_5$ | H | H | H | O | C-Cl |
| Cyclopropyl | H | $C_2H_5$ | H | O | C-F |
| Cyclopropyl | H | H | H | O | C-$CH_3$ |
| Cyclopropyl | H | H | H | O | N |
| Cyclopropyl | H | $CH_3$ | H | O | C-F |
| Cyclopropyl | H | H | Cl | O | C-F |
| Cyclopropyl | H | H | $CH_3$ | O | C-F |
| $C_2H_5$ | H | H | $NH_2$ | O | C-F |

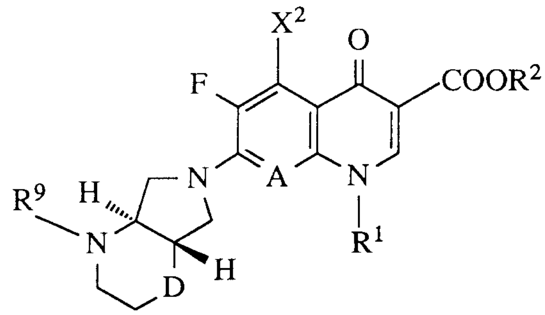

(II)

| $R^1$ | $R^2$ | $R^9$ | $X^2$ | D | A |
|---|---|---|---|---|---|
| Cyclopropyl | H | H | H | $CH_2$ | C-H |
| Cyclopropyl | H | H | H | $CH_2$ | C-F |
| Cyclopropyl | H | H | H | $CH_2$ | C-Cl |
| Cyclopropyl | H | H | H | $CH_2$ | C-$OCH_3$ |
| Cyclopropyl | H | H | H | $CH_2$ | C-$CH_3$ |
| Cyclopropyl | H | H | H | $CH_2$ | N |
| Cyclopropyl | H | H | Br | $CH_2$ | C-F |
| Cyclopropyl | H | H | F | $CH_2$ | C-F |
| Cyclopropyl | H | H | $CH_3$ | $CH_2$ | C-F |
| Cyclopropyl | H | H | $NH_2$ | $CH_2$ | C-F |
| Cyclopropyl | H | H | H | O | C-H |
| Cyclopropyl | H | H | H | O | C-F |
| Cyclopropyl | H | H | H | O | C-Cl |
| Cyclopropyl | H | $CH_3$ | H | O | C-F |
| Cyclopropyl | H | $C_2H_5$ | H | O | C-F |
| Cyclopropyl | H | H | H | O | N |
| Cyclopropyl | H | H | Br | O | C-F |
| Cyclopropyl | H | H | F | O | C-F |
| Cyclopropyl | H | H | $CH_3$ | O | C-F |
| Cyclopropyl | H | H | $NH_2$ | O | C-F |

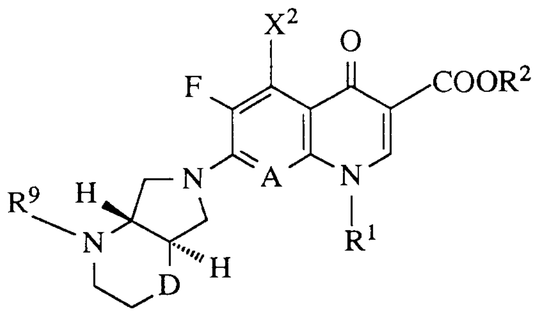

(II)

| $R^1$ | $R^2$ | $R^9$ | $X^2$ | D | A |
|---|---|---|---|---|---|
| Cyclopropyl | H | H | H | $CH_2$ | C-H |
| Cyclopropyl | H | H | H | $CH_2$ | C-F |
| Cyclopropyl | H | H | H | $CH_2$ | C-Cl |
| Cyclopropyl | H | H | H | $CH_2$ | $C-OCH_3$ |
| Cyclopropyl | H | H | H | $CH_2$ | $C-CH_3$ |
| Cyclopropyl | H | H | H | $CH_2$ | N |
| Cyclopropyl | H | H | Br | $CH_2$ | C-F |
| Cyclopropyl | H | H | F | $CH_2$ | C-F |
| Cyclopropyl | H | H | $CH_3$ | $CH_2$ | C-F |
| Cyclopropyl | H | H | $NH_2$ | $CH_2$ | C-F |
| Cyclopropyl | H | H | H | O | C-H |
| Cyclopropyl | H | H | H | O | C-F |
| Cyclopropyl | H | H | H | O | C-Cl |
| Cyclopropyl | H | $CH_3$ | H | O | C-F |
| Cyclopropyl | H | $C_2H_5$ | H | O | C-F |
| Cyclopropyl | H | H | H | O | N |
| Cyclopropyl | H | H | Br | O | C-F |
| Cyclopropyl | H | H | F | O | C-F |
| Cyclopropyl | H | H | $CH_3$ | O | C-F |
| Cyclopropyl | H | H | $NH_2$ | O | C-F |

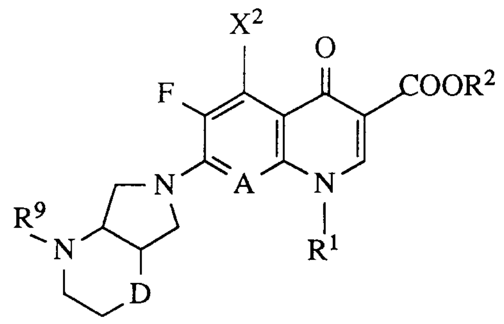

| $R^1$ | $R^2$ | $R^9$ | $X^2$ | D | A |
|---|---|---|---|---|---|
| 2,4-Difluorphenyl | H | H | Cl | $CH_2$ | C-F |
| 2,4-Difluorphenyl | H | H | $CH_3$ | $CH_2$ | C-F |
| 2,4-Difluorphenyl | H | $CH_3$ | H | $CH_2$ | C-F |
| 2,4-Difluorphenyl | H | H | H | O | C-F |
| 2,4-Difluorphenyl | H | H | H | O | C-Cl |
| 4-Fluorphenyl | H | H | H | O | CH |
| 2,4-Difluorphenyl | H | H | H | O | N |
| 2,4-Difluorphenyl | H | H | H | O | C-$OCH_3$ |
| 2,4-Difluorphenyl | H | $C_2H_5$ | H | O | C-F |
| 2,4-Difluorphenyl | H | H | H | $CH_2$ | C-F |
| 2,4-Difluorphenyl | H | H | F | $CH_2$ | C-F |
| 2,4-Difluorphenyl | H | H | H | $CH_2$ | C-Cl |
| 2,4-Difluorphenyl | H | H | H | O | C-Cl |
| 2,4-Difluorphenyl | H | H | H | $CH_2$ | N |
| 2,4-Difluorphenyl | H | H | H | O | N |
| 2,4-Difluorphenyl | H | H | H | O | C-H |
| 2,4-Difluorphenyl | $C_2H_5$ | H | H | O | C-F |

Die Ausgangsverbindungen der Strukturen (III) sind bekannt oder können nach bekannten Methoden hergestellt werden. Als Beispiel sei genannt:
6R,7R-3-Chlormethyl-8-oxo-7-phenylacetylamino-5-thia-1-aza-bicyclo[4.3.0]oct-2-en-2-carbonsäure-benzhydrylester.

Die Umsetzung von (II) mit (III) wird vorzugsweise in einem Verdünnungsmittel wie Dimethylsulfoxid, N,N-Dimethylformamid, N-Methylpyrrolidon, Hexamethylphosphorsäuretrisamid, Sulfolan, Acetonitril, Aceton, Wasser, einem Alkohol wie Methanol, Ethanol, n-Propanol, Isopropanol, Glykolmonomethylether oder Pyridin in Gegenwart eines Säurebinders vorgenommen. Ebenso können Gemische dieser Verdüunungsmittel verwendet werden.

Als Säurebinder können alle üblichen anorganischen und organischen Säurebindungsmittel verwendet werden. Hierzu gehören vorzugsweise die Alkalihydroxide, Alkalicarbonate, organische Amine und Amidine. Als besonders geeignet seien im einzelnen genannt: Triethylamin, 1,4-Diazabicyclo[2.2.2]octan (DABCO), 1,8-Diazabicyclo[5.4.0]undec-7-en (DBU) oder überschüssiges Amin (VI).

Die Umsetzung von (II) mit (III) kann auch nach Silylierung der Verbindung der Formel (II) mit einem Silylierungsmittel wie zum Beispiel Trimethylchlorsilan, Bis-(trimethylsilyl)acetamid, N-(trimethylsilyl)-trifluoracetamid oder Hexamethyldisilazan ohne weiteren Zusatz einer Hilfsbase durchgeführt werden.

Als Schutzgruppen zur Blockierung reaktiver Gruppen, wie zum Beispiel Amino-, Hydroxy-, Thiol- oder Carboxygruppen, werden die in der β-Lactamchemie üblichen Reste verwendet. Beispielhaft seien genannt (Einzelheiten siehe in J.W.F. McOmie, Protective Groups in Organic Chemistry (1973), T.W. Greene, Protective Groups in Organic Synthesis (1981):

a) für den Schutz von Aminogruppen: tert.-Butoxycarbonyl, Allyloxycarbonyl, Isopropenyloxycarbonyl, Trichlorethoxycarbonyl, 4-Nitrobenzyloxycarbonyl, Trichloracetyl, Trifluoracetyl, Trityl, Trimethylsilyl

b) für den Schutz von Hydroxygruppen: Acetyl, Trimethylsilyl, Tetrahydropyranyl

c) für den Schutz von Carboxylgruppen: Estergruppen wie Benzhydryl-, 4-Methoxybenzyl, 4-Nitrobenzyl, Acetoxymethyl, tert.-Butylester.

Die Reaktionstemperaturen können in einem größeren Bereich variiert werden. Im allgemeinen arbeitet man zwischen etwa 0° und 80°C, vorzugsweise zwischen 20° und 50°C.

Die Umsetzung kann bei Normaldruck, aber auch bei erhöhtem Druck durchgeführt werden. Im allgemeinen arbeitet man bei Drucken zwischen etwa 1 und 100 bar, vorzugsweise zwischen 1 und 10 bar.

Bei der Durchführung dieses Verfahrens setzt man auf 1 Mol der Verbindung (II) 1 bis 5 Mol, vorzugsweise 1 bis 2 Mol der Verbindung (III) ein.

Die Herstellung der Säureadditionssalze der erfindungsgemäßen Verbindungen erfolgt in üblicher Weise zum Beispiel durch Lösen des Betains in wäßriger Säure und Ausfällen des Salzes mit einem mit Wasser mischbaren organischen Lösungsmittel wie Methanol, Ethanol, Aceton, Acetonitril. Man kann auch äquivalente Mengen Betain und Säure in Wasser oder einem Alkohol wie Glykolmonomethylether erhitzen und anschließend bis zur Trockne eindampfen oder das ausgefallene Salz absaugen. Als pharmazeutisch verwendbare Salze sind beispielsweise die Salze der Salzsäure, Schwefelsäure, Essigsäure, Glykolsäure, Milchsäure, Bernsteinsäure, Zitronensäure, Weinsäure, Methansulfonsäure, 4-Toluolsulfonsäure, Galacturonsäure, Gluconsäure, Embonsäure, Glutaminsäure oder Asparaginsäure zu verstehen.

Die Alkali- oder Erdalkalisalze der erfindungsgemäßen Carbonsäuren werden beispielsweise durch Lösen des Betains in unterschüssiger Alkali- oder Erdalkalilauge, Filtration von ungelöstem Betain und Eindampfen des Filtrats bis zur Trockne erhalten. Pharmazeutisch geeignet sind Natrium-, Kalium- oder Calciumsalze. Durch Umsetzung eines Alkali- oder Erdalkalisalzes mit einem geeigneten Silbersalz wie Silbernitrat werden die entsprechenden Silbersalze erhalten.

Nach den beschriebenen Verfahren können außer den in den Beispielen genannten Wirkstoffe beispielsweise auch die in folgenden Tabellen aufgeführten Verbindungen hergestellt werden. Die erfindungsgemäßen Verbindungen können sowohl als Racemate als auch als enantiomeren- oder diastereomerenreine Verbindungen vorliegen:

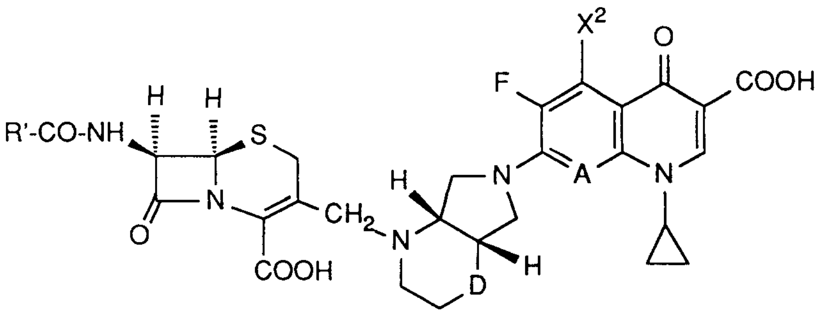

| R' | A | $X^2$ | D |
|---|---|---|---|
| | CF | H | $CH_2$ |
| | CF | F | $CH_2$ |
| 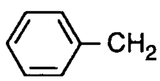 | CF | $NH_2$ | $CH_2$ |
| | C-$OCH_3$ | H | $CH_2$ |
| | N | H | $CH_2$ |
| | CCl | H | O |
| 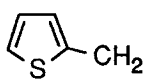 | CCl | H | $CH_2$ |
| | CF | H | $CH_2$ |
| | CH | H | $CH_2$ |
| 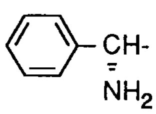 | CCl | H | $CH_2$ |
| | CF | H | $CH_2$ |
| | CH | H | $CH_2$ |
| 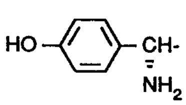 | CCl | H | $CH_2$ |
| | CF | H | $CH_2$ |

| R' | A | $X^2$ | D |
|---|---|---|---|
| | CH | H | $CH_2$ |
| 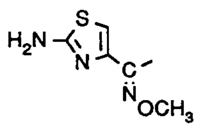 | CF | H | $CH_2$ |
| | CCl | H | $CH_2$ |
| | N | H | $CH_2$ |
| | CF | H | O |
| | CF | H | $CH_2$ |
| 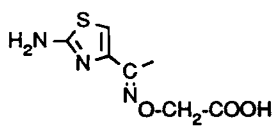 | CF | F | $CH_2$ |
| | CCl | H | $CH_2$ |
| | CF | H | $CH_2$ |
| 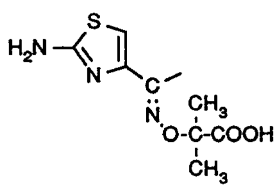 | CF | F | $CH_2$ |
| | CCl | H | $CH_2$ |
| | CH | H | $CH_2$ |
| 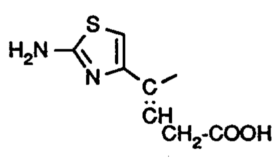 | CF | H | $CH_2$ |
| | CCl | H | $CH_2$ |
| | CH | H | $CH_2$ |
| 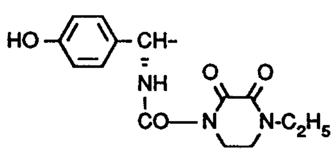 | CF | H | $CH_2$ |
| | CCl | H | $CH_2$ |
| | N | H | $CH_2$ |
| | CH | H | $CH_2$ |
| 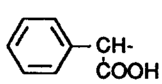 | CF | H | $CH_2$ |
| | CCl | H | $CH_2$ |

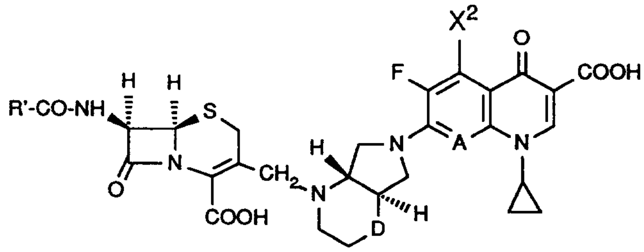

| R' | A | $X^2$ | D |
|---|---|---|---|
| 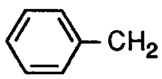 | CCl | H | $CH_2$ |
| | CF | H | $CH_2$ |
| | CF | F | $CH_2$ |
| | CF | $NH_2$ | $CH_2$ |
| | CF | H | O |
| | CH | H | O |
| | CCl | H | O |
| 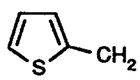 | CCl | H | $CH_2$ |
| | CCl | H | O |
| | CF | H | $CH_2$ |
| | CF | H | O |
| 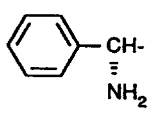 | CCl | H | $CH_2$ |
| | CCl | H | O |
| | CF | H | $CH_2$ |
| | CF | H | O |

| R' | A | $X^2$ | D |
|---|---|---|---|
| 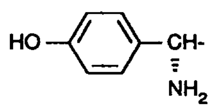 | CH | H | $CH_2$ |
| | CCl | H | $CH_2$ |
| | CF | H | $CH_2$ |
| | CF | H | O |
| 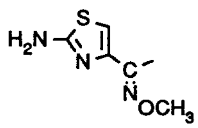 | CH | H | $CH_2$ |
| | CF | H | $CH_2$ |
| | CCl | H | $CH_2$ |
| | N | H | $CH_2$ |
| | CF | H | O |
| 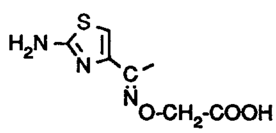 | CF | H | $CH_2$ |
| | CF | H | O |
| | CF | $NH_2$ | $CH_2$ |
| | CCl | H | $CH_2$ |
| 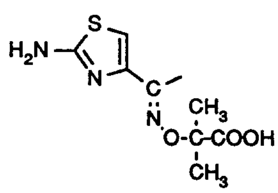 | CF | H | $CH_2$ - |
| | CF | H | O |
| | CF | F | $CH_2$ |
| | CCl | H | $CH_2$ |
| 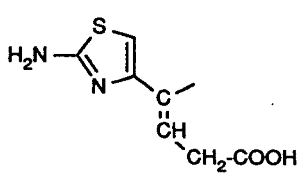 | CH | H | $CH_2$ |
| | CF | H | $CH_2$ |
| | CF | H | O |
| | CCl | H | $CH_2$ |

| R' | A | $X^2$ | D |
|---|---|---|---|
| 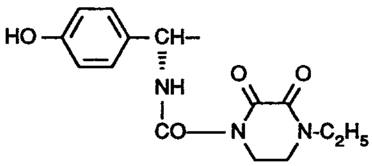 | CH | H | $CH_2$ |
| | CF | H | $CH_2$ |
| | CF | H | O |
| | CCl | H | $CH_2$ |
| | N | H | $CH_2$ |
| 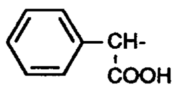 | CH | H | $CH_2$ |
| | CF | H | $CH_2$ |
| | CF | H | O |
| | CCl | H | $CH_2$ |

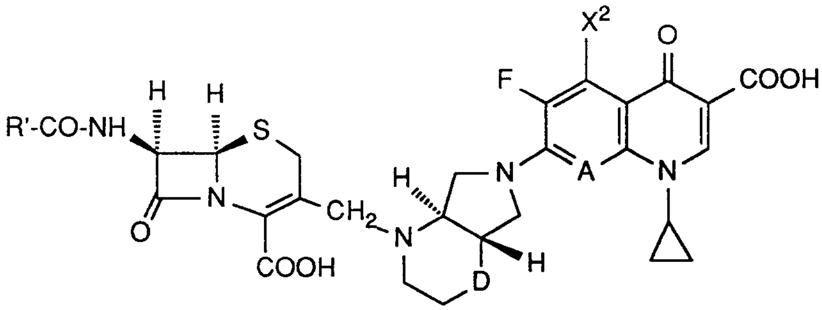

| R' | A | $X^2$ | D |
|---|---|---|---|
| | CCl | H | $CH_2$ |
| | CF | H | $CH_2$ |
| | CF | F | $CH_2$ |
| 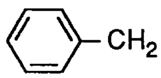 | CF | $NH_2$ | $CH_2$ |
| | CF | H | O |
| | CH | H | O |
| | CCl | H | O |
| | CCl | H | $CH_2$ |
| | CCl | H | O |
| 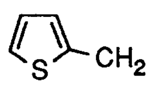 | CF | H | $CH_2$ |
| | CF | H | O |
| | CH | H | $CH_2$ |
| 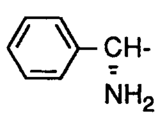 | CH | H | O |
| | CCl | H | $CH_2$ |
| | CCl | H | O |
| | CF | H | $CH_2$ |
| | CF | H | O |
| | CH | H | $CH_2$ |
| 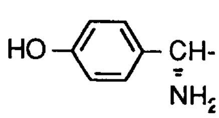 | CCl | H | $CH_2$ |
| | CF | H | $CH_2$ |
| | CF | H | O |

| R' | A | $X^2$ | D |
|---|---|---|---|
| | CH | H | $CH_2$ |
| | CF | H | $CH_2$ |
| 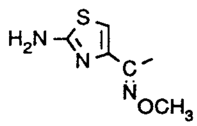 | CCl | H | $CH_2$ |
| | N | H | $CH_2$ |
| | CF | H | O |
| | CF | H | $CH_2$ |
| 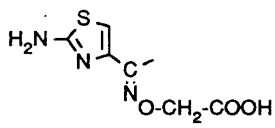 | CF | H | O |
| | CF | $NH_2$ | $CH_2$ |
| | CCl | H | $CH_2$ |
| 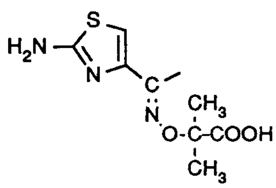 | CF | H | $CH_2$ |
| | CF | H | O |
| | CF | F | $CH_2$ |
| | CCl | H | $CH_2$ |
| 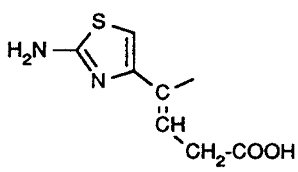 | CH | H | $CH_2$ |
| | CF | H | $CH_2$ |
| | CF | H | O |
| | CCl | H | $CH_2$ |

| R' | A | $X^2$ | D |
|---|---|---|---|
| 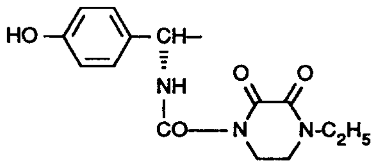 | CH | H | $CH_2$ |
| | CF | H | $CH_2$ |
| | CF | H | O |
| | CCl | H | $CH_2$ |
| | N | H | $CH_2$ |
| 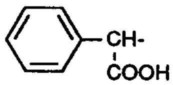 | CH | H | $CH_2$ |
| | CF | H | $CH_2$ |
| | CF | H | O |
| | CCl | H | $CH_2$ |
| | CCl | H | $CH_2$ |

Fortsetzung

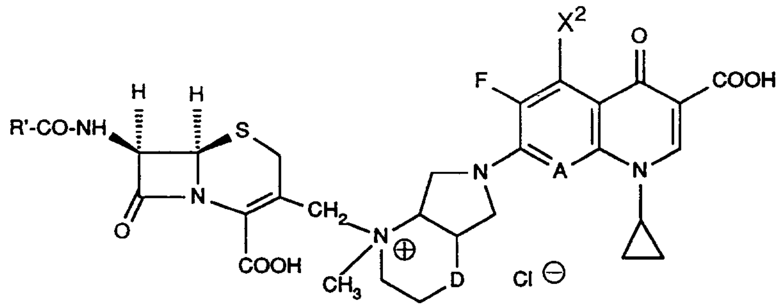

| R' | A | $X^2$ | D |
|---|---|---|---|
| 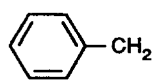 | CCl | H | $CH_2$ |
| | CF | H | $CH_2$ |
| | CF | F | $CH_2$ |
| | CF | H | O |
| | CH | H | $CH_2$ |
| | N | H | $CH_2$ |
| | CCl | H | O |
| 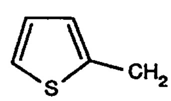 | CCl | H | $CH_2$ |
| | CF | H | $CH_2$ |
| 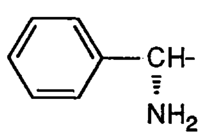 | CH | H | $CH_2$ |
| | CCl | H | $CH_2$ |
| | CF | H | $CH_2$ |
| 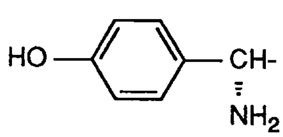 | CH | H | $CH_2$ |
| | CCl | H | $CH_2$ |
| | CF | H | $CH_2$ |
| | CF | H | O |

Fortsetzung

| R' | A | $X^2$ | D |
|---|---|---|---|
| 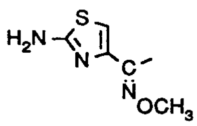 | CH | H | $CH_2$ |
| | CF | H | $CH_2$ |
| | CCl | H | $CH_2$ |
| | N | H | $CH_2$ |
| | CF | H | O |
| 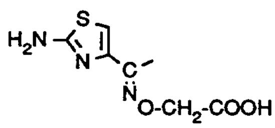 | CF | H | $CH_2$ |
| | CF | $NH_2$ | $CH_2$ |
| | CCl | H | $CH_2$ |
| 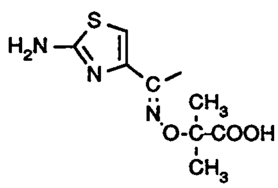 | CF | H | $CH_2$ |
| | CF | F | $CH_2$ |
| | CCl | H | $CH_2$ |
| 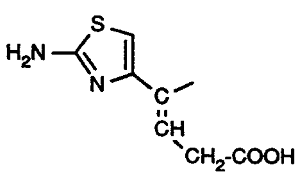 | CH | H | $CH_2$ |
| | CF | H | $CH_2$ |
| | CCl | H | $CH_2$ |
| 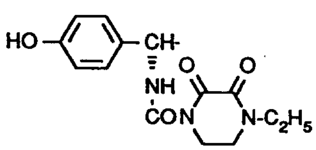 | CH | H | $CH_2$ |
| | CF | H | $CH_2$ |
| | CCl | H | $CH_2$ |
| | N | H | $CH_2$ |

Fortsetzung

| R' | A | $X^2$ | D |
|---|---|---|---|
| | CH | H | $CH_2$ |
| 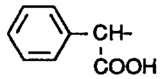 | CF | H | $CH_2$ |
| | CCl | H | $CH_2$ |

Die erfindungsgemäßen Verbindungen wirken stark antibiotisch und zeigen bei geringer Toxizität ein breites antibakterielles Spektrum gegen grampositive und gramnegative Keime, insbesondere gegen Enterobakterien; vor allem auch gegen solche, die resistent sind gegen verschiedene Antibiotika, wie z.B. Penicilline, Cephalosporine, Aminoglykoside, Sulfonamide, Tetracycline und Chinolone.

Diese wertvollen Eigenschaften ermöglichen ihre Verwendung als chemotherapeutische Wirkstoffe in der Medizin sowie als Stoffe zur Konservierung von anorganischen und organischen Materialien, insbesondere von organischen Materialien aller Art, z.B. Polymeren, Schmiermitteln, Farben, Fasern, Leder, Papier und Holz, von Lebensmitteln und von Wasser.

Die erfindungsgemäßen Verbindungen sind gegen ein sehr breites Spektrum von Mikroorganismen wirksam. Mit ihrer Hilfe können gramnegative und grampositive Bakterien und bakterienähnliche Mikroorganismen bekämpft sowie die durch diese Erreger hervorgerufenen Erkrankungen verhindert, gebessert und/oder geheilt werden.

Die erfindungsgemäßen Verbindungen zeichnen sich durch verstärkte Wirkung auf ruhende und resistente Keime aus. Bei ruhenden Bakterien, also Bakterien, die kein nachweisbares Wachstum zeigen, wirken die Verbindungen weit unterhalb von Konzentrationen bisher bekannter Substanzen. Dies bezieht sich nicht nur auf die einzusetzende Menge, sondern auch auf die Geschwindigkeit der Abtötung. Solche Ergebnisse konnten bei grampositiven und -negativen Bakterien, insbesondere bei Staphylococcus aureus, Pseudomonas aeruginosa, Enterococcus faecalis und Escherichia coli beobachtet werden.

Auch gegenüber Bakterien, die gegenüber vergleichbaren Substanzen als weniger empfindlich eingestuft werden, insbesondere resistenten Staphylococcus aureus, Escherichia coli, Pseudomonas aeruginosa und Enterococcus faecalis zeigen die erfindungsgemäßen Verbindungen überraschende Wirkungssteigerungen.

Besonders wirksam sind die erfindungsgemäßen Verbindungen gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Die erfindungsgemäßen Verbindungen können in verschiedenen pharmazeutischen Zubereitungen angewendet werden. Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Die Bestimmung der "Minimalen Hemmkonzentrationen" (MHK) erfolgte in Mikrotiterplatten unter Verwendung von Brain-Heart-Infusion-Bouillon, wobei ca. $10^5$ Keime/ml als Inokulum eingesetzt werden.

Die nachstehende Tabelle belegt die überraschenden Vorteile der -erfindungsgemäßen Verbindung A (Verbindung aus Beispiel 2) im Vergleich zu der Referenzverbindung B (Verbindung aus Beispiel 15 der EP 492 277):

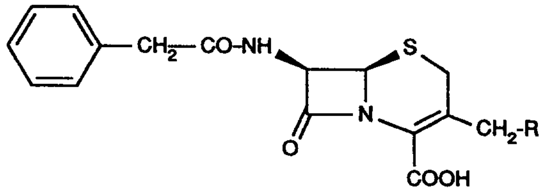

A : R = 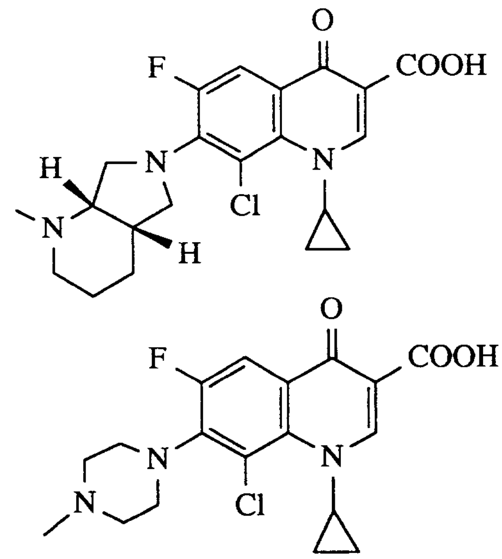

B : R =

Tabelle = MHK-Werte (µg/ml)

| Teststamm | Verbindung A | Referenzverbindung B |
| --- | --- | --- |
| Escherichia coli Neumann | 0,03 | 0,25 |
| Klebsiella sp. 63 | 0,125 | 1 |
| Serratia marcescens 16040 | 4 | 16 |
| Staphylococcus aureus ICB 25701 | 0,25 | 32 |
| ICB 25768 | 2 | 128 |
| 1756 | 0,004 | 0,03 |
| 133 | 0,03 | 0,125 |
| Enterococcus faecalis 27101 | 0,25 | 8 |
| 9790 | 0,25 | 8 |

Herstellung der Vorprodukte

Beispiel A

[S,S]-2,8-Diazabicyclo[4.3.0]nonan

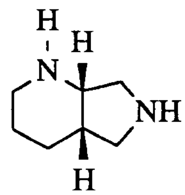

1) [S,S]-8-Benzyl-2,8-diazabiyclo[4.3.0]nonan

Methode I:

a) Trennung der diastereomeren Salze:
3,0 g (20 mmol) D(-)-Weinsäure werden in 10 ml Dimethylformamid durch Erwärmen auf 80 °C gelöst und mit einer Lösung von 2,16 g (10 mmol) cis-8-Benzyl-2,8-diazabicyclo[4.3.0]nonan in 3 ml Dimethylformamid versetzt. Es wird 1 Stunde bei 0 °C nachgerührt, dann wird abgesaugt und mit Dimethylformamid und Methoxyethanol gewaschen.
Ausbeute: 1,93 g,
Schmelzpunkt: 146-151 °C,
$[\alpha]_D^{23}$ = -19,3 ° (c = 1, $H_2O$).

Durch einmaliges Umkristallisieren aus Methoxyethanol wird diastereomerenreines [S,S]-8-Benzyl-2,8-diazabicyclo[4.3.0]nonan-D-tartrat erhalten.
$[\alpha]_D^{23}$ = -22,7 ° (c = 1, $H_2O$),
Schmelzpunkt: 148-154 °C.

b) Freisetzung der Base:
40 g [S,S]-8-Benzyl-2,8-diazabicyclo[4.3.0]nonan-D-tartrat werden in 250 ml Wasser gelöst und mit 32 g 45 %iger Natronlauge versetzt. Das ausfallende Öl wird in 150 ml tert.-Butyl-methylether aufgenommen, die wäßrige Phase nochmals mit 150 ml tert.-Butyl-methylether extrahiert und die vereinigten organischen Phasen nach dem Trocknen über Natriumsulfat eingeengt. Dann wird im Vakuum destilliert.
Ausbeute: 18,5 g [S,S]-8-Benzyl-2,8-diazabicyclo[4.3.0]nonan,
Siedepunkt: 107-109 °C/0,1 mbar,
$[\alpha]_D^{24}$ = 17,3 ° (unverdünnt).

Methode II:

75,0 g (0,5 mol) L(+)-Weinsäure werden bei 80 °C in 250 ml Dimethylformamid gelöst und 54,1 g (0,25 mol) cis-8-Benzyl-2,8-diazabicyclo[4.3.0]nonan als Lösung in 75 ml Dimethylformamid zugetropft. Es wird langsam auf 20 °C abgekühlt und die Kristallsuspension 1 Stunde nachgerührt. Die Kristalle ([R,R]-8-Benzyl-2,8-diazabicyclo[4.3.0]nonan-L-tartrat) werden abgesaugt und das Filtrat am Rotationsverdampfer eingeengt. Der Rückstand wird in 500 ml Wasser gelöst und mit 63 g 45 %iger Natronlauge wie unter Methode I beschrieben aufgearbeitet.

Ausbeute: 25,2g [S,S]-8-Benzyl-2,8-diazabicyclo[4.3.0]nonan;
das Produkt enthält 3,6 % des R,R-Enantiomeren (nach Derivatisierung mit Chlorameisensäure-menthylester gaschromatographisch bestimmt).

Die Verbindung kann nach Methode I mit D(-)-Weinsäure zu diastereomerenreinem [S,S]-8-Benzyl-2,8-diazabicyclo[4.3.0]nonan-D-tartrat umgesetzt werden. Ein Umkristallisieren ist dabei nicht erforderlich.

Methode III:

Zu einer Lösung von 102,9 g (0,685 mol) L(+)-Weinsäure in 343 ml Dimethylformamid werden bei 80 bis 90 °C 73,6 g (0,34 mol) cis-8-Benzyl-2,8-diazabicyclo[4.3.0]nonan als Lösung in 111 ml Dimethylformamid getropft. Man impft mit [R,R]-8-Benzyl-2,8-diazabicyclo[4.3.0]nonan-L-tartrat an und kühlt langsam bis auf 18°C Innentemperatur ab. Die Kristalle werden abgesaugt, das Filtrat mit [S,S]-8-Benzyl-2,8-diazabicyclo[4.3.0]nonan-L-tartrat angeimpft und bis zur vollständigen Kristallisation gerührt. (Aus der Mutterlauge kann nach Einengen und Freisetzung der Base wie unter Methode I beschrieben durch Aufreinigung mit D(-)-Weinsäure [S,S]-8-Benzyl-2,8-diazabicyclo[4.3.0]nonan-D-tartrat erhalten werden.) Dann wird abgesaugt, mit Dimethylformamid und Isopropanol gewaschen und an der Luft getrocknet. Die Kristalle werden aus 88 %igem Ethanol umkristallisiert Es werden 52 g [S,S]-8-Benzyl-2,8-diazabicyclo-[4.3.0]nonan-L-tartrat-trihydrat erhalten.
Schmelzpunkt: 201-204°C,
$[\alpha]_D^{23}$ = +5,2° (c = 1, $H_2O$).

Das Salz kann wie unter Methode I (Freisetzung der Base) beschrieben zu enantiomerenreinem [S,S]-8-Benzyl-2,8-diazabicyclo[4.3.0]nonan verarbeitet werden.

Methode IV:

a) Enantiomerentrennung von cis-8-Benzyl-7,9-dioxo-2,8-diazabicyclo[4.3.0]nonan zu [1S,6R]-8-Benzyl-7,9-dioxo-2,8-diazabicyclo[4.3.0]nonan
Man verfährt analog Beispiel B (Methode II/a), wobei als chirales Hilfsreagens D(-)-Weinsäure verwendet wird, oder man verfährt wie folgt:
Mutterlauge und Waschlauge von [1R,6S]-8-Benzyl-7,9-dioxo-2,8-diazabicyclo[4.3.0]nonan-L-tartrat (aus Beispiel B, Methode II/a) werden zusammen eingeengt, in Wasser aufgenommen und dreimal mit Toluol extrahiert. Die Toluolphasen werden verworfen. Die wäßrige Phase wird mit gesättigter Natriumhydrogencarbonatlösung versetzt, bis ein pH-Wert von 7 bis 8 erreicht ist, anschließend viermal mit Methylenchlorid extrahiert. Die vereinigten Methylenchloridphasen werden über Magnesiumsulfat getrocknet und eingeengt.
Ausbeute: 14,4 g (60 % der Theorie des ursprünglich eingesetzten racemischen cis-8-Benzyl-7,9-dioxo-2,8-diazabicyclo[4.3.0]nonans).
$[\alpha]_D^{23}$: -4,5° (c = 5, Ethanol).

Diese 14,4 g (59 mmol) werden mit 8,6 g (57 mmol) D(-)-Weinsäure aus 120 ml Ethanol analog Beispiel B (Methode II/a) kristallisiert.
Ausbeute: 8,9 g (77 % der Theorie) [1S,6R]-8-Benzyl-7,9-dioxo-2,8-diazabicyclo[4.3.0]nonan-D-tartrat.
$[\alpha]_D^{23}$: -46,2° (c = 0,5, 1n HCl);
nach Umkristallisation aus einer Mischung Ethanol/Glykolmonomethylether erfolgt eine weitere Reinigung:
$[\alpha]_D^{23}$: -59,3° (c = 0,5, 1n HCl).

5,0 g (12,7 mmol) des auf diese Weise erhaltenen diastereomerenreinen Tartrats wurden, wie unter Beispiel B, Methode II/a beschrieben, in das freie Amin überführt:
Ausbeute: 3,0 g (96 % der Theorie) [1S,6R]-8-Benzyl-7,9-dioxo-2,8-diazabicyclo[4.3.0]nonan, Schmelzpunkt: 60-61°C,
$[\alpha]_D^{23}$: -22,2° (c = 5, Ethanol).

Gaschromatographisch wurde nach Derivatisierung mit Chlorameisensäurementhylester ein Enantiomerenüberschuß von 96,6 % ee ermittelt.

b) Reduktion von [1S,6R]-8-Benzyl-7,9-dioxo-2,8-diazabicyclo[4.3.0]nonan zu [S,S]-8-Benzyl-2,8-diazabicyclo[4.3.0]nonan
Man verfährt analog Beispiel B (Methode II, b), wobei als Edukt jedoch [1S,6R]-8-Benzyl-7,9-dioxo-2,8-diazabicyclo[4.3.0]nonan eingesetzt wird.

Das nach der Aufarbeitung erhaltene Rohprodukt erwies sich bei der Derivatisierung mit Chlorameisensäurementhylester als [S,S]-8-Benzyl-2,8-diazabicyclo[4.3.0]nonan. Eine Racemisierung wurde bei der Reduktion nicht beobachtet.

2) [S,S]-2,8-Diazabicyclo[4.3.0]nonan
28,4 g (0,131 mol) [S,S]-8-Benzyl-2,8-diazabicyclo[4.3.0]nonan werden in 190 ml Methanol über 5,8 g Palladium auf Aktivkohle (5 %) bei 90°C und 90 bar innerhalb von 5 Stunden hydriert. Dann wird der Katalysator abgesaugt, mit Methanol gewaschen und das Filtrat am Rotationsverdampfer eingeengt. Der Rückstand wird ohne zu fraktionieren destilliert.
Ausbeute: 15,0g (90,5 % der Theorie) [S,S]-2,8-Diazabicyclo[4.3.0]nonan,
Siedepunkt: 44-59°C/0,18 mbar,
$[\alpha]_D^{22}$ = -2,29° (unverdünnt),
ee >99 % (gaschromatographisch nach Derivatisierung mit Mosher's Reagenz bestimmt).

Methode V:

3,75 g (25 mmol) L(+)-Weinsäure werden in 50 ml Dimethylformamid bei 80°C gelöst vorgelegt und 10,82 g (50 mmol) cis-8-Benzyl-2,8-diazabicyclo[4.3.0]nonan als Lösung in 15 ml Dimethylformamid zugetropft. Man impft mit [R,R]-8-Benzyl-2,8-diazabicyclo[4.3.0]-nonan-L-tartrat an und rührt eine Stunde bei circa 72°C, um die Kristallkeim-Bildung zu vervollständigen. Dann wird langsam auf 15°C abgekühlt, abgesaugt und zweimal mit jeweils 13 ml Dimethylformamid gewaschen. Die vereinigten Filtrate werden auf 80°C erhitzt und mit weiteren 3,75 g (25 mmol) L(+)-Weinsäure versetzt. Es wird noch bis auf 119°C erhitzt, bis eine klare Lösung entstanden ist, und wieder langsam auf Raumtemperatur unter Animpfen mit [S,S]-8-Benzyl-2,8-diazabicyclo[4.3.0]nonan-L-tartrat abgekühlt. Die Kristalle werden abgesaugt, nacheinander mit Dimethylformamid, 2-Methoxy-ethanol und Ethanol gewaschen und an der Luft getrocknet.
Ausbeute: 9,59 g
Schmelzpunkt: 188 bis 192°C.
Die Kristalle werden aus 95 ml 80 %igem Ethanol umkristallisiert. Es werden 8,00 g [S,S]-8-Benzyl-2,8-diazabicyclo[4.3.0]nonan-L-tartrat-trihydrat (76 % der Theorie) erhalten, das bei 112 bis 118°C unter Aufschäumen schmilzt, dann wieder erstarrt und bei 199 bis 201°C erneut schmilzt.
$[\alpha]_D^{23}$ = 4,5° (c = 1, Wasser)
ee: 98,0 % (gaschromatographisch nach Derivatisierung mit Chlorameisensäure-menthylester bestimmt).

Beispiel B

[R,R]-2,8-Diazabicyclo[4.3.0]nonan

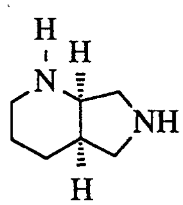

1) [R,R]-8-Benzyl-2,8-diazabicyclo[4.3.0]nonan

Methode I:

Die nach Beispiel A, Methode II erhaltenen Kristalle von [R,R]-8-Benzyl-2,8-diazabicyclo[4.3.0]nonan werden mit Dimethylformamid und Methoxyethanol gewaschen (49,2 g) und aus 300 ml Methoxyethanol umkristallisiert. Es werden 45,6 g enantiomerenreines [R,R]-8-Benzyl-2,8-diazabicyclo[4.3.0]nonan-L-tartrat erhalten (Enantiomerenreinheit gaschromatographisch nach Derivatisierung mit Chlorameisensäure-menthylester bestimmt).
Schmelzpunkt: 121-124°C,
$[\alpha]_D^{23}$ = +22,3° (c = 1, $H_2O$).

Das Salz (44,5 g) wird wie in Beispiel A, Methode Ib beschrieben, in die freie Base überführt. Es werden 20,2 g [R,R]-8-Benzyl-2,8-diazabicyclo[4.3.0]nonan erhalten.
Siedepuukt: 107-111°C/0,04 mbar,
$[\alpha]_D^{24}$ = -17,5° (unverdünnt).

Methode II

a) Enantiomerentrennung von cis-8-Benzyl-7,9-dioxo-2,8-diazabicyclo[4.3.0]nonan zu [1R,6S]-8-Benzyl-7,9-dioxo-2,8-diazabicyclo[4.3.0]nonan
24,1 g (98,8 mmol) cis-8-Benzyl-7,9-dioxo-2,8-diazabicyclo[4.3.0]nonan werden in einer Mischung aus 410 ml Ethanol und 25 ml Acetonitril in einem Dreihalskolben unter Rühren zum Rückfluß erhitzt Anschließend gibt man 14,8 g (98,8 mmol) L(+)-Weinsäure auf einmal zu. Nachdem sich die gesamte Menge Weinsäure vollständig gelöst hat, wird zunächst die Heizung abgestellt, der Kolben aber im Ölbad gelassen. Wenn das System sich soweit abgekühlt hat, daß die Lösung nicht mehr siedet, wird der Rührer abgestellt. Bei einer Temperatur von 50°C erfolgt Kristallisation auf Zugabe von Impfkristallen. Nach Stehen über Nacht und Abkühlung auf Raumtemperatur werden die ausgefallenen Kristalle abgesaugt und mit wenig Ethanol/Petrolether (1:1) gewaschen und 2 Stunden bei 80 °C getrocknet.
Ausbeute: 9,8 g (50 % der Theorie) [1R,6S]-8-Benzyl-7,9-dioxo-2,8-diazabicyclo[4.3.0]nonan-L-tartrat,
$[\alpha]_D^{23}$ = +47,7 ° (c = 0,5, 1n HCl).

Durch zweimaliges Umkristallisieren aus einer Mischung von Ethanol und Glykolmonomethylether läßt sich die Verbindung noch weiter reinigen:
$[\alpha]_D^{23}$ = +58,6 ° (c = 0,5, 1n HCl).
$^1$H-NMR (DMSO): 7,22-7,35(2m, 2H, Aryl-H); 4,55 (s, 2H, Benzyl-$CH_2$); 4,28 (s, 2H, Weinsäure-CH); 3,91 (d, 1H, 1-CH); 2,97 (dd, 1H, 6-CH); 2,53-2,66 (m, 2H, 3-$CH_2$); 1,78 und 1,68 (2m, 2H, 5-$CH_2$); 1,42 und 1,28 ppm (2m, 2H, 4-$CH_2$).

| $C_{18}H_{22}N_2O_8$ (394) | | | | |
|---|---|---|---|---|
| Berechnet: | C 54,4 | H 5,6 | N 7,1 | O 32,5 |
| Gefunden: | C 54,7 | H 5,8 | N 7,1 | O 32,4 |

Die Bestimmung der Absolutkonfiguration erfolgte über eine Röntgenstrukturanalyse:

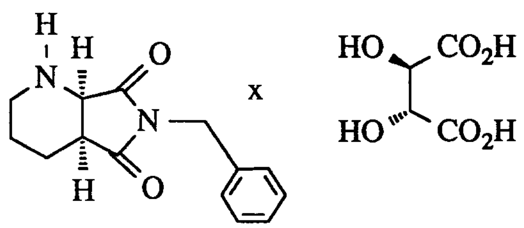

3,6 g (9,1 mmol) des auf diese Weise erhaltenen diastereomerenreinen Tartrats werden zur Freisetzung der Base in Wasser gelöst und mit gesättigter Natriumhydrogencarbonatlösung versetzt, bis ein pH-Wert von 7 bis 8 erreicht ist. Die wäßrige Lösung wird viermal mit je 20 ml Methylenchlorid extrahiert Die vereinigten Methylenchloridphasen werden über Magnesiumsulfat getrocknet und eingeengt.
Ausbeute: 2,2 g (99 % der Theorie) [1R,6S]-8-Benzyl-7,9-dioxo-2,8-diazabicyclo[4.3.0]nonan,
Schmelzpunkt: 60-61 °C,
$[\alpha]_D^{23}$: + 21,8 ° (c = 5, Ethanol).

Gaschromatographisch wurde nach Derivatisierung mit Chlorameisensäurementhylester ein Enantiomerenüberschuß von 93,8 % ee ermittelt.

b) Reduktion von [1R,6S]-8-Benzyl-7,9-dioxo-2,8-diazabicyclo[4.3.0]nonan zu [R,R]-8-Benzyl-2,8-diazabicyclo[4.3.0]nonan

In einem ausgeheizten Kolben werden unter $N_2$ 0,34 g (9 mmol) Lithiumaluminiumhydrid in 18 ml wasserfreiem Tetrahydrofuran vorgelegt und 0,73 g (3 mmol) [1R,6S]-8-Benzy-7,9-dioxo-2,8-diazabicyclo[4.3.0]nonan als Lösung in 3 ml wasserfreiem Tetrahydrofuran zugetropft Dann wird 16 Stunden unter Rückflußkühlung gekocht Die Aufarbeitung erfolgt durch Zutropfen von 0,34 ml Wasser in 10 ml Tetrahydrofuran, 0,34 ml 10 %ige Natronlauge sowie 1,02 ml Wasser. Der Niederschlag wird abgesaugt, mit Tetrahydrofuran gewaschen und das Filtrat eingeengt. Es bleiben 0,7 g rohes [R,R]-8-Benzyl-2,8-diazabicyclo[4.3.0]nonan zurück (GC-Gehalt: 99 %).

Bei der gaschromatographischen Bestimmung der Enantiomerenreinheit mit Chlorameisensäurementhylester konnte keine Racemisierung festgestellt werden.

2) [R,R]-2,8-Diazabicyclo[4.3.0]nonan

Nach der Vorschrift von Beispiel A, 2 werden 19,4 g (0,09 mol) [R,R]-8-Benzyl-2,8-diazabicyclo[4.3.0]-nonan hydriert
Ausbeute: 9,61 g (85 %) [R,R]-2,8-Diazabicyclo[4.3.0]nonan,
Siedepunkt: 45-58 °C/0,08 mbar,
$[\alpha]_D^{23}$ = +2,30 ° (unverdünnt).

Beispiel C

[S,S]-2-Methyl-2,8-diazabicyclo[4.3.0]nonan

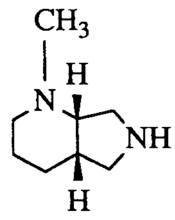

1) [S,S]-8-Benzyl-2-methyl-2,8-diazabicyclo[4.3.0]nonan
43,2 g (0,2 mmol) [S,S]-8-Benzyl-2,8-diazabicyclo[4.3.0]nonan werden mit 20 ml 37 % Formaldehyd-Lösung, 40 ml Wasser und 24 g Essigsäure versetzt und 10 Stunden bei 20 °C und 20 bar über 2 g Palladium auf Aktivkohle (5 %) hydriert Dann wird abgesaugt, das Filtrat mit Kaliumcarbonat alkalisch gestellt und das Produkt mit tert.-Butylmethylether extrahiert. Nach dem Trocknen über Natriumsulfat wird eingeengt und der Rückstand im Vakuum destilliert.
Ausbeute: 14,8 g,
Siedepunkt: 114-124 °C/0,14 mbar.

2) [S,S]-2-Methyl-2,8-diazabicyclo[4.3.0]nonan
12,9 g (56 mmol) [S,S]-8-Benzyl-2-methy-2,8-diazabicyclo[4.3.0]nonan werden in 90 ml Methanol bei 90 °C und 90 bar über 1,1 g Palladium auf Aktivkohle (5 %) hydriert Dann wird filtriert, das Filtrat am Rotationsverdampfer eingeengt und der Rückstand im Vakuum destilliert.
Ausbeute: 5,5 g enantiomerenreines [S,S]-2-Methyl-2,8-diazabicyclo[4.3.0]nonan (Nachweis durch Derivatisierung mit Mosher's Reagenz),
Siedepunkt: 78-81 °C/14 mbar.

Beispiel D

cis-2-Oxa-5,8-diazabicyclo[4.3.0]nonan

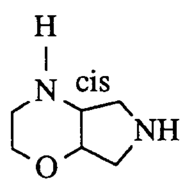

1) trans-1-Benzoyl-3-brom-4-(2-hydroxyethoxy)-pyrrolidin
Man löst 95 g (0,55 mol) 1-Benzoyl-3-pyrrolin in 380 g Ethylenglykol und setzt bei Raumtemperatur 101 g (0,57 mol) N-Bromsuccinimid in 5 g-Portionen innerhalb von 2 Stunden hinzu. Man rührt anschließend über Nacht bei Raumtemperatur, gießt auf Wasser, extrahiert mit Methylenchlorid, trocknet über Magnesiumsulfat und engt die Lösung ein. Der Rückstand (188 g) wurde mit Essigsäureethylester an Kieselgel chromatographiert.
Ausbeute: 136,5 g (78 % der Theorie),
Gehalt nach GC: 99%.
2) trans-1-Benzoyl-3-brom-4-(2-tosyloxyethoxy)-pyrrolidin
Man löst 92 g (0,239 mol) trans-1-Benzoyl-3-brom-4-(2-hydroxyethoxy)-pyrrolidin, 32 g (0,316 mol) Triethylamin und 1 g 4-Dimethylaminopyridin in 750 ml Toluol und tropft 60 g (0,31 mol) Tosylchlorid in 450 ml Toluol hinzu. Man rührt zwei Tage bei Raumtemperatur, setzt Wasser zu, trennt die wäßrige Phase ab und extrahiert sie mit Toluol. Die Toluollösungen werden mit 10 %iger Salzsäure gewaschen,

über Magnesiumsulfat getrocknet, eingeengt, in Essigsäureethylester gelöst und über Kieselgel filtriert Das Filtrat wird eingeengt.

Ausbeute: 125 g (91 % der Theorie).

Das Dünnsctichtchromatogramm zeigt eine einheitliche Verbindung.

3) cis-8-Benzoyl-5-benzyl-2-oxa-5,8-diazabicyclo[4.3.0]nonan

Man erhitzt 124 g (0,265 mol) trans-1-Benzoyl-3-brom-4-(2-tosyloxyethoxy)-pyrrolidin mit 86 g (0,8 mol) Benzylamin in 1,5 l Xylol über Nacht unter Rückfluß. Man saugt die Salze des Benzylamins ab und engt das Filtrat ein.

Rohausbeute: 91,2g.

4) cis-5-Benzyl-2-oxa-5,8-diazabicyclo[4.3.0]nonan

Man erhitzt 91 g (0,265 mol) cis-8-Benzoyl-5-benzyl-2-oxa-5,8-diazabicyclo[4.3.0]nonan mit 200 ml konzentrierter Salzsäure und 140 ml Wasser über Nacht unter Rückfluß. Nach dem Abkühlen saugt man die Benzoesäure ab, engt auf das halbe Volumen ein, stellt die Lösung mit Kaliumcarbonat alkalisch, extrahiert mit Chloroform, trocknet über Kaliumcarbonat, engt ein und destilliert.

Ausbeute: 30,7 g (48,8 % der Theorie),

Siedepunkt: 134-142 °C/0,6 mbar,

Gehalt nach GC: 92%.

5) cis-2-Oxa-5,8-diazabicyclo[4.3.0]nonan-dihydrochlorid

Man hydriert 26 g (0,11 mol, 92 %ig) cis-5-Benzyl-2-oxa-5,8-diazabicyclo[4.3.0]nonan in 180 ml Ethanol und 19 ml konzentrierter Salzsäure an 3 g Palladium/Aktivkohle (10 % Pd) bei 100 °C und 100 bar $H_2$. Man saugt den Katalysator ab, engt das Filtrat ein und trocknet die ausgeschiedenen Kristalle im Exsikkator über Phosphorpentoxid.

Ausbeute: 17,1 g (77 % der Theorie),

Schmelzpunkt: 244-250 °C.

Beispiel E

Enantiomerentrennung von cis-5-Benzyl-2-oxa-5,8-diazabicyclo[4.3.0]nonan

150,1 g (1 mol) D(-)-Weinsäure werden bei 60 bis 65 °C in 700 ml Methanol vorgelegt und 218,3 g (1 mol) cis-5-Benzyl-2-oxa-5,8-diazabicyclo[4.3.0]nonan als Lösung in 300 ml Methanol zugetropft. Dann läßt man langsam auf etwa 49 °C erkalten bis die Lösung trübe wird, impft mit in einem Vorversuch erhaltenen Kristallen von 1R,6S-5-Benzyl-2-oxa-5,8-diazabicyclo[4.3.0]nonan-D-tartrat an, rührt 30 Minuten bei dieser Temperatur zur Kristallkeim-Bildung nach und kühlt dann langsam bis auf 0 bis 3 °C ab. Nach dem Absaugen wird mit einer auf 0 °C gekühlten Mischung aus 200 ml Ethanol und 100 ml Methanol und dann 3 mal mit jeweils 300 ml Ethanol gewaschen und anschließend das Produkt an der Luft getrocknet.

Ausbeute: 160,3 g 1R,6S-5-Benzyl-2-oxa-5,8-diazabicyclo[4.3.0]nonan-tartrat (87 % der Theorie)

Schmelzpunkt: 174,5 bis 176,5 °C

ee > 97 % (nach Derivatisierung mit 1-Phenyl-ethylisocyanat und HPLC-Auswertung)

$[\alpha]_D^{23}$ = +24,0 ° (c = 1, Methanol)

156,9 g des 1. Kristallisats werden aus 1 500 ml Methanol umkristallisiert.

Ausbeute: 140,0g (89 % wiedergewonnen)

Schmelzpunkt: 176 bis 177 °C

$[\alpha]_D^{23}$ = +25,2 ° (c = 1, Methanol)

Die methanolische Mutterlauge der 1. Kristallisation wird am Rotationsverdampfer eingeengt. Der sirupöse Rückstand (236 g) wird in 500 ml Wasser gelöst, mit 250 ml 6 n Natronlauge auf pH 12 bis 13 gestellt, 3 mal mit je 350 ml Toluol extrahiert, der Extrakt über Natriumcarbonat getrocknet und im Vakuum eingeengt. Der Rückstand, 113,1 g eines braunen Öls, das nach gaschromatographischer Untersuchung 97 % cis-5-Benzyl-2-oxa-5,8-diazabicyclo[4.3.0]nonan enthält, wird ohne Aufreinigung zur Herstellung des 1S,6R-Enantiomeren eingesetzt.

113,1 g (0,518 mol) rohes angereichertes 1S,6R-5-Benzyl-2-oxa-5,8-diazabicyclo[4.3.0]nonan werden in 155 ml Methanol gelöst und zu einer siedenden Lösung von 77,8 g (0,518 mol) L(+)-Weinsäure in 363 ml Methanol getropft. Schon während des Zutropfens bildet sich allmählich ein Kristallbrei. Es wird 1 Stunde bei 60 °C nachgerührt und dann langsam innerhalb von 2 Stunden auf 0 °C abgekühlt. Die Kristalle werden abgesaugt und mit einer auf 0 °C gekühlten 2:1-Mischung aus Ethanol und Methanol und anschließend 3 mal mit Ethanol gewaschen. Dann wird an der Luft getrocknet.

Ausbeute: 145,5 g 1S,6R-5-Benzyl-2-oxa-5,8-diazabicyclo[4.3.0]nonan-L-tartrat (79 % der Theorie)

Schmelzpunkt: 174,5 bis 176,5 °C

ee > 97 % (nach Derivatisierung mit 1-Phenyl-ethylisocyanat und HPLC-Auswertung)
$[\alpha]_D^{23}$ = -24,0° (c = 1,Methanol)
Freisetzung der enantiomerenreinen Basen:
144 g (0,39 mol) 1S,6R-5-Benzyl-2-oxa-5,8-diazabicyclo[4.3.0]nonan-tartrat werden in 250 ml Wasser gelöst und 175 ml (1,05 mol) 6 n Natronlauge zugesetzt. Das abgeschiedene Öl wird in 500 ml Toluol aufgenommen, die organische Phase abgetrennt und die wäßrige noch 3 mal mit jeweils 250 ml Toluol extrahiert. Die vereinigten organischen Phasen werden über Natriumcarbonat getrocknet, filtriert und am Rotationsverdampfer eingeengt. Der Rückstand wird über eine 20 cm Vigreuxkolonne im Hochvakuum destilliert
Ausbeute: 81,6 g (96 % der Theorie) 1S,6R-5-Benzyl-2-oxa-5,8-diazabicyclo[4.3.0]nonan
Siedepunkt: 120 bis 139° C/0,04 bis 0,07 mbar
Gehalt: 100% gaschromatographisch bestimmt
Dichte: $\delta$ = 1,113 g/ml
$[\alpha]_D^{23}$ = -60,9° (unverdünnt)
Destillationsrückstand: 0,12 g
In gleicher Weise werden aus 139,2 g (0,376 mol) 1R,6S-5-Benzyl-2-oxa-5,8-diazabicyclo[4.3.0]nonan-tartrat 76,0 g (93 % der Theorie) 1R,6S-5-Benzyl-2-oxa-5,8-diazabicyclo[4.3.0]nonan erhalten.
$[\alpha]_D^{23}$ = +61,2° (unverdünnt).

Die für das cis-5-Benzyl-2-oxa-5,8-diazabicyclo[4.3.0]nonan beschriebene Enantiomerentrennung kann analog auch mit trans-5-Benzyl-2-oxa-5,8-diazabicyclo[4.3.0]nonan zu R,R- und S,S-5-Benzyl-2-oxa-5,8-diazabicyclo[4.3.0]nonan durchgeführt werden.

Beispiel F

1) 3S,4S-4-Allyloxy-3-hydroxypyrrolidin-1-carbonsäure-tert.-butylester
Man legt 16,5 g (0,55 mol) 80 %iges NaH in 500 ml absolutem Dioxan vor und tropft bei 60° C eine Lösung von 107,5 g (0,53 mol) S,S-3,4-Dihydroxypyrrolidin-1-carbonsäure-tert.-butylester (DE-A-3 403 194) in absolutem Dioxan heiß gelöst hinzu. Man rührt eine Stunde bei 60° C und tropft dann 64 g (0,53 mol) Allylbromid hinzu. Anschließend rührt man drei Stunden bei 60° C. Man engt ein und löst den Rückstand in 200 ml Wasser und 600 ml Methanol. Man extrahiert dreimal mit je 200 ml Pentan, zieht das Methanol am Rotationsverdampfer ab, verdünnt mit 200 ml Wasser und extrahiert mit Methylenchlorid. Die Methylenchloridlösung wird über $MgSO_4$ getrocknet,eingeengt und in tert.-Butylmethylether (200 ml) gelöst. Über Nacht kristallisierten hieraus 9 g Edukt (44 mmol). Die Etherlösung wird eingeengt und destilliert.
Ausbeute: 83 g (80 % der Theorie bezogen auf wiedergewonnenes Edukt und Diallylether)
Siedepunkt: 149° C/0,7 mbar bis 159° C/0,9 mbar.
Das Destillat enthält 5 % des Eduktes und 4 % des Diallylethers.
Der Pentanextrakt lieferte 17 g eines Gemisches aus 15 % gewünschtem Produkt und 84 % des Diallylethers.
$[\alpha]_D^{23}$ = -10,5° (c = 1, Methanol).
2) 3S,4S-3-Hydroxy-4-(2-hydroxyethoxy)-pyrrolidin-1-carbonsäure-tert.-butylester
Man löst 64 g (0,24 mol, 91 %ig) 3S,4S-4-Allyloxy-3-hydroxypyrrolidin-1-carbonsäure-tert.-butylester in 250 ml Methanol, kühlt auf 0° C und leitet Ozon durch die Lösung, bis eine nachgeschaltete Waschflasche mit Kaliumiodidlösung das Auftreten von Ozon und damit vollständige Umsetzung anzeigt. Ozonreste werden durch einen Stickstoffstrom ausgetragen, dann wird bei 0° C das entstandene Ozonid mit 18 g Natriumborhydrid reduziert, welches in 1 g-Portionen zugesetzt wird. Anschließend rührt man über Nacht bei Raumtemperatur, engt den Ansatz ein, verdünnt mit Wasser, versetzt mit 20 g Kaliumcarbonat und extrahiert fünfmal mit je 100 ml Methylenchlorid. Man trocknet die organischen Lösungen über Magnesiumsulfat und engt ein.
Ausbeute: 65,8 g (100 % der Theorie).
Das Produkt ist gaschromatografisch 91 %ig.
$[\alpha]_D^{20}$ = -15,2° (c = 0,97, Methanol).
3) 3S,4S-1-tert.-Butoxycarbonyl-3-tosyloxy-4-(2-tosyloxyethoxy)-pyrrolidin
Man legt 2,7 g (10 mmol, 91 %ig) 3S,4S-3-Hydroxy-4-(2-hydroxyethoxy)pyrrolidin-1-carbonsäure-tert.-butylester in 30 ml Methylenchlorid vor, setzt 6 ml 45 %ige Natronlauge und 0,1 g Benzyltriethylammoniumchlorid hinzu und tropft dann unter Kühlung eine Lösung von 2,86 g (20 mmol) Tosylchlorid in 10 ml Methylenchlorid hinzu. Man rührt anschließend noch eine Stunde bei Raumtemperatur, gießt auf 20 ml Wasser, trennt die organische Phase ab und extrahiert die wäßrige Phase mit Methylenchlorid. Die

organischen Phasen werden über Magnesiumsulfat getrocknet und eingeengt. Ausbeute: 5 g (90 % der Theorie).

Das Produkt ist dünnschichtchromatografisch einheitlich.

4) 1S,6R-5-Benzyl-2-oxa-5,8-diazabicyclo[4.3.0]nonan-8-carbonsäure-tert.-butylester

Man erhitzt 87 g (156 mmol) 3S,4S-1-tert.-Butoxycarbonyl-3-tosyloxy-4-(2-tosyloxyethoxy)-pyrrolidin mit 58 g (0,54 mol) Benzylamin in 1 l Xylol über Nacht unter Rückfluß. Man kühlt ab, saugt ausgefallene Salze des Benzylamins ab und engt den Rückstand ein.

Ausbeute: 43 g (58 % der Theorie).

Das Produkt ist gaschromatografisch 67 %ig.

5) 1S,6R-5-Benzyl-2-oxa-5,8-diazabicyclo[4.3.0]nonan

Man erhitzt 43 g (90 mmol) 1S,6R-5-Benzyl-2-oxa-5,8-diazabicyclo[4.3.0]nonan-8-carbonsäure-tert.-butylester in 35 ml konzentrierter Salzsäure und 35 ml Wasser bis zum Ende der Kohlendioxidentwicklung unter Rückfluß. Man stellt mit Kaliumcarbonat alkalisch, extrahiert mit Chloroform, trocknet die organischen Lösungen über $MgSO_4$, engt ein und destilliert zweimal über eine 20 cm-Vigreux-Kolonne.

Ausbeute: 11,1 g (55 % der Theorie)

Siedepunkt: 108 - 115 ° C/0,07 mbar

$[\alpha]_D^{26}$ = -58,3 ° (unverdünnt).

Beispiel G

1) 3R,4R-4-Allyloxy-3-hydroxypyrrolidin-1-carbonsäure-tert.-butylester

Die Umsetzung erfolgt analog Beispiel F1) mit R,R-3,4-Dihydroxypyrrolidin-1-carbonsäure-tert.-butylester:

Siedepunkt: 145 ° C/0,1 mbar

$[\alpha]_D^{23}$ = +9,5 ° (c = 1,0, Methanol)

Das Produkt ist gaschromatografisch 95 %ig.

2) 3R,4R-3-Hydroxy-4-(2-hydroxyethoxy)-pyrrolidin-1-carbonsäure-tert.-butylester

Die Umsetzung erfolgt analog Beispiel F2) mit 3R,4R-4-Allyloxy-3-hydroxypyrrolidin-1-carbonsäure-tert.-butylester:

Ausbeute: 99 % der Theorie (0,175 molarer Ansatz)

$[\alpha]_D^{20}$ = +16,5 ° (c = 0,94, Methanol)

3) 3R,4R-1-tert.-Butoxycarbonyl-3-tosyloxy-4-(2-tosyloxyethoxy)-pyrrolidin

Die Umsetzung erfolgt analog Beispiel F3) mit 3R,4R-3-Hydroxy-4-(2-hydroxyethoxy)-pyrrolidin-1-carbonsäure-tert.-butylester:

Ausbeute: quantitativ (0,11 molarer Ansatz).

4) 1R,6S-5-Benzyl-2-oxa-5,8-diazabicyclo[4.3.0]nonan-8-carbonsäure-tert.-butylester

Die Umsetzung erfolgt analog Beispiel F4) mit 3R,4R-1-tert.-butoxycarbonyl-3-tosyloxy-4-(2-tosyloxyethoxy)-pyrrolidin:

Ausbeute: 40 % der Theorie (0,1 molarer Ansatz).

5) 1R,6S-5-Benzyl-2-oxa-5,8-diazabicyclo[4.3.0]nonan

Die Umsetzung erfolgt analog Beispiel F5) mit 1R,6S-5-Benzyl-2-oxa-5,8-diazabicyclo[4.3.0]nonan-8-carbonsäure-tert.-butylester:

Ausbeute: 63 % der Theorie (40 mmolarer Ansatz)

Siedepunkt: 120 ° C/0,06 mbar

Das Produkt ist gaschromatografisch 95 %ig

$[\alpha]_D^{23}$ = +58,5 ° (unverdünnt).

Beispiel H

1) 1S,6R-2-Oxa-5,8-diazabicyclo[4.3.0]nonan-Dihydrochlorid

Man hydriert 7,5 g (34,4 mmol) 1S,6R-5-Benzyl-2-oxa-5,8-diazabicyclo[4.3.0]nonan in 200 ml Ethanol unter Zusatz von 7 ml konzentrierter Salzsäure an 1 g Palladium-Aktivkohle (10 % Pd) bei 100 ° C und 100 bar. Man saugt den Katalysator ab und wäscht ihn mehrfach mit Wasser. Das wäßrige Filtrat wird eingeengt, worauf der Rückstand kristallisiert. Die Kristalle werden gründlich mit Ethanol verrieben, abgesaugt und an der Luft getrocknet.

Ausbeute: 4,6 g (66,5 % der Theorie)

Schmelzpunkt: 233 - 235 ° C.

2) 1S,6R-2-Oxa-5,8-diazabicyclo[4.3.0]nonan

Man hydriert 59 g (0,27 mol) 1S,6R-5-Benzyl-2-oxa-5,8-diazabicyclo[4.3.0]nonan in 500 ml Ethanol an 5

g Palladium-Aktivkohle (10 % Pd) bei 120°C und 120 bar. Der Katalysator wird abgesaugt, das Filtrat eingeengt und der Rückstand destilliert
Ausbeute: 32,9 g (95 % der Theorie)
Siedepunkt: 65°C/0,03 mbar
Drehwert: $[\alpha]_D^{28}$ = +8,2° (unverdünnt).
ee-Wert: ≧99,5 % (durch Derivatisierung mit Mosher-Reagenz).

Beispiel I

1) 1R,6S-2-Oxa-5,8-diazabicyclo[4.3.0]nonan-Dihydrochlorid
Die Umsetzung erfolgt analog Beispiel H1) mit 1R,6S-5-Benzyl-2-oxa-5,8-diazabicyclo[4.3.0]nonan:
Ausbeute: 77 % der Theorie (23,8 mmolarer Ansatz)
Schmelzpunkt: 230 - 232°C.
2) 1R,6S-2-Oxa-5,8-diazabicyclo[4.3.0]nonan
Die Umsetzung erfolgt analog Beispiel H2) mit 1R,6S-5-Benzyl-2-oxa-5,8-diazabicyclo[4.3.0]nonan:
Ausbeute: 93,3 % der Theorie (1,58 molarer Ansatz)
Siedepunkt: 63 - 65°C/0,03 mbar
Drehwert: $[\alpha]_D^{23}$ = -8,4° (unverdünnt)
ee-Wert: ≧99,5 % (durch Derivatisierung mit Mosher-Reagenz).
1R,6R- bzw. 1S,6S-2-Oxa-5,8-diazabicyclo[4.3.0]-nonan können analog erhalten werden.

Beispiel J

1R,6S-2-Oxa-5,8-diazabicyclo[4.3.0]nonan-Dihydrobromid

1) 1R,6S-5-(1R-Phenylethyl)-8-tosyl-2-oxa-5,8-diazabicyclo[4.3.0]nonan
Man erhitzt 101,8 g (0,196 mol) trans-3-Brom-1-tosyl-4-(2-tosyloxyethoxy)-pyrrolidin und 72 g (0,584 mol) R-(+)-1-Phenylethylamin in 900 ml Xylol über Nacht unter Rückfluß. Man wäscht die abgekühlte Lösung mit 2 n-Natronlauge, trocknet über Kaliumcarbonat, entfernt das Trockenmittel und engt die Lösung ein. Beim Abkühlen scheiden sich aus dem Rückstand Kristalle ab, die abgesaugt und aus einem Gemisch aus 750 ml Waschbenzin und 200 ml n-Butanol umkristallisiert wurden.
Ausbeute: 15 g (39,6 % der Theorie an optisch reinem Material)
Schmelzpunkt: 188°C,
Drehwert: $[\alpha]_D^{28}$ = +103,7° (c = 1,$CHCl_3$).
2) 1R,6S-8-Tosyl-2-oxa-5,8-diazabicyclo[4.3.0]nonan
Man hydriert 13 g (33,6 mmol) 1R,6S-5-(1R-Phenylethyl)-8-tosyl-2-oxa-5,8-diazabicyclo[4.3.0]nonan in 200 ml Ethanol an 2,5 g Palladium-A-Kohle (10 % Pd) bei 100°C und 100 bar. Man saugt den Katalysator ab, engt das Filtrat ein und kristallisiert aus 30 ml Toluol um.
Ausbeute: 7,5 g (79 % der Theorie),
Schmelzpunkt: 160- 161°C,
Drehwert: $[\alpha]_D^{23}$ = +17,5° (c = 1,21, $CHCl_3$).
3) 1R,6S-2-Oxa-5.8-diazabicyclo[4.3.0]nonan-Dihydrobromid
Man löst 7 g (24,8 mmol) 1R,6S-8-Tosyl-2-oxa-5,8-diazabicyclo[4.3.0]nonan in 25 ml 33 %iger Bromwasserstofflösung in Eisessig, setzt 5 g Phenol hinzu und rührt über Nacht bei Raumtemperatur. Man verdünnt mit Diisopropylether, saugt das auskristallisierte Salz ab und trocknet es an der Luft.
Ausbeute: 5,5 g.

Die Derivatisierung mit Mosher-Reagenz und gaschromatografische Analyse zeigt nur ein detektierbares Enantiomeres (ee ≧99,5 %).

Herstellung der Zwischenprodukte

Beispiel Z1

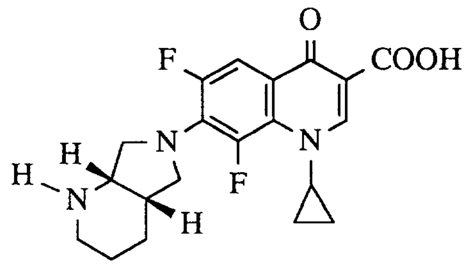

A. 1-Cyclopropyl-7-([S,S]-2,8-diazabicyclo[4.3.0]non-8-yl)-6,8-difluor-1,4-dihydro-4-oxo-3-chinolincarbonsäure

141,5 g (0,5 mol) 1-Cyclopropyl-6,7,8-trifluor-1,4-dihydro-4-oxo-3-chinolincarbonsäure werden in einer Mischung aus 1500 ml Acetonitril und 750 ml Dimethylformamid in Gegenwart von 55 g (0,5 mol) 1,4-Diazabicyclo[2.2.2]octan mit 69,25 g (0,55 mol) (+)-[S,S]-2,8-Diazabicyclo[4.3.0]nonan (ee 99,5 %, GC 99,8 %ig) 1 Stunde unter Rückfluß erhitzt. Die Suspension wird abgekühlt, der Niederschlag abgesaugt, mit Wasser gewaschen und anschließend noch mit 1 l Wasser verrührt (pH 7). Man saugt ab und trocknet bei 60 °C im Umluftschrank.
Ausbeute: 163,4 g (84 % der Theorie),
Schmelzpunkt: 249-251 °C (unter Zersetzung).

B. (-)-1-Cyclopropyl-7-([S,S]-2,8-diazabicyclo[4.3.0]non-8-yl)-6,8-difluor-1,4-dihydro-4-oxo-3-chinolincarbonsäure-Hydrochlorid

6,0 g (15,4 mmol) 1-Cyclopropyl-7-([S,S]-2,8-diazabicyclo[4.3.0]non-8-yl)-6,8-difluor-1,4-dihydro-4-oxo-3-chinolincarbonsäure werden in 40 ml halbkonzentrierter Salzsäure bei 60 °C gelöst und die Lösung des Hydrochlorids filtriert. Das Filtrat wird bis auf die Hälfte eingeengt, in Eis gekühlt und mit 40 ml Ethanol versetzt. Das gelbe Kristallisat wird abgesaugt, mit Ethanol gewaschen und bei 60 °C im Hochvakuum getrocknet, wobei sich die Farbe aufhellt. Man erhält 5,51 g (84 % der Theorie) des Hydrochlorids, das bereits sehr rein ist.

Zur weiteren Reinigung löst man es in 50 ml Wasser in der Hitze. Die gelbe Lösung wird mit 5 ml halbkonzentrierter Salzsäure versetzt, in Eis gekühlt, das ausgefallene Kristallisat abgesaugt, gut mit Ethanol gewaschen und zunächst bei Raumtemperatur und danach im Hochvakuum bei 100 °C getrocknet.
Ausbeute: 4,64 g (70,8 % der Theorie),
Schmelzpunkt: 324-325 °C(unter Zersetzung),
DC (Kieselgel, Dichlormethan/Methanol/17 %iges wäßriges Ammoniak = 30:8:1): einheitlich, $R_f$-Wert: 0,3,
$[\alpha]_D^{25}$: -256 ° (c = 0,5, $H_2O$),
Gehalt (HPLC): 99,4 %ig,

Beispiel Z2

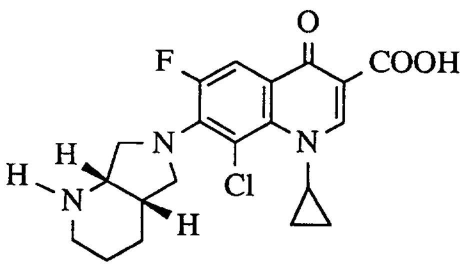

A. 8-Chlor-1-cyclopropyl-7-([S,S]-2,8-diazabicyclo[4.3.0]non-8-yl)-6-fluor-1,4-dihydro-4-oxo-3-chinolincarbonsäure

15 g (50 mmol) 8-Chlor-1-cyclopropyl-6,7-difluor-1,4-dihydro-4-oxo-3-chinolincarbonsäure werden in einer Mischung aus 150 ml Acetonitril/75 ml Dimethylformamid in Gegenwart von 8,25 g (75 mmol) 1,4-Diazabicyclo[2.2.2]octan (DABCO) mit 7,0 g (55 mmol) (+)-[S,S]-2,8-Diazabicyclo[4.3.0]nonan 1 Stunde unter Rückfluß erhitzt. Die Lösung wird gekühlt, zur Kristallisation angekratzt und der ausgefallene Niederschlag nach Stehen über Nacht abgesaugt, mit Acetonitril gewaschen und bei 100 °C im Umluft-Trockenschrank getrocknet.
Ausbeute: 13,5g (66,6 % der Theorie),
Schmelzpunkt: 193-196 °C (unter Zersetzung),
$R_f$-Wert (Kieselgel; Methylenchlorid/Methanol/17 %iges wäßriges $NH_3$ = 30:8:1): 0,4.

B. 8-Chlor-1-cyclopropyl-7-([S,S]-2,8-diazabicyclo[4.3.0]non-8-yl)-6-fluor-1,4-dihydro-4-oxo-3-chinolincarbonsäure-Hydrochlorid

13,1 g (32 mmol) 8-Chlor-1-cyclopropyl-7-([S,S]-2,8-diazabicyclo[4.3.0]non-8-yl)-6-fluor-1,4-dihydro-4-oxo-3-chinolincarbonsäure werden in 50 ml Wasser suspendiert und durch Zugabe von 50 ml halbkonzentrierter Salzsäure in Lösung gebracht. Man filtriert über eine Glasfritte, engt im Vakuum ein und verrührt den Rückstand mit ca. 300 ml absol. Ethanol. Die Suspension wird in Eis gekühlt, der Niederschlag abgesaugt, mit Ethanol gewaschen und zunächst bei Raumtemperatur und danach bei 100 °C im Vakuum getrocknet.
Ausbeute: 13,4 g (93,8 % der Theorie);
Schmelzpunkt: 328-330 °C (unter Zersetzung);
$R_f$-Wert (Kieselgel; Methylenchlorid/Methanol/17 %iges wäßriges $NH_3$ = 30:8:1): 0,4;
Gehalt (HPLC): 99,9 %ig,
$[\alpha]_D^{24}$: -164,4° (c = 0,45, $H_2O$).

Beispiel Z3

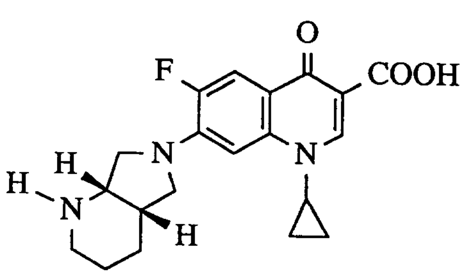

Analog Beispiel Z1 erhält man mit 1-Cyclopropyl-6,7-difluor-1,4-dihydro-4-oxo-3-chinolincarbonsäure:

A. 1-Cyclopropyl-7-([S,S]-2,8-diazabicyclo[4.3.0]non-8-yl)-6-fluor-1,4-dihydro-4-oxo-3-chinolincarbonsäure,
Schmelzpunkt: 256-258 ° C(unter Zersetzung).
B. 1-Cyclopropyl-7-([S,S]-2,8-diazabicyclo[4.3.0]non-8-yl)-6-fluor-1,4-dihydro-4-oxo-3-chinolincarbonsäure-Hydrochlorid,
Schmelzpunkt: >320 ° C(unter Zersetzung),
$[\alpha]_D^{26}$: -90,6 ° (c = 0,48, $H_2O$).

Beispiel Z4

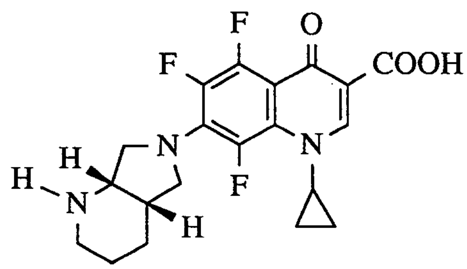

A. 6 g (20 mmol) 1-Cyclopropyl-5,6,7,8-tetrafluor-1,4-dihydro-oxo-3-chinolincarbonsäure werden in 40 ml Acetonitril/20 ml N-Methylpyrrolidon in Gegenwart von 2,2 g (20 mmol) 1,4-Diazabicyclo[2.2.2]octan mit 2,7 g (21,4 mmol) (+)-[S,S]-2,8-Diazabicyclo[4.3.0]nonan 1 Stunde unter Rückfluß erhitzt. Die erhaltene Suspension wird abgekühlt, der Niederschlag abgesaugt, mit Acetonitril gewaschen und bei 100 ° C/12 mbar getrocknet.
Ausbeute: 6,7 g (82,3 % der Theorie) 1-Cyclopropyl-7-([S,S]-2,8-diazabicyclo[4.3.0]non-8-yl)-5,6,8-trifluor-1,4-dihydro-4-oxo-3-chinolincarbonsäure,
Schmelzpunkt: 257-259 ° C (unter Zersetzung); nach Umkristallisation aus Glykolmonomethylether: Schmelzpunkt: 260-265 ° C (unter Zersetzung).
B. 1,5 g (3,7 mmol) des Produkts aus Stufe A werden in 6 ml 1n-Salzsäure eingetragen. Nach kurzer Zeit fällt das Hydrochlorid aus, das abgesaugt, zweimal mit je 5 ml Ethanol gewaschen und bei 100 ° C/12 mbar getrocknet wird.
Ausbeute: 1,4 g (85,7 % der Theorie) 1 -Cyclopropyl-7-([S,S]-2,8-diazabicyclo[4.3.0]non-8-yl)-5,6,8-trifluor-1,4-dihydro-4-oxo-3-chinolincarbonsäure-Hydrochlorid,
Schmelzpunkt: >310 ° C (unter Zersetzung),
$[\alpha]_D^{26}$: -272 ° (c = 0,5, $H_2O$).

Beispiel Z5

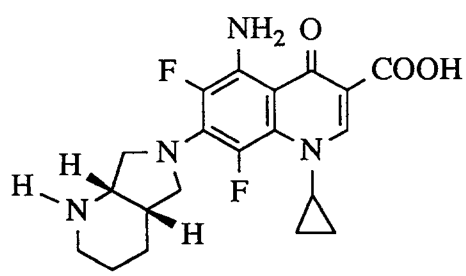

x HCl

5,2 g (13 mmol) des Produkts aus Beispiel Z4A werden in 80 ml Pyridin im Autoklaven mit 15 ml flüssigem Ammoniak versetzt und 12 Stunden auf 130 ° C erhitzt. Anschließend wird abgekühlt, der Autoklav entspannt, die Mischung eingeengt, der Rückstand mit Acetonitril im Ultraschallbad behandelt. Der ungelöste Niederschlag wird abgesaugt, der Rückstand in etwa 150 ml Wasser in der Hitze gelöst, die Lösung filtriert und das Hydrochlorid mit 10 ml halbkonzentrierter Salzsäure ausgefällt, abgesaugt und bei 100 ° C im

Umluft-Trockenschrank getrocknet. Das erhaltene Produkt wird in 100 ml Glykolmonomethylether bei 110-115°C suspendiert und durch Zugabe von 38 ml halbkonzentrierter Salzsäure in Lösung gebracht. Die Lösung wird heiß über eine Glasfritte filtriert, abgekühlt und das ausgefallene gelbe Kristallisat abgesaugt, mit Ethanol gewaschen und bei 120°C/12 mbar getrocknet.
Ausbeute: 2,5 g (44 % der Theorie) 5-Amino-1-cyclopropyl-7-([S,S]-2,8-diazabicyclo[4.3.0]non-8-yl)-6,8-difluor-1,4-dihydro-4-oxo-3-chinolincarbonsäure-Hydrochlorid,
Schmelzpunkt: >335°C (unter Zersetzung; bereits unterhalb 335°C Dunkelfärbung),
$[\alpha]_D^{28}$: -280,8° (c = 0,53, $H_2O$).

Beispiel Z6

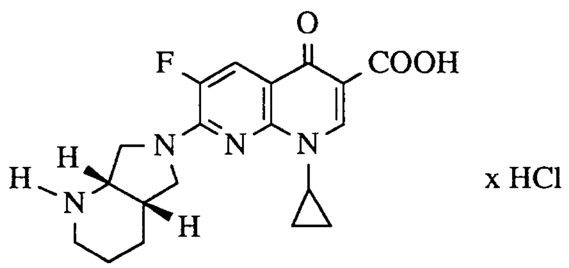

1,4 g (5 mmol) 7-Chlor-1-cyclopropyl-6-fluor-1,4-dihydro-4-oxo-1,8-naphthyridin-3-carbonsäure werden in 15 ml Acetonitril mit 1,3 g (10,3 mmol) (+)-[S,S]-2,8-Diazabicyclo[4.3.0]nonan unter Wasserausschluß 1 Stunde bei Raumtemperatur gerührt. Nach Stehen über Nacht wird abgesaugt, mit Acetonitril gewaschen und zur Reinigung an Kieselgel chromatographiert (Laufmittel: Methylenchlorid/Methanol/17 %iges wäßriges Ammoniak 30:8:1; $R_f$-Wert: 0,4). Die erhaltene 1-Cyclopropyl-7-([S,S]-diazabicyclo[4.3.0]non-8-yl)-6-fluor-1,4-dihydro-4-oxo-1,8-naphthyridin-3-carbonsäure wird in 15 ml halbkonzentrierter Salzsäure gelöst, die Lösung eingedampft und der Rückstand mit Ethanol verrührt. Der Niederschlag wird abgesaugt, mit Ethanol gewaschen und bei 120°C/12 mbar getrocknet.
Ausbeute: 960 mg (47 % der Theorie) 1-Cyclopropyl-7-([S,S]-2,8-diazabicyclo[4.3.0]non-8-yl)-6-fluor-1,4-dihydro-4-oxo-1,8-naphthyridin-3-carbonsäure-Hydrochlorid,
Schmelzpunkt: 345-346°C(unter Zersetzung),
$[\alpha]_D^{30}$: +5,4° (c = 0,5, $H_2O$).

Beispiel Z7

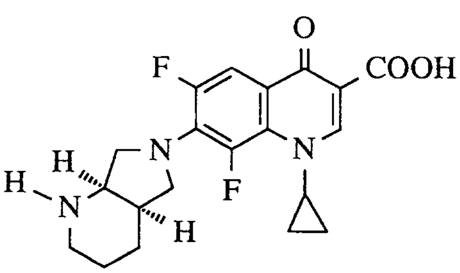

Analog Beispiel Z1 erhält man mit (-)-[R,R]-2,8-Diazabicyclo[4.3.0]nonan:

A. 1-Cyclopropyl-7-([R,R]-2,8-diazabicyclo[4.3.0]non-8-yl)-6,8-difluor-1,4-dihydro-4-oxo-3-chinolincarbonsäure,
Schmelzpunkt: 247-249°C (unter Zersetzung).

B. 1-Cyclopropyl-7-([R,R]-2,8-diazabicyolo[4.3.0]non-8-yl)-6,8-difluor-1,4-dihydro-4-oxo-chinolincarbonsäure-Hydrochlorid,
Schmelzpunkt: 322-326°C (unter Zersetzung),
Gehalt (HPLC): 99,4 %ig, ee: 98,6 %,
$[\alpha]_D^{24}$: +250° (c = 0,5, $H_2O$).

Beispiel Z8

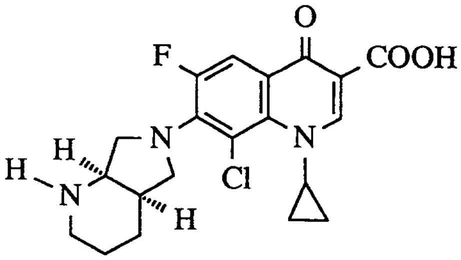

Analog Beispiel Z2 erhält man mit (-)-[R,R]-2,8-Diazabicyclo[4.3.0]nonan:

A. 8-Chlor-1-cyclopropyl-7-[R,R]-2,8-diazabicyclo[4.3.0]non-8-yl)-6-fluor-1,4-dihydro-4-oxo-chinolincarbonsäure,
Schmelzpunkt: 192-195 °C (unter Zersetzung).

B. 8-Chlor-1-cyclopropyl-7-[R,R]-2,8-diazabicyclo[4.3.0]non-8-yl)-6-fluor-1,4-dihydro-4-oxo-3-chinolincarbonsäure-Hydrochlorid,
Schmelzpunkt: 323-324 °C (unter Zersetzung),
Gehalt (HPLC): 99,9 %ig,
$[\alpha]_D^{24}$: +164,5° (c = 0,53, $H_2O$),

| $C_{20}H_{21}ClFN_3O_3.HCl$ (442,3) | | | | |
|---|---|---|---|---|
| Berechnet: | C 54,3 | H 5,0 | N 9,5 | Cl 16,0 |
| Gefunden: | C 54,2 | H 5,0 | N 9,5 | Cl 16,1 |

Beispiel Z9

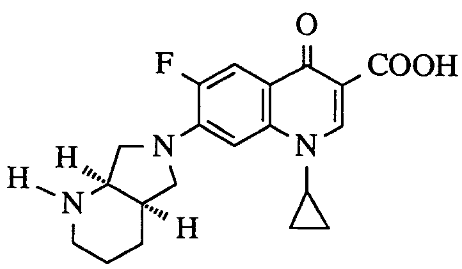

Analog Beispiel Z1 erhält man aus 1-Cyclopropyl-6,7-difluor-1,4-dihydio-4-oxo-3-chinolincarbonsäure und (-)-[R,R]-2,8-Diazabicyclo[4.3.0]nonan:

A. 1-Cyclopropyl-7-([R,R]-2,8-diazabicyclo[4.3.0]non-8-yl)-6-fluor-1,4-dihydio-4-oxo-3-chinolincarbonsäure,
Schmelzpunkt: 254-258 °C (unter Zersetzung).

B. 1-Cyclopropyl-7-([R,R]2,8-diazabicyclo[4.3.0]non-8-yl)-6-fluor-1,4-dihydro-4-oxo-3-chinolincarbonsäure,
Schmelzpunkt: oberhalb 320 °C Zersetzung,
$[\alpha]_D^{24}$: +92,5° (c =0,53, $H_2O$).

Beispiel Z10

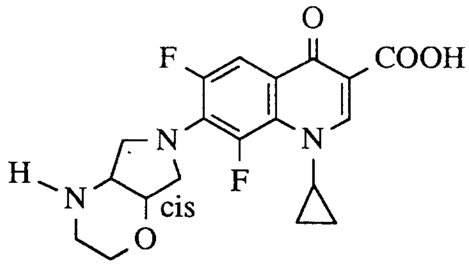

A. 1-Cyclopropyl-6,8-difluor-1,4-dihydro-7-(cis-2-oxa-5,8-diazabicyclo[4.3.0]non-8-yl)-4-oxo-3-chinolincarbonsäure:

1,43 g (5 mmol) 1-Cyclopropyl-6,7,8-trifluor-1,4-dihydro-4-oxo-3-chinolincarbonsäure werden in einer Mischung aus 15 ml Acetonitril/75 ml Dimethylformamid in Gegenwart von 0,67 g (6 mmol) 1,4-Diazabicyclo[2.2.2]octan mit 0,74 g (5,4 mmol) 93 %igem cis-2-Oxa-5,8-diazabicyclo[4.3.0]nonan 1 Stunde unter Rückfluß erhitzt. Die Suspension wird eingeengt, der Rückstand mit Wasser verrührt, der Niederschlag abgesaugt und bei 80 °C im Vakuum getrocknet.

Ausbeute: 1,67 g (85,4 % der Theorie),

Schmelzpunkt: 210-212 °C (unter Zersetzung).

B. 1-Cyclopropyl-6,8-difluor-1,4-dihydro-7-(cis-2-oxa-5,8-diazabicyclo[4.3.0]non-8-yl)-4-oxo-3-chinolincarhonsäure-Hydrochlorid:

1,6 g (4 mmol) des Produkts aus Stufe A werden in 120 ml halbkonzentrierter Salzsäure bei 60 °C gelöst, die Lösung eingeengt, der Rückstand mit Ethanol und der Niederschlag abgesaugt und bei 90 °C im Vakuum getrocknet.

Ausbeute: 1,57 g,

Schmelzpunkt: 300-303 °C (unter Zersetzung),

Gehalt (HPLC): 97 %ig.

C. Analog Beispiel Z10A erhält man mit 1R,6S-2-Oxa-5,8-diazabicyclo[4.3.0]nonan 1-Cyclopropyl-6,8-difluor-1,4-dihydro-7-(1R,6S-2-oxa-5,8-diazabicyclo[4.3.0]non-8-yl)-4-oxo-3-chinolincarbonsäure vom Schmelzpunkt 204 - 206 °C (unter Zersetzung).

D. Analog Beispiel Z10B erhält man mit dem Betain aus Beispiel Z10C 1-Cyclopropyl-6,8-difluor-1,4-dihydro-7-(1R,6S-2-oxa-5,8-diazabicyclo[4.3.0]non-8-yl)-4-oxo-3-chinolincarbonsäure-Hydrochlorid vom Schmelzpunkt 324-325 °C (unter Zersetzung).

$[\alpha]_D^{24}$: -241 ° (c = 0,59, $H_2O$).

E. Analog Beispiel Z10A erhält man mit 1S,6R-2-Oxa-5,8-diazabicyclo[4.3.0]nonan 1-Cyclopropyl-6,8-difluor-1,4-dihydro-7-(1S,6R-2-oxa-5,8-diazabicyclo[4.3.0]non-8-yl)-4-oxo-3-chinolincarbonsäure vom Schmelzpunkt 204 - 206 °C (unter Zersetzung).

$[\alpha]_D^{25}$: + 248 ° (c = 0,57, DMF).

F. Analog Beispiel Z10B erhält man mit dem Betain aus Beispiel Z10E 1-Cyclopropyl-6,8-difluor-1,4-dihydro-7-(1S,6R-2-oxa-5,8-diazabicyclo[4.3.0]non-8-yl)-oxo-3-chinolincarbonsäure-Hydrochlorid vom Schmelzpunkt 323 °C (unter Zersetzung).

$[\alpha]_D^{26}$: + 238 ° (c = 0,5, $H_2O$).

Beispiel Z11

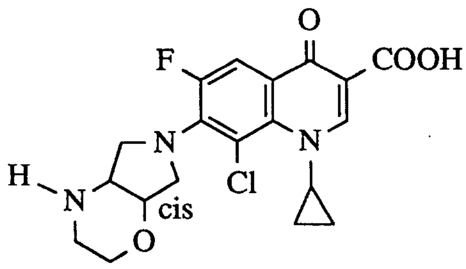

Analog Beispiel Z10 erhält man mit 8-Chlor-1-cyclopropyl-6,7-difluor-1,4-dihydro-4-oxo-3-chinolincarbonsäure:

A. 8-Chlor-1-cyclopropyl-6-fluor-1,4-dihydro-7-(cis-2-oxa-5,8-diazabicyclo[4.3.0]non-8-yl)-4-oxo-3-chinolincarbonsäure,
Schmelzpunkt: 180-185 ° C (unter Zersetzung).

B. 8-Chlor-1-cyclopropyl-6-fluor-1,4-dihydro-7-(cis-2-oxa-5,8-diazabicyclo[4.3.0]non-8-yl)-4-oxo-3-chinolincarbonsäure-Hydrochlorid,
Schmelzpunkt: 227-232 ° C (unter Zersetzung).

C. 8-Chlor-1-cyclopropyl-6-fluor-1,4-dihydro-7-(1R,6S-2-oxa-5,8-diazabicyclo[4.3.0]non-8-yl)-4-oxo-3-chinolincarbonsäure,
Schmelzpunkt: 186-188 ° C (unter Zersetzung).
$[\alpha]_D^{26}$: -269 ° (c = 0,5, DMF).

D. 8-Chlor-1-cyclopropyl-6-fluor-1,4-dihydro-7-(1R,6S-2-oxa-5,8-diazabicyclo[4.3.0]non-8-yl)-4-oxo-3-chinolincarbonsäure-Hydrochlorid,
Schmelzpunkt: 278-280 ° C (unter Zersetzung).
$[\alpha]_D^{24}$: -208 ° (c = 0,5, $H_2O$).

E. 8-Chlor-1-cyclopropyl-6-fluor-1,4-dihydro-7-(1S,6R-2-oxa-5,8-diazabicyclo[4.3.0]non-8-yl)-4-oxo-3-chinolincarbonsäure,
Schmelzpunkt: 188-190 ° C (unter Zersetzung).
$[\alpha]_D^{25}$: + 270 ° (c = 0,5, DMF).

F. 8-Chlor-1-cyclopropyl-6-fluor-1,4-dihydro-7-(1S,6R-2-oxa-5,8-diazabicyclo[4.3.0]non-8-yl)-4-oxo-3-chinolincarbonsäure-Hydrochlorid,
Schmelzpunkt: 292-294 ° C (unter Zersetzung).
$[\alpha]_D^{27}$: + 193 ° (c = 0,5, $H_2O$).

Beispiel Z12

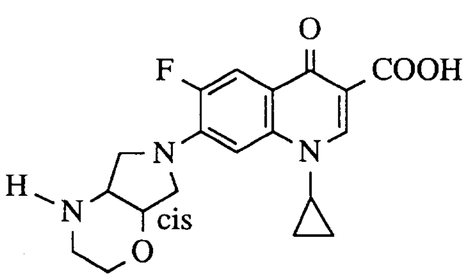

Analog Beispiel Z10A erhält man mit 1-Cyclopropyl-6,7-difluor-1,4-dihydro-4-oxo-3-chinolincarbonsäure:

A. 1-Cyclopropyl-6-fluor-1,4-dihydro-7-(cis-2-oxa-5,8-diazabicyclo[4.3.0]non-8-yl)-4-oxo-3-chinolincarbonsäure,
Schmelzpunkt: 246-249 ° C (unter Zersetzung) (aus Glykolmonomethylether).

B. 1-Cyclopropyl-6-fluor-1,4-dihydro-7-(1R,6S-2-oxa-5,8-diazabicyclo[4.3.0]non-8-yl)-4-oxo-3-chinolincarbonsäure,

Schmelzpunkt: 243-245 ° C (unter Zersetzung).

C. 1-Cyclopropyl-6-fluor-1,4-dihydro-7-(1R,6S-2-oxa-5,8-diazabicyclo[4.3.0]non-8-yl)-4-oxo-3-chinolincarbonsäure-Hydrochlorid,

Schmelzpunkt: 300 ° C (Zersetzung)

$[\alpha]_D^{23}$: -99 ° (c = 0,5, $H_2O$).

Beispiel Z13

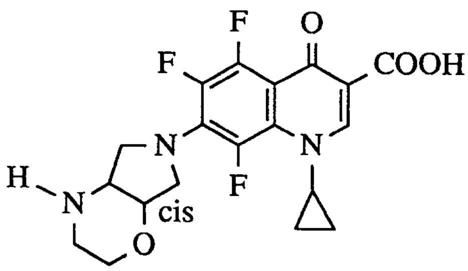

Analog Beispiel Z10A erhält man mit 1-Cyclopropy-5,6,7,8-tetrafluor-1,4-dihydro-4-oxo-3-chinolincarbonsäure:

A. 1-Cyclopropyl-5,6,8-trifluor-1,4-dihydro-7-(cis-2-oxa-5,8-diazabicyclo[4.3.0]non-8-yl)-4-oxo-3-chinolincarbonsäure,

Schmelzpunkt: 210-216 ° C (unter Zersetzung).

B. 1-Cyclopropyl-5,6,8-trifluor-1,4-dihydro-7(1R,6S-2-oxa-5,8-diazabicyclo[4.3.0]non-8-yl)-4-oxo-3-chinolincarbonsäure,

Schmelzpunkt: 234-237 ° C (unter Zersetzung).

$[\alpha]_D^{24}$: -287 ° (c = 0,5, DMF).

C. 1-Cyclopropyl-5,6,8-trifluor-1,4-dihydro-7-(1S,6R-2-oxa-5,8-diazabicyclo[4.3.0]non-8-yl)-4-oxo-3-chinolincarbonsäure,

Schmelzpunkt: 236-237 ° C (unter Zersetzung).

$[\alpha]_D^{24}$: +282 ° (c = 0,5, DMF).

Beispiel Z14

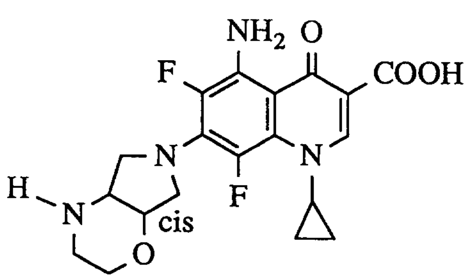

A. 4,1 g (10 mmol) des Produkts aus Beispiel Z13A werden in 40 ml Pyridin mit 5 ml flüssigem Ammoniak versetzt und im Autoklaven 10 Stunden auf 130 ° C erhitzt. Nach dem Abkühlen wird der Niederschlag abgesaugt, mit Wasser gewaschen und bei 100 ° C im Umluft-Trockenschank getrocknet. Das Rohprodukt (2 g) wird durch Umkristallisation aus Glykolmonomethylether gereinigt: gelbes Kristallisat.

Ausbeute: 1,3 g (31 % der Theorie) 5-Amino-1-cyclopropyl-6,8-difluor-1,4-dihydro-7-(cis-2-oxa-5,8-diazabicyclo[4.3.0]non-8-yl)-4-oxo-3-chinolincarbonsäure,

Schmelzpunkt: 233-240 ° C (unter Zersetzung).

B. Analog erhält man mit dem Produkt aus Beispiel Z13C 5-Amino-1-cyclopropyl-6,8-difluor-1,4-dihydro-7-(1R,6S-2-oxa-5,8-diazabicyclo[4.3.0]non-8-yl)-4-oxo-3-chinolincarbonsäure,

Schmelzpunkt: 212-214 °C (unter Zersetzung),
$[\alpha]_D^{25}$: -260 ° (c = 0,5, DMF).
C. Analog erhält man mit dem Produkt aus Beispiel Z13C 5-Amino-1-cyclopropyl-6,8-difluor-1,4-dihydro-7-(1S,6R-2-oxa-5,8-diazabicyclo[4.3.0]non-8-yl)-4-oxo-3-chinolincarbonsäure,
Schmelzpunkt: 213-215 °C (unter Zersetzung),
$[\alpha]_D^{26}$: +261 ° (c = 0,5, DMF),
Massenspektrum: $^m/_e$ 406 ($M^+$,95 %), 346, 249, 98, 41, 28 (100 %).

BeispielZ15

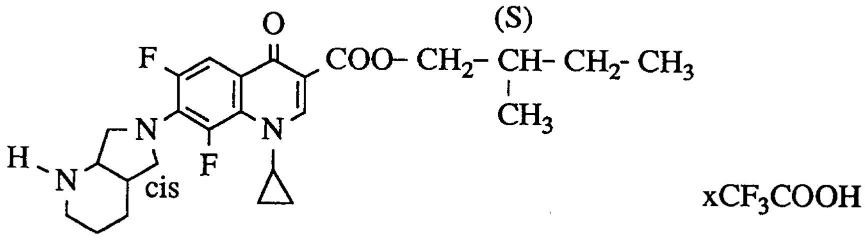

A. 7-(2-tert.-Butoxycarbonyl-2,8-diazabicyclo[4.3.0]non-8-yl)-1-cyclopropyl-6,8-difluor-1,4-dihydro-4-oxo-3-chinolincarbonsäure
7,8 g (20 mmol) 1-Cyclopropyl-7-(2,8-diazabicyclo[4.3.0]non-8-yl)-6,8-difluor-1,4-dihydro-4-oxo-3-chinolincarbonsäure werden in einer Mischung aus 60 ml Dioxan/Wasser (2:1) und 20 ml 1n-Natronlauge gelöst und unter Eiskühlung und Rühren mit 5,24g (24 mmol) Pyrokohlensäure-di-tert.-butylester versetzt. Man rührt 1 Stunde bei Raumtemperatur nach und läßt über Nacht stehen. Der ausgefallene Niederschlag wird abgesaugt, mit 250 ml Wasser gewaschen und über Nacht bei 50 °C im Umluft-Trockenschrank getrocknet.
Ausbeute: 9,34 g (95,5 % der Theorie),
Schmelzpunkt: 216-219 °C (unter Zersetzung).
B. 7-(2-tert.-Butoxycarbonyl-2,8-diazabicyclo[4.3.0]non-8-yl)-1-cyclopropyl-6,8-difluor-1,4-dihydro-4-oxo-3-chinolincarbonsäure-(2S-methyl-1-butylester)
2,15 g (4,4 mmol) des Produktes aus Stufe A werden in 60 ml Tetrahydrofuran/Wasser (1:1) bei Raumtemperatur suspendiert und 1,65 g (5 mmol) Cäsiumcarbonat zugegeben. Man läßt 20 Minuten im Ultraschallbad bei etwa 40 °C reagieren, destilliert etwa 40 ml des Lösungsmittels bei 40 °C/12 mbar ab und lyophilisiert die zurückbleibende Lösung, wobei das leichtlösliche rohe Cësiumsalz erhalten wird. 3,3 g dieses rohen Salzes werden in 40 ml Dimethylformamid gelöst und mit 1,4g S(+)-1-Brom-2-methylbutan versetzt und über Nacht im Ultraschallbad bei 40-50 °C umgesetzt. Die erhaltene Suspension wird eingeengt, der Rückstand mit Wasser versetzt und mit Methylenchlorid extrahiert. Nach dem Trocknen mit Natriumsulfat wird die Losung eingeengt und der Rückstand chromatographisch gereinigt (Kieselgel, Laufmittel: Methylenchlorid/Methanol 95:5).
Ausbeute: 950 mg (38 % der Theorie),
Schmelzpunkt: 72-83 °C (unter Zersetzung).
C. 1-Cyclopropyl-7-(2,8-diazabicyclo[4.3.0]non-8-yl)-6,8-difluor-1,4-dihydro-4-oxo-3-chinolincarbonsäure-(2S-methyl-1-butylester)-Trifluoracetat
570 mg (1 mmol) des Produktes der Stufe B werden in 3 ml Trifluoressigsäure bei Raumtemperatur gelöst und die Lösung bei 60 °C/12 mbar eingeengt. Das erhaltene zähe Öl wird mit 5 ml Ether verrührt, wobei ein Festprodukt anfällt. Dieses wird abgesaugt, mit Ether gewaschen und bei 80 °C im Hochvakuum getrocknet.
Ausbeute: 450 mg (78 % der Theorie),
Schmelzpunkt: 214-216 °C (unter Zersetzung),
$[\alpha]_D^{25}$: + 2,8 ° (c = 0,5, DMF).

Beispiel Z16

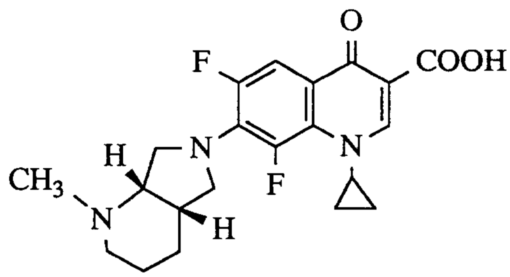

Analog Beispiel Z1 erhält man mit [S,S]-2-Methyl-2,8-diazabicyclo[4.3.0]nonan:

A. 1-Cyclopropyl-6,8-difluor-1,4-dihydro-7-([S,S]-2-methyl-2,8-diazabicyclo[4.3.0]non-8-yl)-4-oxo-3-chinolincarbonsäure,
Schmelzpunkt: 230-233 °C (unter Zersetzung) (umkristallisiert aus Glykolmonomethylether);

B. 1-Cyclopropyl-6,8-difluor-1,4-dihydro-7-([S,S]-2-methyl-2,8-diazabicyclo[4.3.0]non-8-yl)-4-oxo-3-chinolincarbonsäure-Hydrochlorid,
Schmelzpunkt: 258-260 °C (unter Zersetzung), $[\alpha]_D^{25}$ : -216,3 °C (c = 1, $H_2O$).

Beispiel Z17

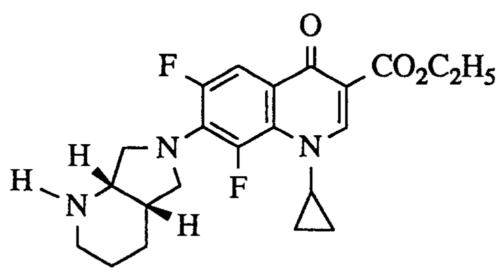

1,52 g (5 mmol) 1-Cyclopropyl-6,7,8-trifluor-1,4-dihydro-4-oxo-3-chinolincarbonsäure-ethylester werden in 30 ml Acetonitril mit 550 mg (5 mmol) 1,4-Diazabicyclo[2.2.2]octan und 760 mg (6 mmol) (+)-[S,S]-2,8-Diazabicyclo[4.3.0]nonan 2 Stunden bei 50 °C und 2 Stunden bei 60 °C umgesetzt. Nach dem Abkühlen wird die erhaltene Suspension abgesaugt, der Niederschlag mit Wasser gewaschen und bei 90 °C im Vakuum getrocknet.
Ausbeute: 0,99 g (47,5 % der Theorie) 1-Cyclopropyl-7-([S,S]-2,8-diazabicyclo[4.3.0]non-8-yl)-6,8-difluor-1,4-dihydro-4-oxo-3-chinolincarbonsäureethylester,
Schmelzpunkt: 194-195 °C (aus Acetonitril),
$[\alpha]_D^{23}$: -188,9 ° (c = 0,51, $CHCl_3$).

Beispiel Z18

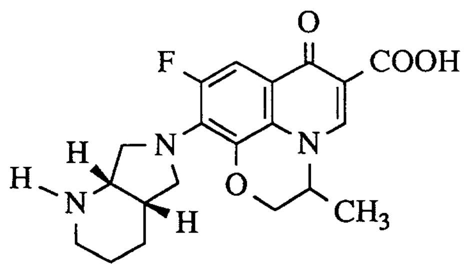

1,4 g (5 mmol) 9,10-Difluor-2,3-dihydro-3-methyl-7-oxo-7H-pyrido[1,2,3-de][1,4]benzoxacin-6-carbonsäure werden in 15 ml Acetonitril/7,5 ml Dimethylformamid mit 0,85 g (7,7 mmol) 1,4-Diazabicyclo[2.2.2]octan und 0,7 g (5,6 mmol) (+)-[S,S]-2,8-Diazabicyclo[4.3.0]nonan analog Beispiel Z1A umgesetzt.
Ausbeute: 1,24 g (64 % der Theorie) 10-([S,S]-2,8-Diazabicyclo[4.3.0]non-8-yl)-9-fluor-2,3-dihydro-3-methyl-7-oxo-7H-pyrido[1,2,3-de][1,4]benzoxacin-6-carbonsäure,
Schmelzpunkt: 265-268 °C (unter Zersetzung),
$[\alpha]_D$: -232,2 ° (c = 0,58, $CHCl_3$).

Analog erhält man auch 3S-10-([S,S]-2,8-Diazabicyclo[4.3.0]non-8-yl)-9-fluor-2,3-dihydro-3-methyl-7-oxo-7H-pyrido[1,2,3-de][1,4]benzoxacin-6-carbonsäure.

Beispiel Z19

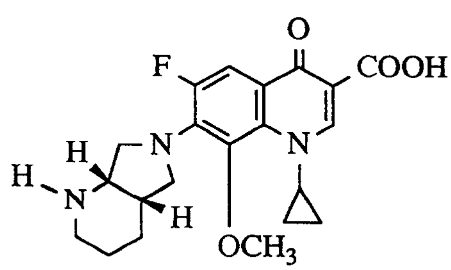

1 -Cyclopropyl-6,7-difluor-1,4-dihydro-8-methoxy-4-oxo-3-chinolincarbonsäure wird analog Beispiel Z1A umgesetzt und das Umsetzungsprodukt chromatographisch gereinigt (Kieselgel, Laufmittel: Methylenchlorid/Methanol/17 %iges wäßriges Ammoniak = 30:8:1).

Man erhält 1-Cyclopropyl-7-([S,S]-2,8-diazabicyclo[4.3.0]non-8-yl)-6-fluor-1,4-dihydro-8-methoxy-4-oxo-3-chinolincarbonsäure vom Schmelzpunkt 203-208 °C (unter Zersetzung).
$[\alpha]_D^{23}$: -193 ° (c = 0,4, $CHCl_3$)

Beispiel Z20

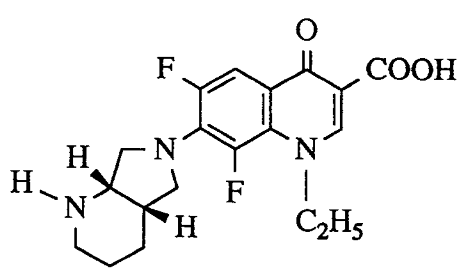

Man setzt analog Beispiel Z1A mit 1-Ethyl-6,7,8-trifluor-1,4-dihydro-4-oxo-3-chinolincarbonsäure um und erhalt 1-Ethyl-7-([S,S]-2,8-diazabicyclo[4.3.0]non-8-yl)-6,8-difluor-1,4-dihydro-4-oxo-3-chinolincarbonsäure

vom Schmelzpunkt 236-239 °C (unter Zersetzung) (umkristallisiert aus Glykolmonomethylether); $[\alpha]_D^{23}$: -186,3° (c = 0,3, $CHCl_3$).

Beispiel Z21

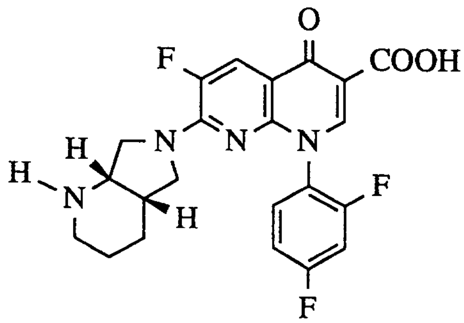

x HCl

A. 7-([S,S]-2,8-Diazabicyclo[4.3.0]non-8-yl)-1-(2,4-difluorphenyl)-6-fluor-1,4-dihydro-4-oxo-1,8-naphthyridin-3-carbonsäureethylester

1,9 g (5 mmol) 7-Chlor-1-(2,4-difluorphenyl)-6-fluor-1,4-dihydro-4-oxo-1,8-naphthyridin-3-carbonsäureethylester werden in 20 ml Acetonitril in Gegenwart von 560 mg (5 mmol) 1,4-Diazabicyclo[2.2.2]octan mit 680 mg (5,4 mmol) [S,S]-2,8-Diazabicyclo[4.3.0]nonan 3 Stunden bei 10 °C verrührt. Die Suspension wird abgesaugt, mit Wasser gewaschen und getrocknet. Man erhalt 0,35 g Produkt. Durch Einengen der Mutterlaugen, Verrühren des Rückstandes mit Wasser, Isolierung des ungelösten Produktes und chromatographische Reinigung (Kieselgel, Laufmittel: Dichlormethan/Methanol/17 %iges wäßriges Ammoniak) werden weitere 0,7 g Produkt isoliert.

Gesamtausbeute: 1,05 g (44 % der Theorie),

Schmelzpunkt: 184-185 °C (unter Zersetzung),

$[\alpha]_D^{23}$: +6,8° (c = 0,46, $CHCl_3$).

B. 7-([S,S]-2,8-Diazabicyclo[4.3.0]non-8-yl)-1-(2,4-difluorphenyl)-6-fluor-1,4-dihydro-4-oxo-1,8-naphthyridin-3-carbonsäure-Hydrochlorid

0,8 g (1,7 mmol) des Produkts aus Stufe A werden in einer Mischung aus 10 ml Essigsäure und 8 ml halbverdünnter Salzsäure 4 Stunden unter Rückfluß erhitzt. Die Mischung wird eingeengt, der Rückstand mit wenig Wasser verrührt, der Niederschlag abgesaugt, mit eiskaltem Ethanol gewaschen und getrocknet.

Ausbeute: 0,67 g (83 % der Theorie),

Schmelzpunkt: 324-326 °C (unter Zersetzung),

$[\alpha]_D^{25}$: +10,8° (c = 0,37, DMF).

Beispiel Z22

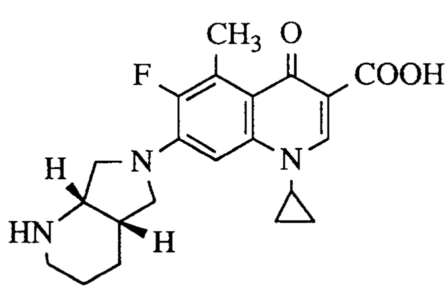

0,56 g (2 mmol) 1-Cyclopropyl-6,7-difluor-1,4-dihydro-5-methyl-4-oxo-3-chinolincarbonsäure werden mit 0,38 g (3 mmol) [S,S]-2,8-Diazabicyclo[4.3.0]nonan und 0,45 g (4 mmol) 1,4-Diazabicyclo[2.2.2]octan in 3,5 ml Dimethylsulfoxid 2 Stunden auf 120 °C erhitzt. Nach Abkühlen wird das Lösungsmittel im Hochvakuum entfernt. Der Rückstand wird mit Acetonitril aufgenommen. Man trennt den Feststoff ab, wäscht ihn mit

Acetonitril und trocknet bei 60 bis 80 ° C.
Ausbeute: 0,5 g (65 % der Theorie) 1-Cyclopropyl-7-([S,S]-2,8-diazabicyclo[4.3.0]non-8-yl)-6-fluor-1,4-dihydro-5-methy-4-oxo-3-chinolincarbonsäure,
Schmelzpunkt: 217-219 ° C (unter Zersetzung),
$[\alpha]_D$: -119 ° (c = 0,5, DMF).

Beispiel Z23

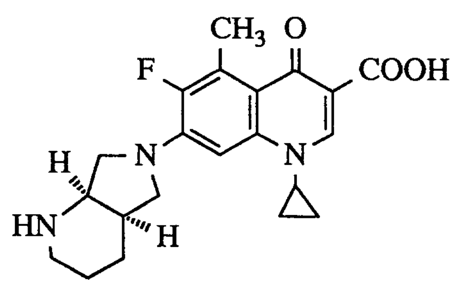

A. 837 mg (3 mmol) 1-Cyclopropyl-6,7-difluor-1,4-dihydro-5-methyl-4-oxo-3-chinolincarbonsäure werden in einer Mischung aus 10 ml Acetonitril und 5 ml Dimethylformamid mit 1,1 g (10 mmol) 1,4-Diazabicyclo[2.2.2]octan und 665 mg (3,3 mmol) 1R,6S-2-Oxa-5,8-diazabicyclo[4.3.0]nonan-Dihydrochlorid 2 Stunden unter Rückfluß erhitzt. Die Mischung wird eingedampft, der Rückstand mit 30 ml Wasser verrührt, der Niederschlag abgesaugt und bei 80 ° C im Vakuum getrocknet.
Ausbeute: 400 mg (34 % der Theorie) 1-Cyclopropyl-6-fluor-1,4-dihydro-5-methyl-7-(1R,6S-2-oxa-5,8-diazabicyclo[4.3.0]non-8-yl)-4-oxo-3-chinolincarbonsäure,
Schmelzpunkt: 213-214 ° C (unter Zersetzung).
B. 0,4 g des Betains aus Stufe A werden in 5 ml halbkonzentrierter Salzsäure bei Raumtemperatur gelöst, die Lösung wird eingeengt und der Rückstand mit etwa 3 ml Ethanol verrührt. Der Niederschlag wird abgesaugt und bei 80 ° C/12 mbar getrocknet.
Ausbeute: 290 mg (66 % der Theorie) 1-Cyclopropyl-6-fluor-1,4-dihydro-5-methyl-7-(1R,6S-2-oxa-5,8-diazabicyclo[4.3.0]non-8-yl)-4-oxo-3-chinolincarbonsäure-Hydrochlorid,
Schmelzpunkt: 305-308 ° C (unter Zersetzung),
$[\alpha]_D^{23}$: -79 ° (c = 0,52, $H_2O$).

Beispiel Z24

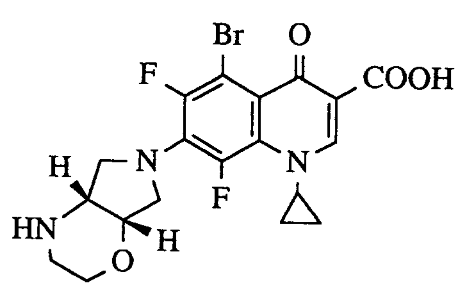

362 mg (1 mmol) 5-Brom-1-cyclopropyl-6,7,8-trifluor-1,4-dihydro-4-oxo-3-chinolincarbonsäure werden in einer Mischung aus 3 ml Acetonitril und 1,5 ml Dimethylformamid mit 220 mg (2 mmol) 1,4-Diazabicyclo-[2.2.2]octan und 220 mg (1,1 mmol) 1S,6R-2-Oxa-5,8-diazabicyclo[4.3.0]nonan-Dihydrochlorid 1,5 Stunden unter Rückfluß erhitzt. Die Suspension wird abgekühlt, der Niederschlag abgesaugt, mit 30 ml Wasser verrührt und bei 90 ° C im Hochvakuum getrocknet.
Ausbeute: 320 mg (68 % der Theorie) 5-Brom-1-cyclopropyl-6,8-difluor-1,4-dihydro-7-(1S,6R-2-oxa-5,8-diazabicyclo[4.3.0]non-8-yl)-4-oxo-3-chinolincarbonsäure,
Schmelzpunkt: 263-264 ° C (unter Zersetzung),

$[\alpha]_D^{30}$: +251° (c=0,3, $CH_2Cl_2$).

Herstellung der Wirkstoffe:

Beispiel 1

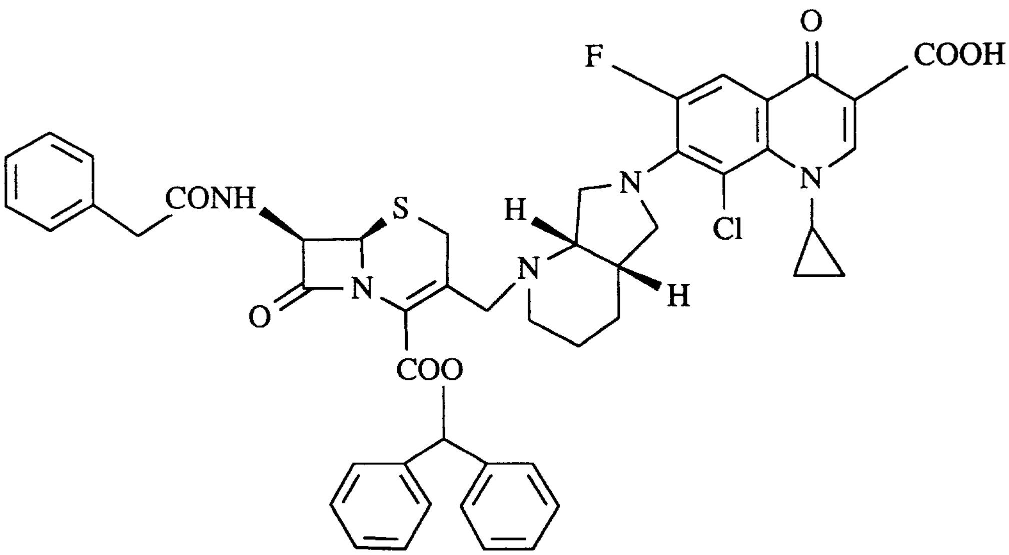

7-[2-([6R,7R]-2-Benzhydryloxycarbonyl-8-oxo-7-phenyl-acetylamino-5-thia-1-aza-bicyclo[4.2.0]oct-2-en-3-ylmethyl)-[1S,6S]-2,8-diazabicyclo[4.3.0]non-8-yl]-8-chlor-1-cyclopropyl-6-fluor-1,4-dihydro-4-oxo-3-c hinolincarbonsäure

Unter $N_2$-Atmosphäre werden 160 mg (0.4 mmol) 8-Chlor-1-cyclopropyl-7-[(1S, 6S)-2,8-diazabicyclo[4.3.0]-non-8-yl]-6-fluor-1,4-dihydro-4-oxo-3-chinolincarbonsäure in 2,25 ml Dichlormethan suspendiert. Man fügt 283 µl (1.6 mmol) Trimethylsilyltrifluoracetamid zu und rührt 20 Minuten bei Raumtemperatur, fügt 0,5 g 3 Å Molekularsieb zu, rührt eine Stunde bei Raumtemperatur und dampft die Lösung zur Trockne ein. Anschließend fügt man die Lösung von 151 mg (0,283 mmol) [6R,7R]-3-Chlormethyl-8-oxo-7-phenylacetylamino-5-thia-1-aza-bicyclo[4.2.0]oct-2-en-2-carbonsäure-benzhydrylester in 2,25 ml Aceton zu. Nach Zugabe von 46,6 mg (0.367 mmol) NaJ wird über Nacht gerührt (20 Stunden), in Wasser gegossen, pH = 4 mit 1 n HCl eingestellt und 3 x mit Essigester extrahiert. Man wäscht die organische Phase mit Wasser, trocknet über $MgSO_4$ und dampft zur Trockne ein. Durch Chromatographie an Kieselgel mit $CH_2Cl_2/CH_3OH$ (30 : 1) werden 96 mg (0.106 mmol) (38% d. Th.) des Titelproduktes erhalten.

NMR ($CDCl_3$): $\delta$ = 14.65 (1H), 8.85 (1H), 7.9 (1H), 7.45 - 7.15 (15H), 6.9 (1H), 6.2 (1H), 5.72 (1H), 4.9 (1H), 4.25 (1H), 4.0 - 2.1 (1OH), 3.7 (1H), 3.4 (1H), 3.7 (2H), 1.9 - 1.5 (4H), 1.2 (2H) und 0.9 ppm (2H).

In der gleichen Weise werden 1,62 g (3,60 mmol) 8-Chlor-1-cyclopropyl-7-[(1S, 6S)-2,8-diazabicyclo-[4.3.0]non-8-yl]-6-fluor-1,4-dihydro-4-oxo-3-chinolincarbonsäure zum Titelprodukt in 40 % Ausbeute umgesetzt.

Amorphes Pulver, Zersetzungspunkt ab 149 °C

$R_f$ = 0,12 (Dichlormethan: Methanol 97:3)

IR(KBr) $\gamma$ = 3412, 2922, 1780, (CO $\beta$-Lactam), 1728, 1616, 1496, 1436, 1314, 1220, 1177, 1100, 696 $cm^{-1}$

MS(FAB) m/z = 902 $(M+H)^+$

$^1H$-NMR (250 MHz, DMSO-$d_6$)

$\delta$ = 0,94 (m, 2H, Cyclopropyl-H), 1,19 (m, 2H, Cyclopropyl-H), 1,3-1,7 (m, 4H, -$CH_2$-), 2,0 (m, 1H), 2,4 (m, 1H), 2,90 (m, 1H), 3,15-3,85 (m, 12H, $SCH_2$, $PhCH_2CO$, $CH_2N$, CHN), 4,36 (m, 1H, Cyclopropyl-H), 5,17 (d, J = 5 Hz, 1H, H-6'), 5,20 (dd, J = 8, 5 Hz, 1H, H-7'), 6,95 (s, 1H $CHPh_2$), 7,2-7,5 (m, 15H, Ph), 7,85 (d, J = 15 Hz, 1H, CH=CF), 8,80 (s, 1H, NCH=C), 9,08 (d,J = 8 Hz, 1H, CONH), 14,82 (s, 1H, COOH).

Beispiel 2

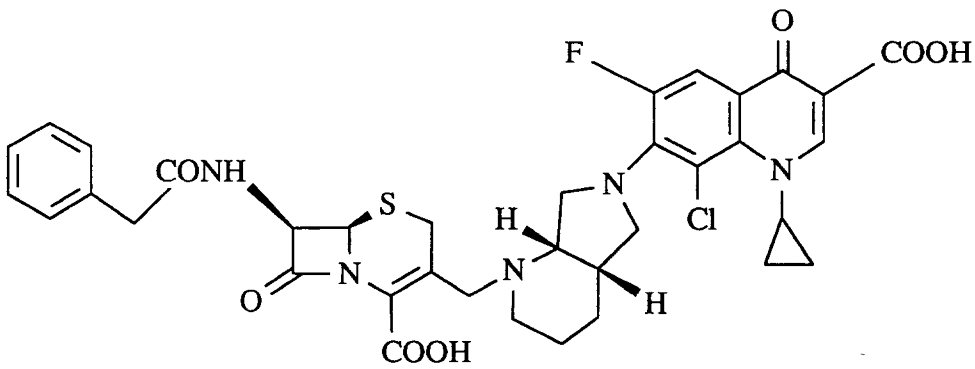

7-[2-([6R,7R]-2-Carboxy-8-oxo-7-phenylacetylamino-5-thia-1-aza-bicyclo[4.2.0]oct-2-en-3-ylmethyl)-[1S,6S]-2,8-diazabicyclo[4.3.0]non-8-yl]-8-chlor-1-cyclopropyl-6-fluor-1,4-dihydro-4-oxo-3-chinolincarbonsäure

96 mg des Produktes von Beispiel 1 werden in 1 ml Dichlormethan gelöst, mit 1 ml Trifluoressigsäure und 10 $\mu$l Anisol versetzt und eine Stunde bei Raumtemperatur gerührt. Man dampft im Vakuum zur Trockne ein, verrührt den Rückstand in ca. 1 ml Essigester/Wasser 1 : 1, stellt auf pH = 4 ein und saugt ab. Man erhält so 10 mg (13 % d. Th.) des Titelproduktes.

NMR ($CF_3COOD$) $\delta$ = 9.53 (1H), 8.22 (1H), 7.5 - 7.2 (5H), 5.72 (1H), 5.22 (1H), 5.03 - 4.5 (5H), 4.17 (1H), 3.9 (3H), 3.7 (2H), 3.45 - 3.1 (3H), 2.3 - 2.1 (4H) und 1.8 - 1.1 ppm (4H).

Aus der abgesaugten Lösung lassen sich weitere 10 mg des Produktes durch Chromatographie an Kieselgel gewinnen.

Beispiel 3

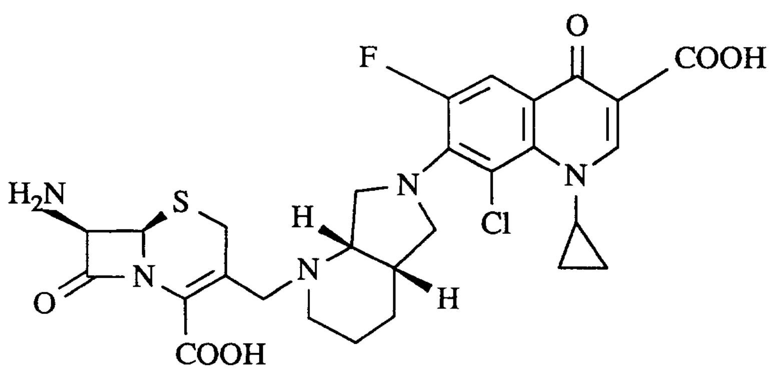

7-[2-(7-Amino-2-carboxy-8-oxo-5-thia-1-aza-bicyclo[4.2.0]oct-2-en-3-ylmethyl)-[1 S,6S]-2,8-diazabicyclo-[4.3.0]non-8-yl]-8-chlor-1-cyclopropyl-6-fluor-1,4-dihydro-4-oxo-3-chinolincarbonsäure

10 mg (0.0136 mmol) des Produktes von Beispiel 2 werden in 1 ml Wasser mit 25 mg Penicillinacylaseharz bei pH = 8,0 (Autotitrator) 4 Stunden bei Raumtemperatur gerührt. Man filtriert und lyophilisiert. Nun wird an HP-20-Harz mit Wasser und Wasser/Aceton 4 : 1 chromatographiert. Ausbeute 5,6 mg (67 % d. Th.).

Beispiel 4

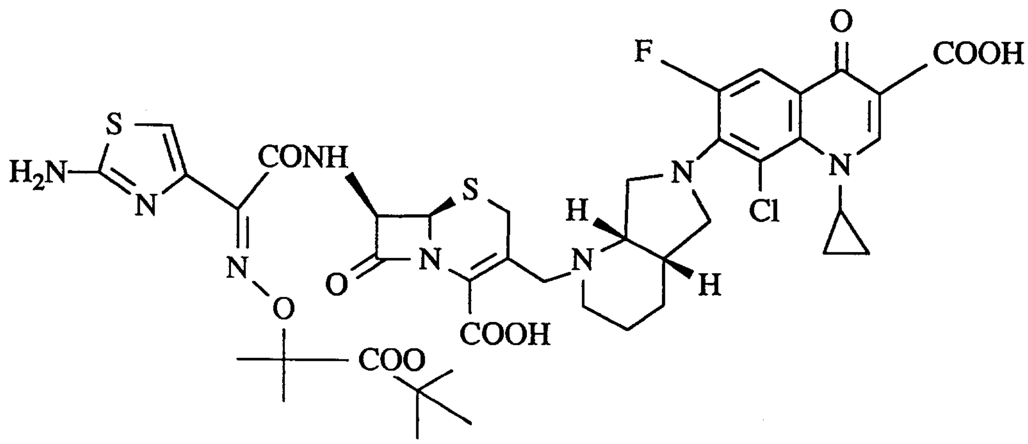

7-[2-([6R,7R]-7-[2-(Amino-thiazol-4-yl)-2-(1-tert.-butoxy carbonyl-1-methyl-ethoxyimino)-acetylamino]-2-carboxy-8-oxo-5-thia-1-aza-bicyclo[4.2.0]oct-2-en-3-ylmethyl)-[1S,6S]-2,8-diazabicyclo[4.3.0]non-8-yl]-8-chlor-1-cyclopropyl-6-fluor-1, 4-dihydro-4-oxo-3-chinolincarbonsäure

Eine Mischung von 62 mg (0.1 mmol) des Produktes von Beispiel 3, 1,5 ml Tetrahydrofuran, 3 mg Natriumbicarbonat und 1,5 ml Wasser wurden 20 Minuten bei 5 ° C gerührt. Eine Lösung von 78 mg (0,164 mmol) (Z)-2-[[[1-(2-Amino-4-thiazolyl)-2-benzothiazol-2-ylthio)- 2-oxoethyl]imino]oxy]-2-methylpropionsäure-1,1-dimethyl-ethylester in 1 ml THF wurden zugefügt und 15 Minuten bei 5 ° C weitergerührt. Ohne Kältebad wurde über Nacht gerührt. Nach Abziehen des Tetrahydrofurans im Vakuum wurde die verbleibende wäßrige Lösung mit Essigester gewaschen, mit 1 n HCl auf pH = 5 gestellt und der ausgefallene Niederschlag abgesaugt, mit Wasser und etwas Essigester gewaschen und im Vakuum getrocknet, Ausbeute 51 mg (55%).

Beispiel 5

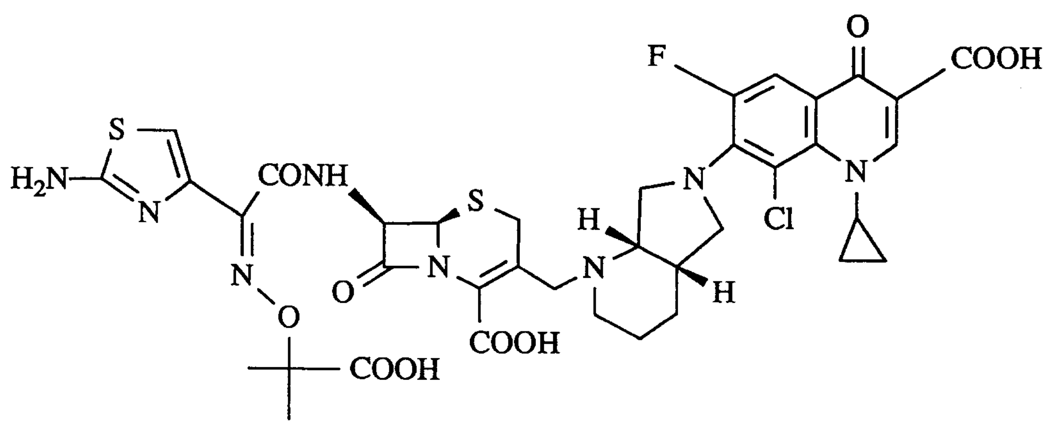

7-[2-([6R,7R]-7-[2-(2-Amino-thiazol-4-yl)-2-(1-carboxy-1-methyl-ethoxyimino)acetylamino]-2-carboxy-8-oxo-5-thia-1-aza-bicyclo[4.2.0]oct-2-en-3-ylmethyl)-[1S,6S]-2,8-diazabicyclo[4.3.0]non-8-yl]-8-chlor-1-cyclopropyl-6-fluor-1,4-dihydro-4-oxo-3-chinolincarbonsäure

50 mg (0.054 mmol) des Produktes von Beispiel 4 wurden über Nacht bei 5 ° C mit 2 ml Trifluoressigsäure, 2 ml Methylenchlorid und 0,2 ml Anisol verrührt. Man engt zur Trockne ein und behandelt den Rückstand mit Ether, wobei das Trifluoressigsäuresalz in fester Form anfällt. Anschließend wird in wäßrigem Natriumbicarbonat auf pH = 7 gestellt und an einer reversed phase-Säule mit Wasser und Wasser/Acetonitril 4 : 1 chromatographiert. Ausbeute nach Lyophilisieren: 28 mg

(0,031 mmol) = 57% d. Th.

Beispiel 6

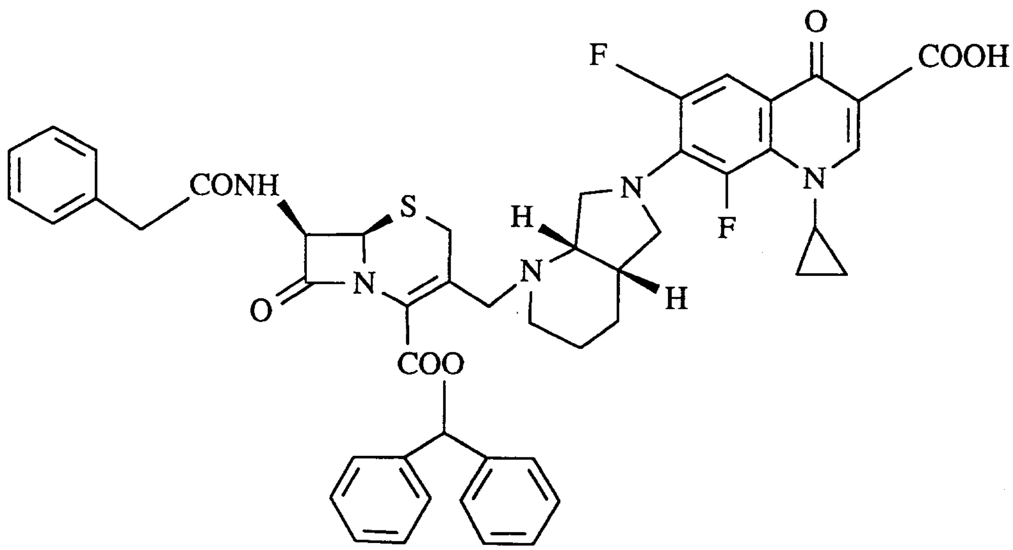

7-[2-([6R,7R]-2-Benzhydryloxycarbonyl-8-oxo-7-phenylacetylamino-5-thia-1-aza-bicyclo[4.2.0]oct-2-en-3-ylmethyl)-[1S,6S]-2,8-diazabicyclo[4.3.0]non-8-yl]-1-cyclo propyl-6,8-difluor-1,4-dihydro-4-oxo-3-chinolincarbonsäure

In der im Beispiel 1 beschriebenen Weise werden 150 mg (0,4 mmol) 1-Cyclopropyl-7-[(1S,6S)-2,8-diazabicyclo[4.3.0]non-8-yl]-6,8-difluor-1,4-dihydro-4-oxo-3-chinolincarbonsäure zum Titelprodukt in 42 % Ausbeute umgesetzt.

Beispiel 7

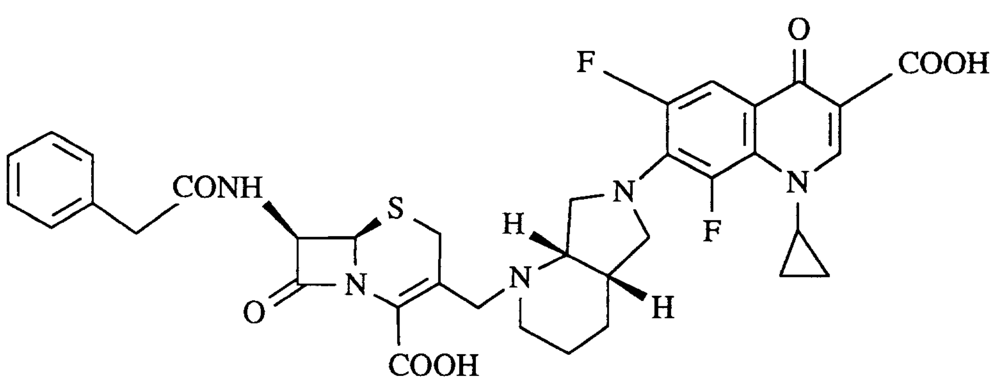

7-[2-([6R,7R]-2-Carboxy-8-oxo-7-phenyl-acetylamino-5-thia-1-aza-bicyclo[4.2.0]oct-2-en-3-ylmethyl)-[1S,6S]-2,8-diazabicyclo[4.3.0]non-8-yl]-1-cyclopropyl-6,8-difluor-1,4-dihydro-4-oxo-3-chinolincarbonsäure

In der im Beispiel 2 beschriebenen Weise wurden 80 mg des Produktes von Beispiel 6 in 40 % Ausbeute zur Titelverbindung umgesetzt.

Beispiel 8

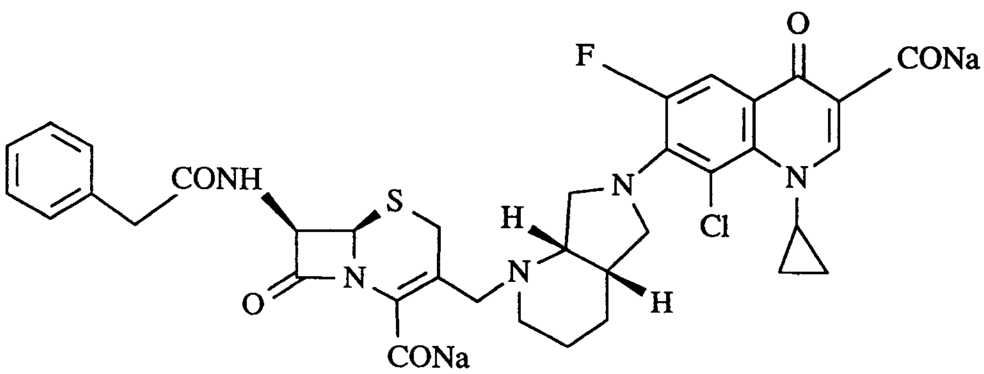

7-[2-([6R,7R]-2-Carboxy-8-oxo-7-phenyl-acetylamino-5-thia-1-aza-bicyclo[4.2.0] oct-2-en-3-ylmethyl)-[1S,6S]-2,8-diazabicyclo[4.3.0]non-8-yl]-8-chlor-1-cyclopropyl-6-fluor-1,4-dihydro-4-oxo-3-chinolincarbonsäure-Bisnatriumsalz

Eine gerührte, auf 0 °C gekühlte Lösung von 9,28 g (10,28 mmol) des Benzhydrylesters aus Beispiel 1 und 10 ml Anisol in 60 ml wasserfreiem Dichlormethan wurde langsam mit 50 ml Trifluoressigsäure versetzt. Das Kühlbad wurde entfernt und das Reaktionsgemisch 30 min bei Raumtemperatur nachgerührt Danach wurden 70 ml Toluol zugegeben und das Gemisch wurde im Vakuum eingeengt. Dieser Vorgang wurde mit 30 ml Toluol wiederholt. Der Rückstand wurde abdekantiert und der zurückbleibende Sirup mit 70 ml Ether verrieben, abgesaugt und erneut mit 100 ml Ether verrieben. Dieser Vorgang wurde zweimal wiederholt. Der so erhaltene Feststoff wurde in mehreren Portionen mittels einer HPLC-Anlage (Säule: Dynamax R-60 A, 21,4 x 250 mm, Rainin Instrument Company Nr. 83-221-C; Typ: C 18, Partikelgröße: 8 µm; Porengröße 60 Å , Fluß: 30 ml/min; Eluent Wasser: Acetonitril 4 : 6 + 0,025 % Trifluoressigsäure) gereinigt. Die produkthaltigen Fraktionen wurden gesammelt und gefriergetrocknet Das Lyophilisat wurde in 32 ml eiskaltem Wasser suspendiert und durch langsame Zugabe von 0,1 N NaOH-Lösung auf pH 8,0 gestellt. Die Lösung wurde durch eine Millipore-Membran (0,22 µm) filtriert und gefriergetrocknet.

Man erhielt 5,48 g (68 %) der Titelverbindung als farbloses Lyophilisat

$R_f$ = 0,27 (Dichlormethan: Methanol 4:1)

IR(KBr) $\gamma$ = 3434, 1750 (CO $\beta$-Lactam), 1668, 1618, 1451, 1208, 1134, 802, 725 $cm^{-1}$

MS(FAB) m/z = 736 $(M+H)^+$, 758 $(M+Na)^+$

$^1$H-NMR (300 MHz, DMSO-$d_6$)

$\delta$ = 0,71 (m, 2H, Cyclopropyl-H), 1,15 (m, 2H, Cyclopropyl-H), 1,35-1,60 (m, 4H, $-CH_2-$), 2,20 (m, 1H), 2,32 (m, 1H), 2,65 (m, 1H), 3,1-3,7 (m, 12H, $SCH_2$, $PhCH_2CO$, $CH_2N$, CHN), 3,89 (m, 1H, $CH_2N$), 4,21 (m, 1H, Cyclopropyl-H), 4,92 (d, J = 5 Hz, 1H, H-6'), 5,42 (dd, J = 8, 5 Hz, 1H, H-7'), 7,32 (m, 5H Ph), 7,75 (d, J = 15 Hz, 1H, CH=CF), 8,70 (s, 1H, NCH=C), 8,97 (d,J = 8 Hz, 1H, CONH).

Beispiel 9

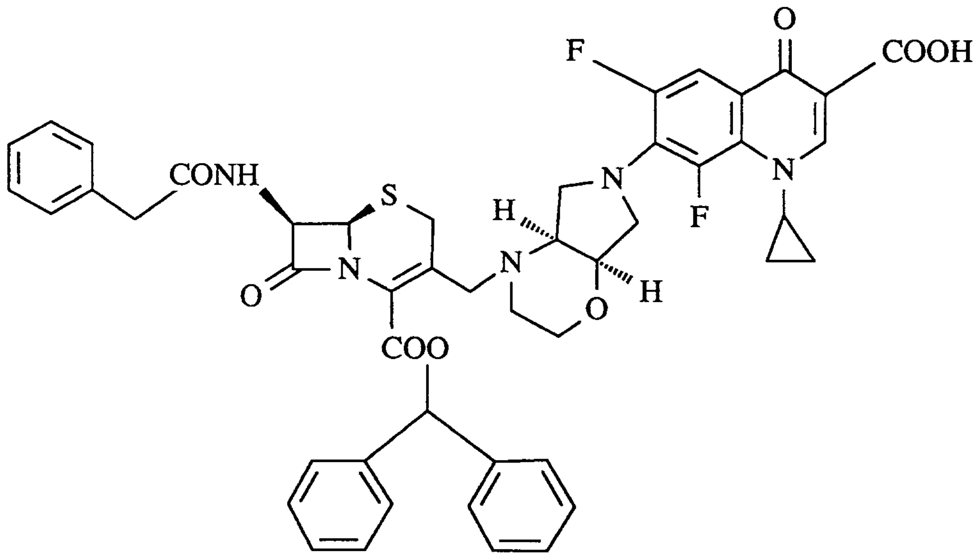

7-[2-([6R,7R]-2-Benzhydryloxycarbonyl-8-oxo-7-phenyl-acetylamino-5-thia-1-aza-bicyclo[4.2.0]oct-2-en-3-ylmethyl)-[1R,6S]-2-oxa-5,8-diazabicyclo[4.3.0]non-8-yl]-1-cyclopropyl-6,8-difluor-1,4-dihydro-4-oxo-3-chinolincarbonsäure

Wie für Beispiel 1 beschrieben erhielt man aus 876 mg (1,64 mmol) [6R, 7R]-3-Chlormethyl-8-oxo-7-phenylacetylamino-5-thia-1-aza-bicyclo [4,2,0]oct-2-en-2-carbonsäure-benzhydrylester und 900 mg (2,30 mmol) der entsprechenden Chinoloncarbonsäure 562 mg (39 %) der Titelverbindung als amorphes Pulver.

Zersetzungspunkt: ab 138 °C

$R_f$ = 0,22 (Dichlormethan: Methanol 96:4)

IR(KBr) $\gamma$ = 3420, 2937, 1738 (CO $\beta$-Lactam), 1726 (CO-Ester), 1626, 1519, 1461, 1411, 1339, 1217, 699 $cm^{-1}$

MS(FAB) m/z = 888 $(M+H)^+$

$^1$H-NMR (200 MHz, DMSO-$d_6$)

$\delta$ = 1,15 (m, 4H, Cyclopropyl-H), 3,40-3,65 (m, $SCH_2$, $PhCH_2CO$, $CH_2N$, CHN, CHO), 3,9-4,1 (m, 3H, $CH_2O$, $CH_2N$, Cyclopropyl-H), 5,18 (d, J = 5 Hz, 1H, H-6'), 5,73 (dd, J = 9,5 Hz, 1H, H-7'), 6,95 (s, 1H, $CHPh_2$), 7,2-7,5 (m, 15H, Ph) 7,24 (d, J = 14H, 1H, CH=CF), 8,60 (s, 1H, NCH=C), 9,15 (d,J = 8 Hz, 1H, CONH), 15,00 (s, 1H, COOH).

Beispiel 10

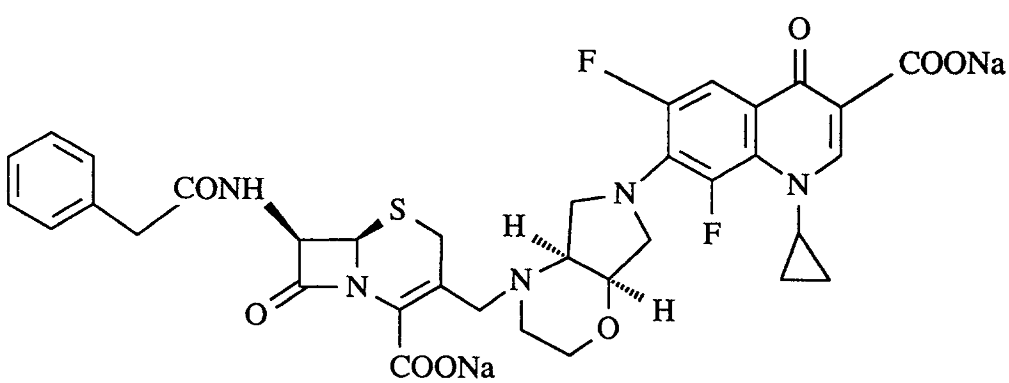

7-[2-([6R,7R]-2-Carboxy-8-oxo-7-phenyl-acetylamino-5-thia-1-aza-bicyclo[4.2.0]oct-2-en-3-ylmethyl)-[1R,6S]-2-oxa-5,8-diazabicyclo[4.3.0]non-8-yl]-1-cyclopropyl-6,8-difluor-1,4-dihydro-4-oxo-3-chinolincarbonsäure-Bisnatriumsalz
Wie für Beispiel 8 beschrieben erhält man aus 560 mg (0,63 mmol) der Verbindung aus Beispiel 9 317 mg (66 %) der Titelverbindung als farbloses Lyophilisat.
$R_f$ = 0,27 (Acetonitril : Wasser 9:1)
IR(KBr) $\gamma$ = 3424, 1762 (CO $\beta$-Lactam), 1662, 1472, 1402, 1340, 1206, 1134, 724 $cm^{-1}$
MS(FAB) m/z = 744 $(M+H)^+$
$^1H$-NMR (250 MHz, DMSO-$d_6$)
$\delta$ = 1,01 (m, 2H, Cyclopropyl-H), 1,12 (m, 2H, Cyclopropyl-H), 3,2-4,2 (m, $SCH_2$, $PhCH_2CO$, $CH_2N$, CHN, CH-O, Cyclopropyl-H)), 4,93 (d, J = 5 Hz, 1H, H-6'), 5,42 (dd, J = 5 Hz, 1H, H-7'), 7,25 (m, 5H, Ph), 7,60 (d, J = 15 Hz, 1H, CH=CF), 8,52 (s, 1H, NCH=C), 9,02 (d,J = 8 Hz, 1H, CONH).

**Patentansprüche**

1. Verbindungen der allgemeinen Formel (I)

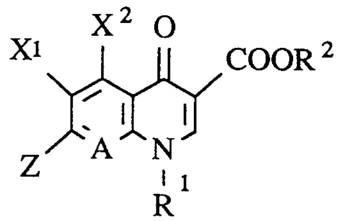

(I),

in welcher

- $X^1$ für Halogen,
- $X^2$ für Wasserstoff, Amino, Alkylamino mit 1 bis 4 Kohlenstoffatomen, Dialkylamino mit 1 bis 3 Kohlenstoffatomen je Alkylgruppe, Hydroxy, Alkoxy mit 1 bis 4 Kohlenstoffatomen, Mercapto, Alkylthio mit 1 bis 4 Kohlenstoffatomen, Arylthio, Halogen oder Methyl,
- $R^1$ für Alkyl mit 1 bis 4 Kohlenstoffatomen, Alkenyl mit 2 bis 4 Kohlenstoffatomen, Cycloalkyl mit 3 bis 6 Kohlenstoffatomen, Bicyclo[1.1.1]pent-1-yl, 1,1-Dimethylpropargyl, 3-Oxetanyl, 2-Hydroxyethyl, 2-Fluorethyl, Methoxy, Amino, Methylamino, Ethylamino, Dimethylamino, gegebenenfalls durch 1 oder 2 Fluoratome substituiertes Phenyl,
- $R^2$ für Wasserstoff, gegebenenfalls durch Hydroxy, Methoxy, Amino, Methylamino oder Dimethylamino substituiertes Alkyl mit 1 bis 5 Kohlenstoffatomen oder (5-Methyl-2-oxo-1,3-dioxol-4-yl)-methyl,
- A für N oder C-$R^5$ steht, worin
- $R^5$ für Wasserstoff, Halogen, Methyl, Alkenyl mit 2 bis 3 Kohlenstoffatomen, Alkinyl mit 2 bis 3 Kohlenstoffatomen, Hydroxy oder Methoxy steht oder auch gemeinsam mit $R^1$ eine Brücke der Struktur

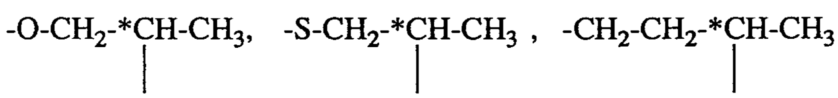

oder 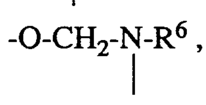

worin $R^6$ Wasserstoff, Methyl oder Formyl bedeutet,
bilden kann, und

- Z für einen Rest der Struktur

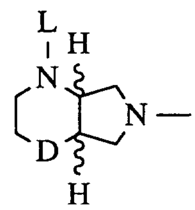

oder

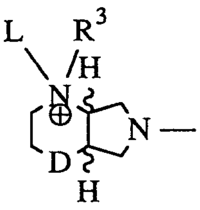

steht, worin

D für $CH_2$ oder O,

$R^3$ für Methyl oder Ethyl und

L für einen Rest der Struktur

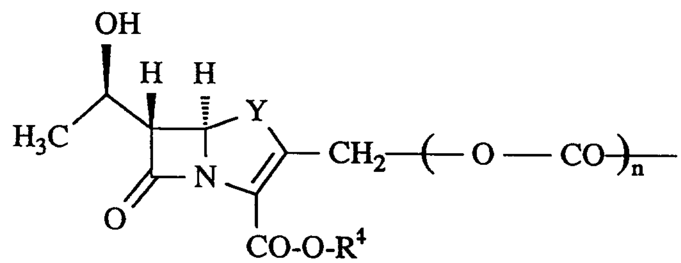

oder

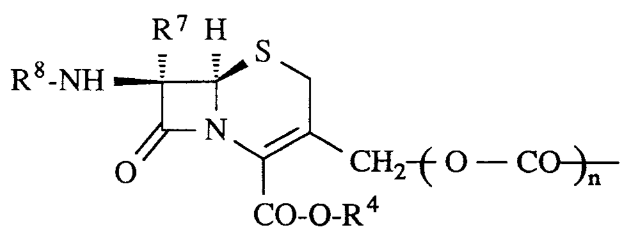

steht, worin

n für 0 oder 1,

Y für $CH_2$, $CH\text{-}CH_3$ oder S,

$R^4$ für H, Benzyl, 4-Methoxybenzyl, Benzhydryl, Allyl, (5-Methyl-2-oxo-1,3-dioxol-4-yl)-methyl oder einen Rest

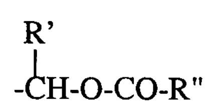

steht,

in welchem

R' H oder Methyl und

R'' Ethoxy oder tert.-Butyl bedeutet,

$R^7$ für H oder Methoxy und

$R^8$ für H, Tri-($C_1$-$C_4$-alkyl)-silyl, Acyl, $C_1$-$C_4$-Alkoxycarbonyl, Benzyloxycarbonyl, Allyloxycarbonyl, Isopropenyloxycarbonyl, steht,

und deren pharmazeutisch verwendbaren Hydrate und Säureadditionssalze sowie die Alkali-, Erdalkali-, Silber- und Guanidiniumsalze der zugrundeliegenden Carbonsäuren.

**2.** Verbindungen gemäß Anspruch 1 Formel (I), dadurch gekennzeichnet, daß

$X^1$ für Fluor,

$X^2$ für Wasserstoff, Amino, Methylamino, Hydroxy, Methoxy, Fluor, Chlor, Brom oder Methyl,

$R^1$ für Alkyl mit 1 bis 3 Kohlenstoffatomen, Vinyl, Cycloalkyl mit 3 bis 4 Kohlenstoffatomen, gegebenenfalls durch 1 oder 2 Fluoratome substituiertes Phenyl,

$R^2$ für Wasserstoff, gegebenenfalls durch Amino, Methylamino oder Dimethylamino substituiertes Alkyl mit 1 bis 2 Kohlenstoffatomen oder (5-Methyl-2-oxo-1,3-dioxol-4-yl)-methyl,

A für N oder C-$R^5$ steht, worin

$R^5$ für Wasserstoff, Fluor, Chlor, Methyl, Vinyl, Ethinyl oder Methoxy steht oder auch gemeinsam mit $R^1$ eine Brücke der Struktur

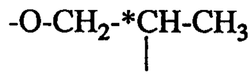 oder 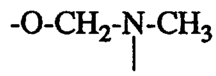

bilden kann und

Z für einen Rest der Struktur

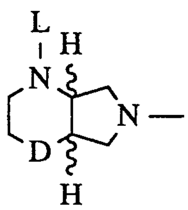 oder 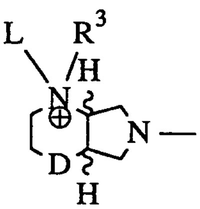

steht, worin

D für $CH_2$ oder O,

$R^3$ für Methyl und

L für einen Rest der Struktur

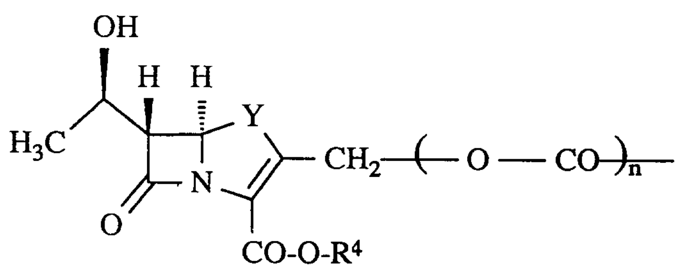

oder

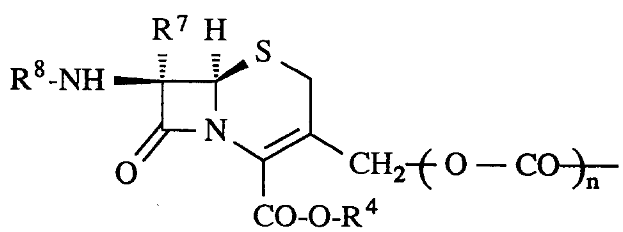

steht, worin

n für 0 oder 1,

Y für $CH_2$ oder S,

$R^4$ für H, Benzhydryl, Allyl, oder einen Rest

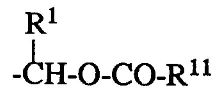

steht, in welchem

$R^1$ H oder Methyl und

$R^{11}$ tert.-Butyl bedeutet,

$R^7$ für H und

$R^8$ für H, $(CH_3)_3Si$-,

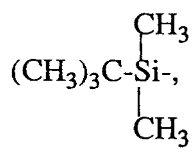

tert.-Butoxycarbonyl, Benzyloxycarbonyl, Allyloxycarbonyl, Isopropenyloxycarbonyl,

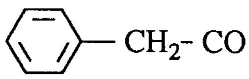 ,

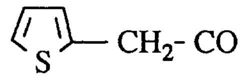 ,

$NC-CH_2CO-$ ,

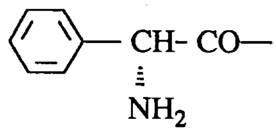 ,

$NC-CH_2S-CH_2CO-$,

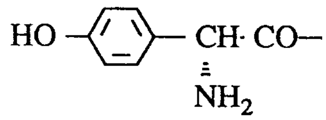 ,

$F_2CH-S-CH_2CO-$,

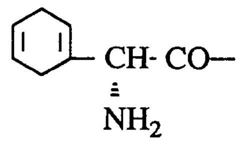 ,

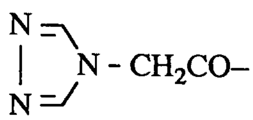 ,

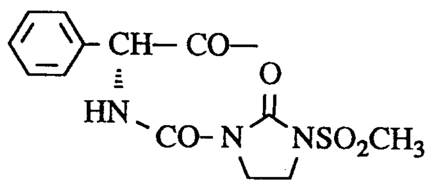 ,

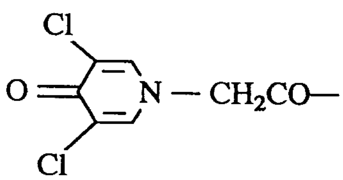 ,

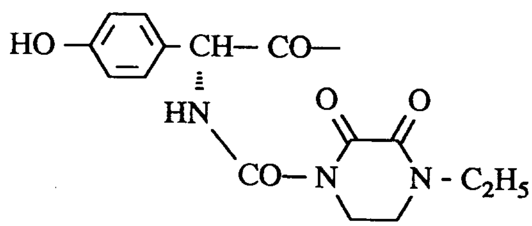 ,

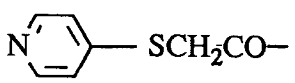 ,

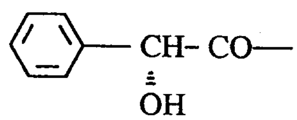 ,

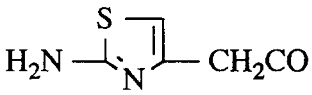 ,

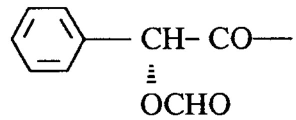 ,

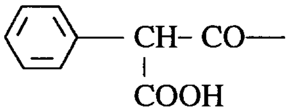 ,

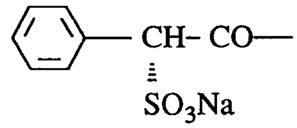 ,

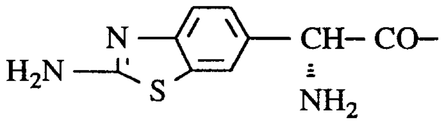 ,

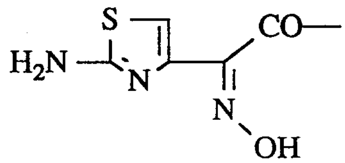 ,

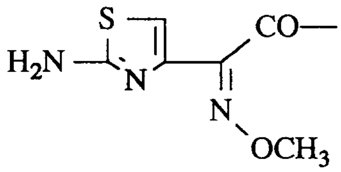 ,

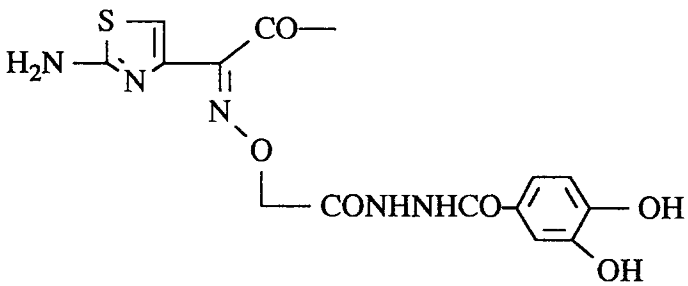 ,

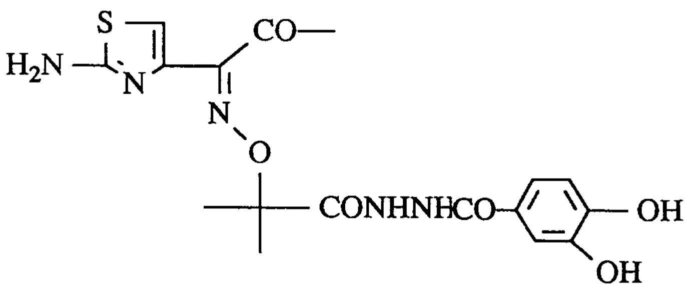 ,

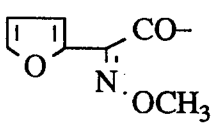 ,

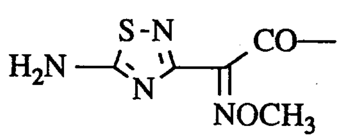 ,

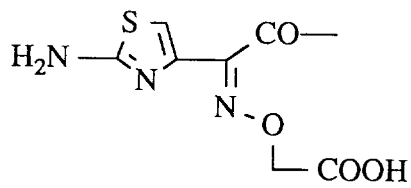 , 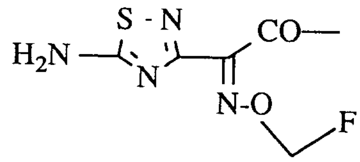 ,

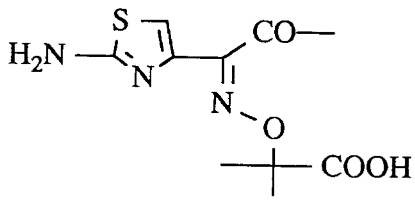 , 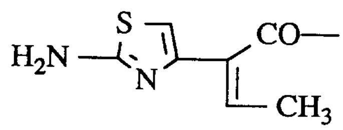 ,

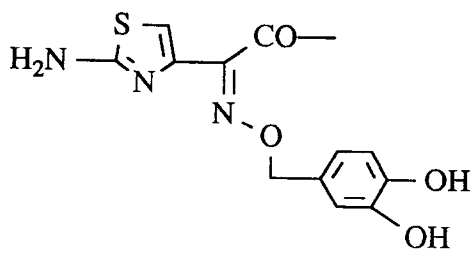 , 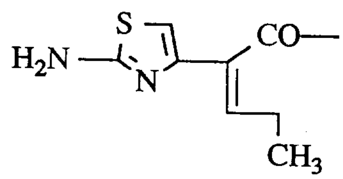 ,

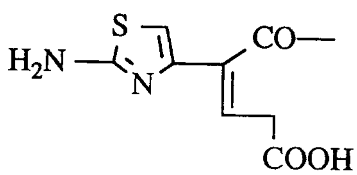 ,

steht,

und deren pharmazeutisch verwendbaren Hydrate und Säureadditionssalze sowie die Alkali-, Erdalkali-, Silber- und Guanidiniumsalze der zugrundeliegenden Carbonsäuren.

**3.** Verbindungen gemäß Anspruch 1 Formel (I), dadurch gekennzeichnet, daß

$X^1$ für Fluor,

$X^2$ für Wasserstoff, Amino oder Fluor,

$R^1$ für Alkyl mit 1 bis 2 Kohlenstoffatomen, Cyclopropyl, gegebenenfalls durch 1 oder 2 Fluoratome substituiertes Phenyl,

$R^2$ für Wasserstoff oder Alkyl mit 1 bis 2 Kohlenstoffatomen,

A für N oder C-$R^5$ steht,

worin

$R^5$ für Wasserstoff, Fluor, Chlor oder Methoxy steht oder auch gemeinsam mit $R^1$ eine Brücke der Struktur

$$-O-CH_2-{}^{*}\underset{|}{C}H-CH_3$$

bilden kann,

Z für einen Rest der Struktur

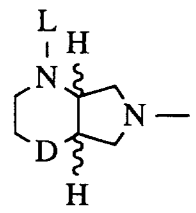

steht, worin

D für $CH_2$ oder O,

L für einen Rest der Struktur

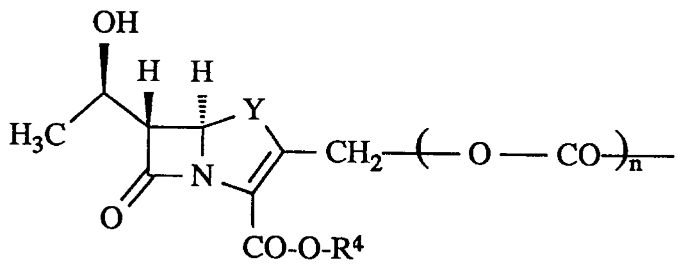

oder

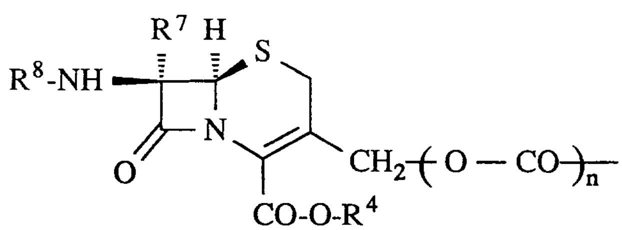

steht, worin

n für 0 oder 1,

Y für S,

$R^4$ für H, Benzhydryl, Allyl,

$R^7$ für H und

$R^8$ für H, $(CH_3)_3Si$-,

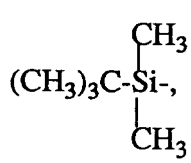

tert.-Butoxycarbonyl, Allyloxycarbonyl,

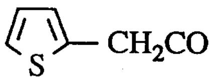 , 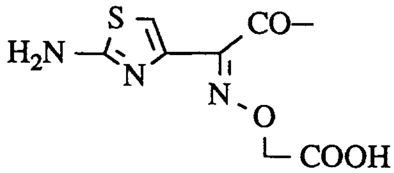 ,

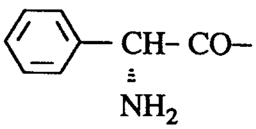 , 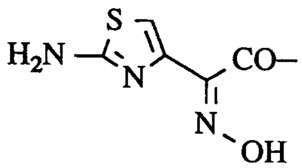 ,

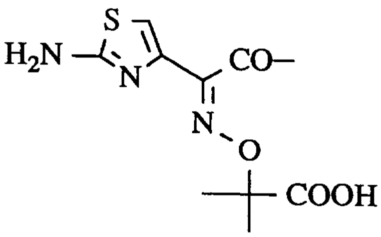

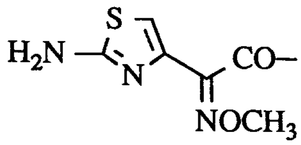

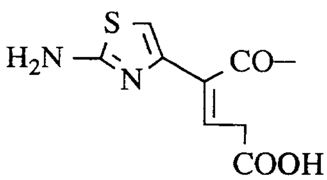

steht,

und deren pharmazeutisch verwendbaren Hydrate und Säureadditionssalze sowie die Alkali-, Erdalkali-, Silber- und Guanidiniumsalze der zugrundeliegenden Carbonsäuren.

4. Arzneimittel enthaltend Verbindungen gemäß Ansprüchen 1 bis 3.

5. Antimikrobielle Mittel enthaltend Verbindungen gemaß Ansprüchen 1 bis 3.

6. Verbindungen gemäß Ansprüchen 1 bis 3 zur Bekämpfung von Krankheiten.

7. Verbindungen gemäß Ansprüchen 1 bis 3 zur Bekämpfung von bakteriellen Infektionen.

8. Verfahren zur Herstellung von Verbindungen gemäß Ansprüchen 1 bis 3, dadurch gekennzeichnet, daß man eine Verbindung der Formel (II)

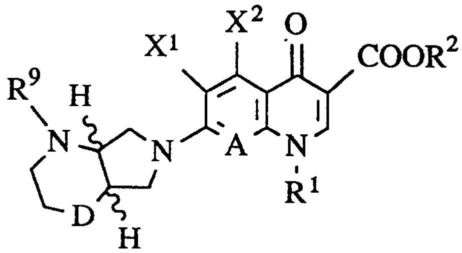 (II)

in welcher

A, $R^1$, $R^2$, $X^1$, $X^2$ und D die Bedeutung gemäß Ansprüchen 1 bis 3 haben, und

$R^9$ für H, $CH_3$ oder $C_2H_5$ steht,

mit Verbindungen der Formel (III)

L-$X^3$ (III),

in welcher

L die Bedeutung gemäß Ansprüchen 1 bis 3 hat und

$X^3$ für Halogen, insbesondere Chlor, Brom oder Jod, oder Acetoxy steht,

gegebenenfalls in Gegenwart von Säurefängern umsetzt und gegebenenfalls vorhandene Schutzgruppen abspaltet.

Europäisches Patentamt

# EUROPÄISCHER RECHERCHENBERICHT

Nummer der Anmeldung
EP 93 11 5565

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| D,Y | EP-A-0 492 277 (F. HOFFMANN-LA ROCHE AG)<br>* Seite 9 - Seite 12 * | 1,4-7 | C07D519/00<br>A61K31/47<br>//(C07D519/00, 501:00, 471:00), (C07D519/00, 501:00,498:00) |
| D,Y | EP-A-0 335 297 (F. HOFFMANN-LA ROCHE AG)<br>* Seite 34, Zeile 40 - Seite 36, Zeile 34; Beispiele 1,4 * | 1,4-7 | |
| Y | EP-A-0 451 764 (F.HOFFMANN-LA ROCHE AG)<br>* Ansprüche; Beispiel 15 * | 1,4-7 | |
| Y | EP-A-0 366 189 (NORWICH EATON PHARMACEUTICALS, INC.)<br>* Beispiele 1,8,10,54,55 * | 1,4-7 | |
| A,D | EP-A-0 350 733 (BAYER AG)<br>* Seiten 57, 58; Beispiele 8-15,22-25,31-34,40,41,43-45,48-54 * | 1,4-7 | |
| A | EP-A-0 391 132 (BAYER AG)<br>* Ansprüche; Beispiele 1,2 * | 1,4-7 | RECHERCHIERTE SACHGEBIETE (Int.Cl.5) |
| A | CHEMICAL ABSTRACTS, vol. 116, no. 3, 20. Januar 1992, Columbus, Ohio, US; abstract no. 21064c, Seite 581 ;<br>& JP-A-03 188 080 (BANYU PHARMACEUTICAL CO., LTD.) 16. August 1991 | 1,4-7 | C07D |

Der vorliegende Recherchenbericht wurde für alle Patentansprüche erstellt

| Recherchenort | Abschlußdatum der Recherche | Prüfer |
|---|---|---|
| BERLIN | 28. Januar 1994 | Hass, C |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)